(19) Europäisches Patentamt
European Patent Office
Office européen des brevets

(11) EP 0 571 870 B1

(12) **EUROPÄISCHE PATENTSCHRIFT**

(45) Veröffentlichungstag und Bekanntmachung des Hinweises auf die Patenterteilung:
**19.08.1998 Patentblatt 1998/34**

(21) Anmeldenummer: **93108044.4**

(22) Anmeldetag: **17.05.1993**

(51) Int Cl.[6]: **C07C 229/48**, C07C 271/24, C07C 271/36, C07C 271/20, C07C 237/04, C07C 239/18, A61K 31/195, A61K 31/215

(54) **Cyclopentan- und -penten-beta-Aminosäuren**

Cyclopentane- and -pentene-beta-amino-acids

Cyclopentane- et -pentène-bêta-amino-acides

(84) Benannte Vertragsstaaten:
**AT BE CH DE DK ES FR GB GR IE IT LI LU MC NL PT SE**

(30) Priorität: **29.05.1992 DE 4217776**
**27.01.1993 DE 4302155**

(43) Veröffentlichungstag der Anmeldung:
**01.12.1993 Patentblatt 1993/48**

(73) Patentinhaber: **BAYER AG**
**51368 Leverkusen (DE)**

(72) Erfinder:
- **Mittendorf, Joachim, Dr.**
  **W-5600 Wuppertal 1 (DE)**
- **Kunisch, Franz, Dr.**
  **W-5068 Odenthal (DE)**
- **Matzke, Michael, Dr.**
  **W-5600 Wuppertal 1 (DE)**
- **Militzer, Hans-Christian, Dr.**
  **W-5060 Bergisch Gladbach 2 (DE)**
- **Endermann, Rainer, Dr.**
  **W-5600 Wuppertal 1 (DE)**
- **Metzger, Karl Georg, Dr.**
  **W-5600 Wuppertal 1 (DE)**
- **Bremm, Klaus-Dieter, Dr.**
  **W-5600 Wuppertal 1 (DE)**
- **Plempel, Manfred, Dr.**
  **W-5657 Haan 1 (DE)**

(56) Entgegenhaltungen:
**EP-A- 0 298 640** **WO-A-92/18477**
**FR-A- 2 313 023** **GB-A- 1 040 546**

- **BULL. SOC. CHIM. FR. Nr. 9-10, 1977, Seiten 995 - 998 M. POLVECHE ET AL**
- **FARMACO, ED. SCI. Dd. 37, Nr. 7, 1982, Seiten 431-437 L.BRASILI ET AL.**
- **J.MED.CHEM. Bd. 14, Nr.6, 1971, Seiten 545-546. J.D. HUDDLE ET AL.**
- **J.ORG.CHEM. Bd. 32, Nr. 6 1967, Seiten 2034-2036 F.PENNINGTON ET AL.**
- **CHEMICAL ABSTRACTS, vol. 55, 1961, Columbus, Ohio, US.abstract no.5483d.**
- **J. ORG. CHEM. Bd. 44, Nr. 2, 1979, Seiten 300 - 301 R.L. CARGILL ET AL**
- **J.CHEM.SOC.,CHEM.COMM. Nr.2 1989, Seiten 113-114, T.UYEHARA ET AL.**
- **FARMACO, ED. SCI. Bd. 38, Nr. 2, 1983, Seiten 73 - 80 P. BERTHELOT ET AL**
- **'BEILSTEINS Handbuch der Organischen Chemie, 4te Auflage, 3tes Ergänzungswerk, Band 14, Teil 2' 1973, SPRINGER-VERLAG , BERLIN, DE**
- **CHEM. BER. Bd. 125, Nr. 8, 1992, Seiten 1927 - 1937 R. ASKANI ET AL**
- **J. ORG. CHEM. Bd. 57, Nr. 11, 1992, Seiten 3139 - 3145 T. UYEHARA ET AL**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 571 870 B1

## Beschreibung

Die Erfindung betrifft Cyclopentan- und -penten-β-Aminosäuren, Verfahren zu ihrer Herstellung und ihre Verwendung als Arzneimittel.

Aminopentencarbonsäurederivate sind aus der J 63 287-754 A bekannt. Außerdem sind aus der Publikation Chem. Ber. 106 (12), 2788 - 95, 2-Oxo-4,5-diphenyl-3,5cyclopentadien-1,3-dicarboxylate bekannt.

Aus EP-A 0.298.640 ist die Verbindung 2-Amino-cyclopenlan-carbonsäure als antimikrobieller Wirkstoff bekannt.

Weitere Aminopenten- bzw. Aminopentencarbonsäurederivate sind aus folgenden Dokumenten bekannt:

Bull Soc. Chim Fr. Nr. 9-10 (1977), S. 995-998; Farmaco Ed. Sci., Bd. 37 (1982), S. 431-437 und Bd. 38 (1983), S. 73-80; GB-A-1 040 546; J. Med. Chem. Bd. 14 (1971), S. 545-546; J. Org. Chem. Bd. 44 (1979) S. 300-301*; J. Chem. Soc. Chem. Commun. Nr. 2 (1989) S. 113-114, Beilstein 4. Aufl., 3tes Ergänzungswerk, Bd. 14, Teil 2' (1973) S. 872, 1392, 1393.

Die Erfindung betrifft jetzt Cyclopentan- und -penten-β-Aminosäuren der allgemeinen Formel (I)

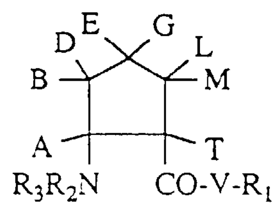

(I),

in welcher

A, B, D, E, G,L, M und T gleich oder verschieden sind und mit der Maßgabe, daß mindestens einer der vorgenanten Substituenten ≠ H ist, oder für Wasserstoff, Halogen, Benzyl, Hydroxy oder für geradkettiges oder verzweigtes Alkyl mit bis zu 8 Kohlenstoffatomen stehen, das gegebenenfalls 1- bis 2-fach gleich oder verschieden durch Halogen, Hydroxy, Phenyl, Benzyloxy, Carboxy oder durch geradkettiges oder verzweigtes Alkoxy, Acyl oder Alkoxycarbonyl mit jeweils bis zu 6 Kohlenstoffatomen oder durch eine Gruppe der Formel $-NR^4R^5$ substituiert ist, worin

$R^4$ und $R^5$ gleich oder verschieden sind und Wasserstoff, Phenyl oder geradkettiges oder verzweigtes Alkyl mit bis zu 6 Kohlenstoffatomen bedeuten,

oder jeweils B und D, E und G oder L und M gemeinsam für einen Rest der Formel

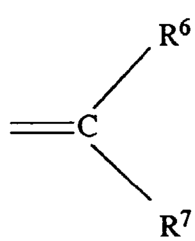

oder =N-OH stehen,
worin

$R^6$ und $R^7$ gleich oder verschieden sind und Wasserstoff, Halogen oder geradkettiges oder verzweigtes Alkyl, Alkoxy oder Oxyacyl mit jeweils bis zu 8 Kohlenstoffatomen, Benzyl oder Phenyl bedeuten,

oder

E und G und/oder B und D zusammen für den Rest der Formel =O oder =S stehen,

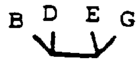 oder 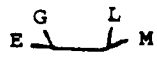

jeweils einen Rest der Formel

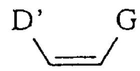 oder 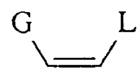

bilden,
worin

D' und G' die oben angegebene Bedeutung von D und G haben, aber nicht gleichzeitig Wasserstoff bedeuten und

G und L die oben angegebene Bedeutung haben

$R^2$ für Wasserstoff oder

für eine Aminoschutzgruppe steht, oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl mit bis zu 8 Kohlenstoffatomen,
oder
für Benzoyl steht, das gegebenenfalls wie oben beschrieben substituiert ist,
oder
für eine Gruppe der Formel $-SO_2R^8$ steht,

worin

$R^8$ geradkettiges oder verzweigtes Alkyl mit bis zu 8 Kohlenstoffatomen, Benzyl oder Phenyl bedeutet, wobei die beiden letztgenannten Reste gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^4R^5$ substitituiert sind,
worin
$R^4$ und $R^5$ die oben angegebene Bedeutung haben, oder

für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel $-NR^4R^5$ oder $-SO_2R^8$ substituiert ist,
worin
$R^4$, $R^5$ und $R^8$ die oben angegebene Bedeutung haben,
oder
für einen Aminosäurerest der Formel

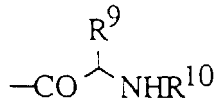

steht,
worin

$R^9$ Cycloalkyl mit 3 bis 8 Kohlenstoffatomen oder Aryl mit 6 bis 10 Kohlenstoffatomen oder Wasserstoff bedeutet, oder geradkettiges oder verzweigtes Alkyl mit bis zu 8 Kohlenstoffatomen bedeutet,

wobei das Alkyl gegebenenfalls durch Cyano, Methylthio, Hydroxy, Mercapto, Guanidyl oder durch eine Gruppe der Formel $-NR^{11}R^{12}$ oder $R^{13}$-OC- substituiert ist,
worin

$R^{11}$ und $R^{12}$ unabhängig voneinander für Wasserstoff, geradkettiges oder verzweigtes Alkyl mit bis zu 8 Kohlenstoffatomen oder Phenyl stehen,
und

$R^{13}$ Hydroxy, Benzyloxy, Alkoxy mit bis zu 6 Kohlenstoffatomen oder die oben aufgeführte Gruppe $-NR^{10}R^{11}$ bedeutet,

oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{11}R^{12}$ substituiert ist,
worin
$R^{11}$ und $R^{12}$ die oben angegebene Bedeutung haben,
und
$R^{10}$ Wasserstoff oder eine Aminoschutzgruppe bedeutet

$R^3$ für Wasserstoff oder für geradkettiges oder verzweigtes Alkyl mit bis zu 8 Kohlenstoffatomen steht, das gegebenenfalls durch Phenyl substituiert ist,
oder

$R^2$ und $R^3$ gemeinsam für den Rest der Formel $=CHR^{14}$ stehen,
worin

$R^{14}$ Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,

V für ein Sauerstoff- oder Schwefelatom oder für die -NH-Gruppe steht,

$R^1$ für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel $-NR^4R^5$ oder $-SO_2R^8$ substituiert sind,
worin
$R^4$, $R^5$ und $R^8$ die oben angegebene Bedeutung haben,
oder für den Fall, daß V für die -NH-Gruppe steht,

$R^1$ für die Gruppe der Formel $-SO_2R^8$ steht,
worin
$R^8$ die oben angegebene Bedeutung hat,

mit der Maßgabe, daß falls V für ein Sauerstoffatom steht, T ungleich Methyl, Ethyl, Benzyl oder Hydroxyl ist, $R^1$ungleich Methyl oder Ethyl ist, und
M und L ungleich $-CH_2-COOH$ sind,
gegebenenfalls in einer isomeren Form, und deren Säureadditions-Salze und Metallsalzkomplexe.

Die erfindungsgemäßen Verbindungen können auch in Form ihrer Salze vorliegen. Im allgemeinen seien hier Salze mit organischen oder anorganischen Basen oder Säuren genannt.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin, Ethylendiamin oder Phenethylamin.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro4,5-dimethoxybenzyloxycarbonyl, Methoxycarbonyl, Ethoxycarbonyl, ter.Butoxycarbonyl, Allyloxycarbonyl, Vinyloxycarbonyl, 2-Nitrobenzyloxycarbonyl, 3,4,5Trimethoxybenzyloxycarbonyl, Phthaloyl, 2,2,2-Trichlorethoxycarbonyl, 2,2,2-Trichlor-tertbutoxycarbonyl, Menthyloxycarbonyl, 4-Nitrophenoxycarbonyl, Fluorenyl-9-methoxycarbonyl (Fmoc), Formyl, Acetyl, Propionyl, Pivaloyl, 2-Chloracetyl, 2-Bromacetyl, 2,2,2-Trifluoracetyl, 2,2,2-Trichloracetyl, Benzoyl, Benzyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl oder 2-Nitrophenylsulfenyl.

Die erfindungsgemäßen Verbindungen können in stereoisomeren Formen existieren, die sich beispielsweise entweder wie Bild und Spiegelbild (Enantiomere), oder nicht wie Bild und Spiegelbild (Diastereomere) verhalten, beziehungsweise als Diastereomerengemisch oder als reine cis- oder trans-Isomere vorliegen. Die Erfindung betrifft sowohl die Antipoden, Racemformen, Diastereomerengemische sowie die reinen Isomeren. Die Racemformen lassen sich ebenso wie die Diastereomeren in bekannter Weise in die stereoisomer einheitlichen Bestandteile trennen. Die Trennung in die stereoisomer einheitlichen Verbindungen erfolgt beispielsweise über eine chromatographische Racematspaltung von diastereomeren Estern und Amiden an optisch aktiven Phasen. Außerdem ist eine Kristallisation von diastereomeren Salzen möglich.

Bevorzugt sind Verbindungen der allgemeinen Formel (I),
in welcher

A, B, D, E, G, L, M und T gleich oder verschieden sind und mit der Maßgabe, daß mindestens einer der vorgenannten Substituenten $\neq$ H ist, oder für Wasserstoff, Halogen, Benzyl, Hydroxy oder für geradkettiges oder verzweigtes Alkyl mit bis zu 6 Kohlenstoffatomen stehen, das gegebenenfalls ein- bis zweifach gleich oder verschieden durch Halogen, Benzyloxy, Hydroxy, durch geradkettiges oder verzweigtes Alkoxy, Acyl oder Alkoxycarbonyl mit jeweils bis zu 4 Kohlenstoffatomen oder durch eine Gruppe der Formel $-NR^4R^5$ substituiert ist,
worin

$R^4$ und $R^5$ gleich oder verschieden sind und Wasserstoff oder geradkettiges oder verzweigtes Alkyl mit bis zu 4 Kohlenstoffatomen bedeuten,

oder jeweils B und D, E und G oder L und M gemeinsam für einen Rest der Formel

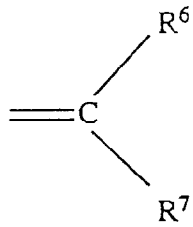

oder =N-OH stehen,
worin

$R^6$ und $R^7$ gleich oder verschieden sind und Wasserstoff, Fluor, Chlor, Brom, oder geradkettiges oder verzweigtes Alkyl mit bis zu 6 Kohlenstoffatomen, Benzyl oder Phenyl bedeuten.

oder

E und G und/oder B und D zusammen für den Rest der Formel =O oder =S stehen,
oder

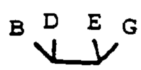 oder 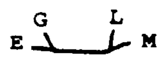

jeweils einen Rest der Formel

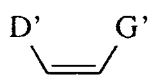 oder 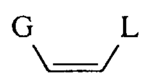

bilden,
worin

D' und G' die oben angegebene Bedeutung von D und G haben, aber nicht gleichzeitig für Wasserstoff stehen und

G und L die oben angegebene Bedeutung haben,

$R^2$ für Wasserstoff oder

für Boc, Benzyl, Benzyloxycarbonyl, Allyloxycarbonyl oder 9-Fluorenylmethyloxycarbonyl (Fmoc) steht, oder
für geradkettiges oder verzweigtes Alkyl mit bis zu 6 Kohlenstoffatomen steht, das gegebenenfalls durch Hydroxy, Formyl oder durch geradkettiges oder verzweigtes Acyl mit bis zu 4 Kohlenstoffatomen oder durch Phenyl oder Benzoyl substiutiert ist, die gegebenenfalls durch Halogen, Nitro, Cyano, oder durch geradkettiges oder verzweigtes Alkyl mit bis zu 4 Kohlenstoffatomen substituiert sind,
oder
für geradkettiges oder verzweigtes Acyl mit bis zu 6 Kohlenstoffatomen oder
für Benzoyl steht, das gegebenenfalls wie oben beschrieben substituiert ist,
oder
für eine Gruppe der Formel $-SO_2R^8$ steht,

worin

$R^8$ geradkettiges oder verzweigtes Alkyl mit bis zu 6 Kohlenstoffatomen, Phenyl oder Benzyl bedeutet, wobei die beiden letzetgenannten Reste gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel $-NR^4R^5$ substituiert ist,
worin
$R^4$ und $R^5$ die oben angegebene Bedeutung haben,
für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel $-NR^6R^7$ oder $-SO_2R^8$ substituiert ist,
worin
$R^6$, $R^7$ und $R^8$ die oben angegebene Bedeutung haben, oder
für einen Aminosäurerest der Formel

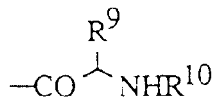

steht,
worin

$R^9$ Wasserstoff, geradkettiges oder verzweigtes Alkyl mit bis zu 6 Kohlenstoffatomen oder Benzyl bedeutet
und

$R^{10}$ Wasserstoff, Benzyloxycarbonyl, Fmoc oder tert. Butoxycarbonyl bedeutet,

$R^3$ für Wasserstoff oder für geradkettiges oder verzweigtes Alkyl mit bis zu 6 Kohlenstoffatomen oder Benzyl steht,
oder
$R^2$ und $R^3$ gemeinsam für den Rest der Formel $=CHR^{14}$ stehen,
worin

$R^{14}$ Wasserstoff oder geradkettiges oder verzweigtes Alkyl mit bis zu 6 Kohlenstoffatomen bedeutet, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist,

V für ein Sauerstoff- oder Schwefelatom oder für die -NH-Gruppe steht,
$R^1$ für Wasserstoff oder für geradkettiges oder verzweigtes Alkyl mit bis zu 6 Kohlenstoffatomen oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe der Formel $-NR^4R^5$ oder $-SO_2R^8$ substituiert sind,
worin
$R^4$, $R^5$ und $R^8$ die oben angegebene Bedeutung haben,
oder für den Fall, daß V für die -NH-Gruppe steht,
$R^1$ für die Gruppe der Formel $-SO_2R^8$ steht,
worin
$R^8$ die oben angegebene Bedeutung hat,

gegebenenfalls in einer isomeren Form und deren Säureadditions-Salze und Metallsalzkomplexe.

Besonders bevorzugt werden Verbindungen der allgemeinen Formel (I),
in welcher

A, B, D, E, G, L, M und T gleich oder verschieden sind und mit der Maßgabe, daß mindestens einer der vorgenannten Substituenten ≠ H ist, oder für Wasserstoff, Fluor, Chlor, Brom, Benzyl, Hydroxy, oder
für geradkettiges oder verzweigtes Alkyl mit bis zu 4 Kohlenstoffatomen stehen, das gegebenenfalls ein- bis zweifach gleich oder verschieden durch Hydroxy oder Benzyloxy substituiert ist,

oder jeweils B und D, E und G oder L und M gemeinsam für einen Rest der Formel

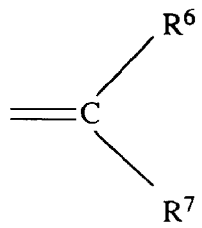

oder =N-OH stehen,
worin

$R^6$ und $R^7$ gleich oder verschieden sind und Wasserstoff, Fluor, Chlor, Brom oder geradkettiges oder verzweigtes Alkyl mit bis zu 4 Kohlenstoffatomen oder Phenyl bedeuten, oder

E und G und/oder B und D zusammen für den Rest der Formel =O oder =S stehen,
oder

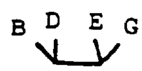 oder 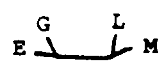

jeweils einen Rest der Formel

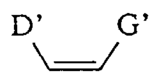 oder 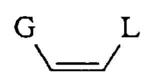

bilden,
worin

D' und G' die oben angegebene Bedeutung von D und G haben, aber nicht gleichzeitig für Wasserstoff stehen

G und L gleich oder verschieden sind und Wasserstoff oder Methyl bedeuten,

$R^2$ für Wasserstoff, Allyloxycarbonyl, Benzyl, Boc oder Fmoc steht, oder für geradkettiges oder verzweigtes Alkyl mit bis zu 4 Kohlenstoffatomen steht, oder
für geradkettiges oder verzweigtes Acyl mit bis zu 4 Kohlenstoffatomen oder für eine Gruppe der Formel $-SO_2R^8$ steht,
worin

$R^8$ geradkettiges oder verzweigtes Alkyl mit bis zu 4 Kohlenstoffatomen, Phenyl oder Benzyl bedeutet, wobei die beiden letztgenannten Reste, gegebenenfalls durch Hydroxy, Fluor, Chlor, Brom, Nitro, Cyano, Methyl, Ethyl oder Methoxy substituiert sind,
oder

für einen Aminosäurerest der Formel

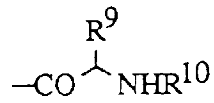

steht,
worin

$R^9$ Wasserstoff, geradkettiges oder verzweigtes Alkyl mit bis zu 4 Kohlenstoffatomen oder Benzyl bedeutet
und

$R^{10}$ Wasserstoff, tert. Butoxycarbonyl oder Fmoc bedeutet,

$R^3$ für Wasserstoff oder
für geradkettiges oder verzweigtes Alkyl mit bis zu 4 Kohlenstoffatomen steht,
oder

$R^2$ und $R^3$ gemeinsam für den Rest der Formel $=CHR^{14}$ stehen,
worin

$R^{14}$ Wasserstoff oder geradkettiges oder verzweigtes Alkyl mit bis zu 4 Kohlenstoffatomen bedeutet,

V für ein Sauerstoff- oder ein Schwefelatom oder für die -NH-Gruppe steht,

$R^1$ für Wasserstoff oder für geradkettiges oder verzweigtes Alkyl mit bis zu 4 Kohlenstoffatomen oder Phenyl steht, wobei letztere gegebenenfalls durch Fluor, Chlor, Brom, Nitro, Cyano, Methoxy, Ethoxy oder durch eine Gruppe der Formel $-NR^4R^5$ oder $-SO_2R^8$ substituiert sind,
worin

$R^4$ und $R^5$ gleich oder verschieden sind und Wasserstoff, Methyl oder Ethyl bedeuten
und

$R^8$ die oben angegebene Bedeutung hat,

oder im Fall, daß V für die -NH-Gruppe steht,

$R^1$ für die Gruppe der Formel $-SO_2R^8$ steht,
worin

$R^8$ die oben angegebene Bedeutung hat,

gegebenenfalls in einer isomeren Form, und deren Säureadditions-Salze und Metallsalzkomplexe.

Außerdem wurden Verfahren zur Herstellung der erfindungsgemäßen Verbindungen der allgemeinen Formel (I) gefunden, dadurch gekennzeichnet, daß man

[A] Verbindungen der allgemeinen Formel (II)

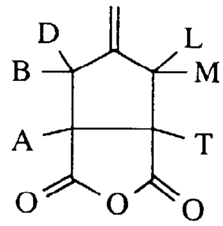 (II),

in welcher
A, B, D, L, M und T die oben angegebene Bedeutung haben,
in organischen Lösemitteln, vorzugsweise Dioxan, zunächst mit ($C_1$-$C_3$)-Triälkylsilylaziden, anschließend mit Ethern, in Anwesenheit von Wasser, in die Verbindungen der allgemeinen Formel (III)

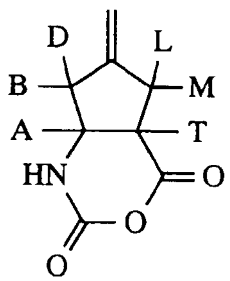 (III),

in welcher
A, B, D, L, M und T die oben angegebene Bedeutung haben,
überführt,
und in einem nächsten Schritt mit Säuren, vorzugsweise Salzsäure, unter Ringöffnung zu den Verbindungen der allgemeinen Formel (Ia)

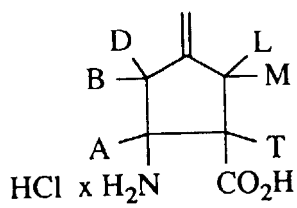 (Ia),

in welcher
A, B, D, L, M und T die oben angegebene Bedeutung haben, umsetzt,
und gegebenenfalls eine Eliminierung mit Säuren, vorzugsweise Salzsäure, anschließt, oder
[B] Verbindungen der allgemeinen Formel (IV)

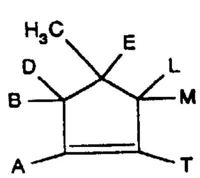 (IV),

in welcher
A, B, D, E, L, M und T die oben angegebene Bedeutung haben,
durch Umsetzung mit Chlorsulfonylisocyanat
zunächst in die Verbindungen der allgemeinen Formel (V)

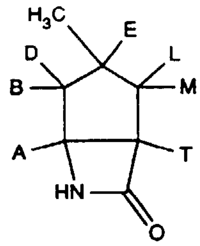
(V),

in welcher
A, B, D, E, L, M und T die oben angegebene Bedeutung haben,
überführt und anschließend mit Säuren, vorzugsweise Salzsäure, unter Ringöffnung die Amin- und Carboxyfunktion freisetzt, oder
[C] Verbindungen der allgemeinen Formel (VI)

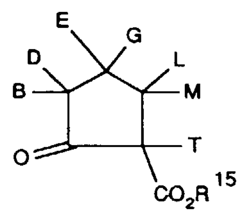
(VI),

in welcher
B, D, E, G, L, M und T die oben angegebene Bedeutung haben,

und

$R^{15}$ für $C_1$-$C_4$-Alkyl steht,

durch Umsetzung mit Aminen der allgemeinen Formel (VII)

$$H_2N\text{-}R^{16} \quad \text{(VII)},$$

in welcher

$R^{16}$ für Benzyl steht, das gegebenenfalls durch Halogen, $NO_2$, Cyano oder $C_1$-$C_4$-Alkyl oder für den Rest der Formel $-CH(C_6H_4\text{-}pOCH_3)_2$ steht,

in organischen Lösemitteln, gegebenenfalls in Anwesenheit einer Base, in die Verbindungen der allgemeinen Formel (VIII)

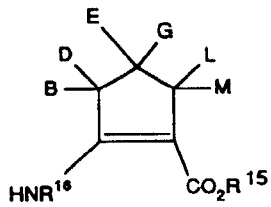

(VIII),

in welcher
B, D, E, G, L, M, $R^{15}$ und $R^{16}$ die oben angegebene Bedeutung haben,
überführt,

und anschließend durch zweifache Hydrierung zunächst die Doppelbindung reduziert, anschließend die Aminfunktion freisetzt und in einem letzten Schritt den Carbonsäureester mit Säuren verseift,

und grundsätzlich die Substituenten A - T, gegebenenfalls unter vorgeschalteter Blockierung der Aminfunktion durch Umsetzung der Schutzgruppen nach üblichen Methoden, beispielsweise durch Oxidation, Reduktion oder Alkylierung derivatisiert,

und im Fall der Säuren, die Ester nach üblichen Methoden verseift

und im Fall der für V und $R^1$ oben aufgeführten anderen Definitionen, ebenfalls nach üblichen Verfahren, wie beispielsweise Amidierung, Sulfonierung oder Sulfoamidierung, gegebenenfalls in Anwesenheit von Hilfsstoffen wie Katalysatoren und Dehydratisierungsmitteln, ausgehend von den entsprechenden Carbonsäuren, gegebenenfalls unter vorgeschalteter Aktivierung, derivatisiert,

und im Fall der reinen Enantiomere eine Racematspaltung durchführt.

Das erfindungsgemäße Verfahren kann durch folgendes Formelschema beispielhaft erläutert werden:

[A]

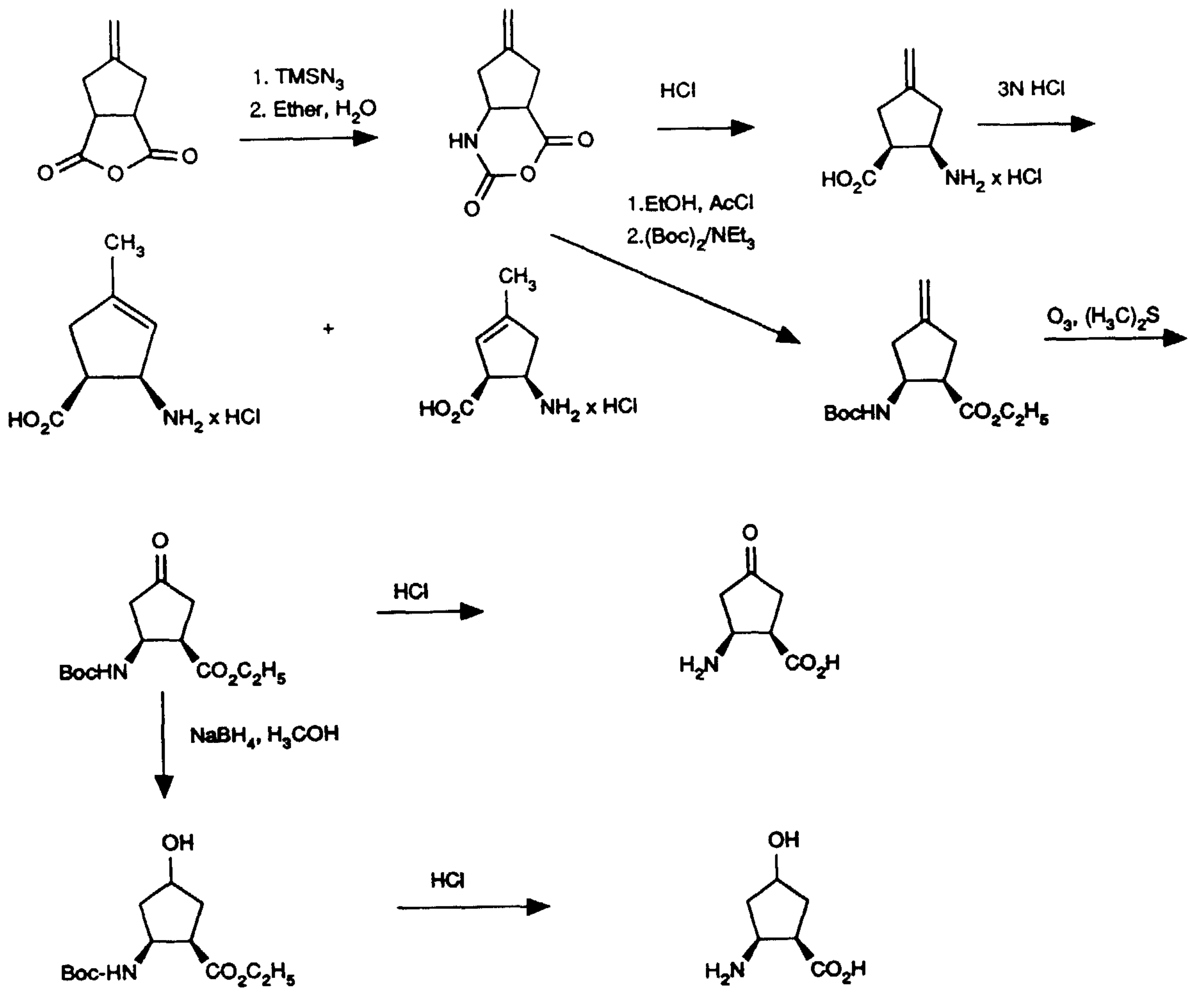
1. TMSN3
2. Ether, H2O
HCl
3N HCl
1.EtOH, AcCl
2.(Boc)2/NEt3
O3, (H3C)2S
HCl
NaBH4, H3COH
HCl

[B]

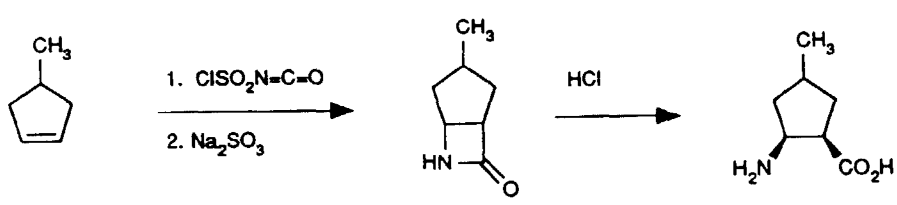
1. ClSO2N=C=O
2. Na2SO3
HCl

[C]

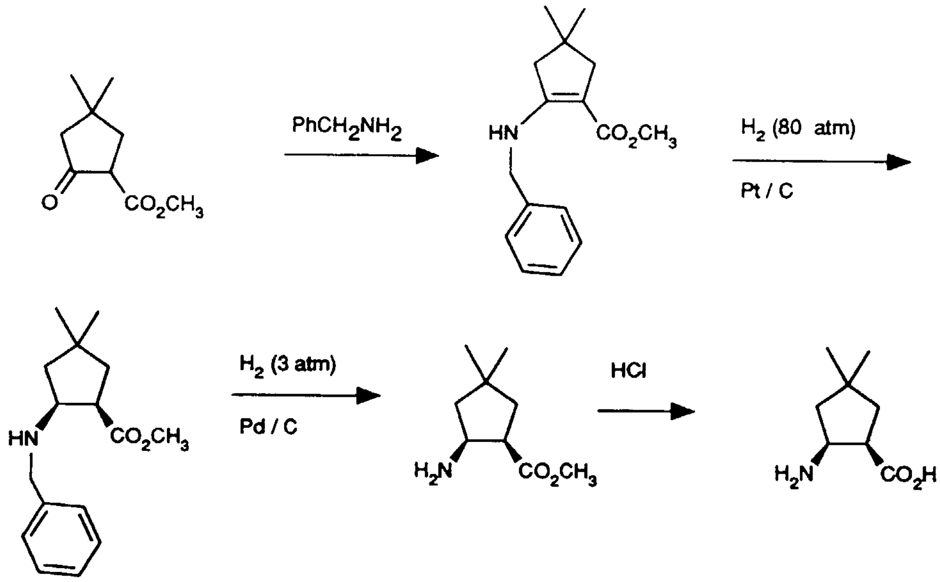


Als Lösemittel für die einzelnen Schritte der Verfahren [A], [B] und [C] kommen Wasser und alle inerten organischen Lösemittel in Frage, die sich unter den Reaktionsbedingungen nicht verändern. Hierzu gehören vorzugsweise Alkohole wie Methanol, Ethanol, Propanol, Isopropanol, Ether wie Diethylether, Dioxan, Diisopropylether, Tetrahydrofuran, Glykolmonomethylether oder Glykoldimethylether, oder chlorierte Kohlenwasserstoffe, wie Chloroform oder Methylenchlorid, oder Amide wie Dimethylformamid, Dimethylacetamid oder Hexamethylphosphorsäuretriamid, oder Eisessig, Dimethylsulfoxid, Acetonitril oder Pyridin. Bevorzugt sind für die einzelnen Schritte Diisopropylether, Diethylether, Dioxan, Methanol, Ethanol und Dichlormethan.

Die Reaktionstemperaturen können in einem größeren Breich variiert werden. Im allgemeinen arbeitet man zwischen -78°C und +150°C, vorzugsweise zwischen -10°C und +100°C.

Die Umsetzungen können bei Normaldruck, aber auch bei erhöhtem oder erniedrigtem Druck (z.B. 0,5 bis 80 bar) durchgeführt werden. Im allgemeinen arbeitet man bei Normaldruck oder bei einem erhöhtem Druck von 3 bis 80 bar.

Bei der Durchführung der erfindungsgemäßen Verfahrensvarianten [A], [B] und [C] ist das Verhältnis der an der Reaktion beteiligten Stoffe beliebig. Im allgemeinen arbeitet man jedoch mit molaren Mengen der Reaktanden. Die Isolierung und Reinigung der erfindungsgmäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Als Oxidationsmittel eignen sich beispielsweise Wasserstoffperoxid, Natriumperjodat, Persäuren wie m-Chlorperbenzoesäure oder Kaliumpermanganat. Bevorzugt sind Wasserstoffperoxid, m-Chlorperbenzoesäure und Natriumperjodat.

Als Basen eignen sich organische Amine (Trialkyl(C1-C6)amine) wie beispielsweise Triethylamin oder Heterocyclen wie Pyridin, Methylpiperidin, Piperidin oder Morpholin. Bevorzugt ist Triethylamin.

Als Säuren für die Ringöffnung (V) werden im allgemeinen Mineralsäuren eingesetzt. Bevorzugt werden hierbei Chlorwasserstoffsäure, Bromwasserstoffsäure, Schwefelsäure, Phosphorsäure oder aber Gemische der genannten Säuren eingesetzt. Bevorzugt ist Chlorwasserstoffsäure.

Als Säuren für die Deblockierung (III) eignen sich $C_1$-$C_6$-Carbonsäuren wie beispielsweise Essigsäure oder Propionsäure. Bevorzugt ist Essigsäure.

Die Säure wird im allgemeinen in einer Menge von 2 mol bis 30 mol, bevorzugt von 5 mol bis 15 mol, jeweils bezogen auf 1 mol der Verbindungen der allgemeinen Formeln (III) und (V), eingesetzt.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Die Verseifung der Carbonsäureester kann ebenso mit einer der oben aufgeführten Säuren durchgeführt werden.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat. Kaliumethanolat, Kaliummethanolat oder Kalium- tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +120°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base oder die Säure im allgemeinen in einer Menge von 1 bis 3 mol, bevorzugt von 1 bis 1,5 mol bezogen auf 1 mol des Esters eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren als vorteilhaft erwiesen, die basische Reaktionsmischung der Verseifung in einem zweiten Schritt ohne Isolierung der Salze anzusäuern. Die Säuren können dann in üblicher Weise isoliert werden.

Beispielhaft für die oben aufgeführten Derivatisierungsmöglichkeiten sollen hier die Amidierung und Sulfonierung bzw. Sulfonamidierung erläutert werden.

Die Amidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung wird im allgemeinen in einem Temperaturbereich von 0°C bis 150°C, bevorzugt bei 25°C bis 40°C, durchgeführt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Als Dehydratisierungsreagenzien eignen sich Carbodiimide wie beispielsweise Diisopropylcarbodiimid, Dicyclohexylcarbodiimid oder N-(3-Dimethylaminopropyl)N'-ethylcarbodiimid-Hydrochlorid oder Carbonylverbindungen wie Carbonyldiimidazol oder 1 ,2-Oxazoliumverbindungen wie 2-Ethyl-5-phenyl-1,2-oxazolium3-sulfonat oder Propanphosphorsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die Sulfonierung oder Sulfoamidierung erfolgt in den oben erwähnten inerten Lösemitteln, gegebenenfalls unter Einsatz der ebenfalls oben aufgeführten Basen und Dehydratisierungsmittel.

Die Sulfonierung und Sulfoamidierung werden im allgemeinen bei Normaldruck durchgeführt. Es ist aber auch möglich, die Verfahren bei Unterdruck oder Überdruck durchzuführen (z.B. in einem Bereich von 0,5 bis 5 bar).

Die Sulfonierung und die Sulfoamidierung wird im allgemeinen in einem Temperaturbereich von 0°C bis +150°C, bevorzugt von +25°C bis +40°C, durchgeführt.

Zur Amidierung eignen sich im allgemeinen die literaturbekannten, käuflichen Amine und deren Derivate.

Die Sulfonierung und Sulfoamidierung erfolgen im allgemeinen ebenfalls mit den üblichen Sulfonsäuren und deren aktivierten Derivaten.

Die Veresterung der Säuren erfolgt nach üblicher Methode, indem man die Säuren gegebenenfalls in einem der

oben aufgeführten Lösemittel in Anwesenheit eines Katalysators mit den entsprechenden Alkoholen umsetzt. Bevorzugt wird dieser Alkohol auch als Lösemittel eingesetzt.

Als Katalysatoren können anorganische Säuren, wie beispielsweise Schwefelsäure oder anorganische Säurechloride, wie beispielsweise Thionylchlorid, oder p-Toluolsulfonsäure eingesetzt werden.

Im allgemeinen setzt man 0,01 bis 1, bevorzugt 0,05 bis 0,5 mol Katalysator bezogen auf 1 mol Reaktionspartner ein.

Sowohl die Veresterung als auch die Amidierung können gegebenenfalls über aktivierte Stufen der Carbonsäuren, wie beispielsweise Säurehalogenide, die aus der entsprechenden Säure durch Umsetzung mit Thionylchlorid, Phosphortrichlorid, Phosphorpentachlorid, Phosphortribromid oder Oxalylchlorid hergestellt werden können, verlaufen.

Die Säureadditions-Salze der Verbindungen der Formel (I) können in einfacher Weise nach üblichen Salzbildungsmethoden, z.B. durch Lösen einer Verbindung der Formel (I) in einem geeigneten Lösemittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösemittel gereinigt werden.

Die Abspaltung der Aminoschutzgruppen erfolgt in an sich bekannter Weise.

Die Überführung von Doppelbindungen in Carbonylfunktionen erfolgt durch Ozonolyse und Reduktion der Ozonide mit Reduktionsmitteln wie beispielsweise Dimethylsulfoxid, Zink oder ($C_1$-$C_3$)-Trialkylphosphine.

Die Reduktion von Alkoxycarbonylverbindungen oder Aldehyden zu den entsprechenden Alkoholen erfolgt im allgemeinen mit Hydriden, wie Natriumborhydrid oder Kaliumborhydrid, bevorzugt mit Natriumborhydrid in inerten Lösemitteln wie Ethem, Kohlenwasserstoffen oder Alkoholen oder deren Gemischen, bevorzugt in Ethem wie beispielsweise Diethylether, Tetrahydrofuran oder Dioxan, oder Alkoholen wie Ethanol, im Fall der Ketone und Aldehyde bevorzugt mit Natriumborhydrid in Ethanol, in einem Temperaturbereich von 0°C bis +150°C, bevorzugt von +20°C bis +100°C, bei Normaldruck.

Die Einführung von Doppelbindungen erfolgt im allgemeinen durch Überführung der Alkohole in die entsprechenden Mesylate, Tosylate, Bromide, Iodide oder Arylselenylverbindungen, vorzugsweise mit 2-Nitrophenylselenocyanat und Tri-n-butylphosphin, und anschließende Eliminierung der Abgangsgruppen mit Basen, vorzugsweise mit einem der oben aufgeführten organischen Amine, oder durch Eliminierung der Selenylgruppen durch Oxidation, vorzugsweise mit $H_2O_2$ in $H_2O$ / THF.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Hydrierungen (Reduktionen, Abspaltungen von Schutzgruppen) erfolgen im allgemeinen in einem der oben aufgeführten Lösemitteln wie Alkoholen, beispielsweise Methanol, Ethanol oder Propanol, in Anwesenheit eines Edelmetallkatalysators wie Platin, Platin/C, Palladium, Palladium auf Tierkohle oder Raney-Nickel, im Fall der Doppelbindung der Verbindung der allgemeinen Formel (VIII) bevorzugt mit $H_2$/Platin oder mit $H_2$/Palladium.

Als Katalysatoren werden im allgemeinen Säuren verwendet Hierzu gehören bevorzugt anorganische Säuren wie beispielsweise Salzsäure oder Schwefelsäure, oder organische Sulfon- oder Carbonsäuren wie beispielsweise Methansulfonsäure, Ethansulfonsäure, Benzolsulfonsäure, Toluolsulfonsäure, Essigsäure oder Propionsäure.

Die Hydrierungen können bei normalem, erhöhtem oder erniedrigtem Druck (z.B. 0,5 - 5 bar) durchgeführt werden.

Die Katalysatoren und Basen werden im allgemeinen in einer Menge von 0 mol bis 10 mol, bevorzugt von 1,5 mol bis 3,5 mol jeweils bezogen auf 1 mol der Verbindungen der allgemeinen Formeln (IV), (V), (VI) und (VIII) eingesetzt.

Die Säure wird im allgemeinen in einer Menge von 2 mol bis 30 mol, bevorzugt von 5 mol bis 15 mol, jeweils bezogen auf 1 mol der Verbindungen der allgemeinen Formeln (IV), (V), (VI) und (VIII), eingesetzt.

Die Verbindungen der allgemeinen Formel (II) sind größenteils neu und können beispielsweise hergestellt werden, indem man in Verbindungen der allgemeinen Formel (IX)

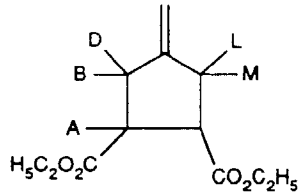

(IX)

in welcher
A, B, D, L, M und T die oben angegebene Bedeutung haben
zunächst durch basische Verseifung, vorzugsweise mit Lithiumhydroxid / $H_2O$ in einem der oben aufgeführten Lösemittel, vorzugsweise Tetrahydrofuran, die entsprechenden Dicarbonsäuren freisetzt, anschließend mit Propionsäureanhydrid umsetzt.

Die Verbindungen der allgemeinen Formel (IX) sind an sich bekannt oder können nach üblichen Methoden hergestellt werden [vgl. H.J. Gais, J. Org. Chem. 1989, 54, 5115].

Die Verbindungen der allgemeinen Formeln (V) und (VIII) sind neu und können nach dem oben aufgeführten Verfahren beispielhaft hergestellt werden.

Die Verbindungen der allgemeinen Formel (IV) sind an sich bekannt oder können nach üblicher Methode hergestellt werden.

Die Verbindungen der allgemeinen Formel (VI) sind größtenteils bekannt oder können nach literaturbekannten Methoden hergestellt werden [vgl. Joc. 1983, 48, 5364; JACS, 1951, 73, 4286; JACS, 1978, 100, 6728].

Die Amine der allgemeinen Formel (VII) sind bekannt und sind nach üblichen Methoden herstellbar oder käuflich.

Die Verbindungen der allgemeinen Formel (Ia) sind neu und können nach dem oben aufgeführten Verfahren hergestellt werden.

Die reinen Enantiomere können erhalten werden, indem man ausgehend vom Racemat, zunächst die Arninfunktion mit einer Schutzgruppe, vorzugsweise Fmoc, blockiert, anschließend nach Umsetzung mit chiralen Aminen, wie beispielsweise Phenethylamin oder (-)-Chinin, bevorzugt mit Phenethylamin die entsprechenden diastereomeren Salze kristallisiert und in einem letzten Schritt die Schutzgruppe, beispielsweise mit flüssigem Ammoniak, abspaltet

Das Verfahren kann durch folgendes Formelschema beispielhaft erläutert werden:

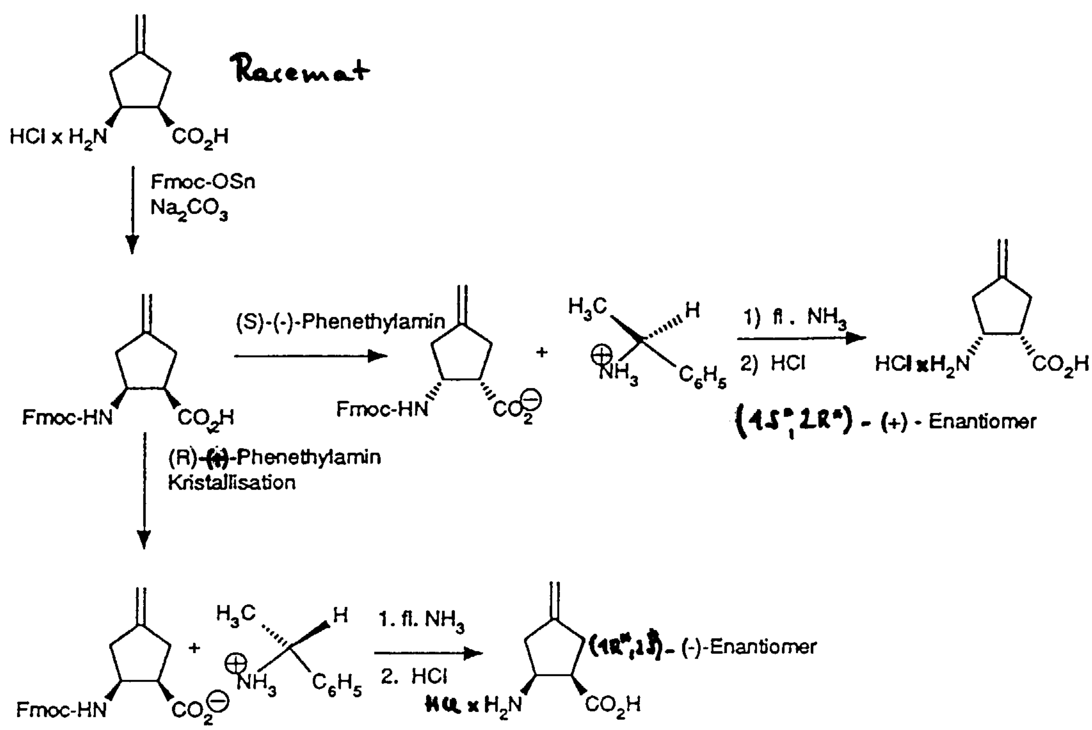

Fmoc = 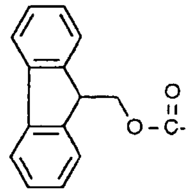

Die vorstehenden Herstellungsverfahren sind lediglich zur Verdeutlichung angegeben. Die Herstellung der erfindungsgemäßen Verbindungen der allgemeinen Formeln (I) und (Ia) ist nicht auf diese Verfahren beschränkt, jede Modifikation dieser Verfahren ist in gleicher Weise für die Herstellung anwendbar.

Die erfindungsgemäßen Verbindungen der allgemeinen Formel (I) und ihre Säureadditions-Salze weisen starke antimikrobielle und antimykotische Wirkungen auf. So besitzen sie beipielsweise ein sehr breites antimykotisches Wirkspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Tabelle A:

| Bsp. Nr. | Dosis [mg/kg, 2 x tägl.] | Applikation | Zahl der überlebenden Tiere |
|---|---|---|---|
| Kontrolle | | | 1/10 |
| 1 | 25,50 | sc,po | 7/10 |
| 2 | 25 | sc,po | 9/10 |
| 9 | 25 | sc | 3/10 |
| 32 | 10 | sc,po | 10/10 |
| 37 | 10 | sc,po | 10/10 |
| 41 | 50 | po | 7/10 |
| 45 | 25 | po | 10/10 |
| 46 | 25 | po | 8/10 |
| 47 | 25 | po | 8/10 |
| 49 | 10 | po | 8/10 |

Als Indikationsgebiete in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiete in der Tiermedizin können beispielsweise aufgeführt werden: Alle Dermatomykosen und Systemmykosen, insbesondere solche, die durch die oben genannten Erreger hervorgerufen werden.

Die erfindungsgemäßen Verbindungen zeigen darüberhinaus ein nicht vorhersehbares, wertvolles antibakterielles Wirkspektrum.

Sie sind bei geringer Toxizität gegen grampositive Keime antibakteriell wirksam, vor allem auch gegen solche Keime, die resistent sind gegen verschiedene Antibiotika, wie z.B. Penicilline, Cephalosporine, Tetrazykline, Makrolide, Chinolone.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Mit Hilfe der erfindungsgemäßen Verbindungen können grampositive Bakterien bekämpft, sowie die durch diese Erreger hervorgerufenen Erkrankungen verhindert, gebessert und/oder geheilt werden. Sie sind daher besonders gut zur Prophylaxe und Chemotherapie von lokalen und systemischen Infektionen in der Human- und Tiermedizin geeignet, die durch diese Erreger hervorgerufen werden.

So wurde eine logarithmisch wachsende S. aureus 133-Kultur mit physiologischer Kochsalzlösung verdünnt, so daß 1 x $10^8$ Bakterien in 0,25 ml intraperitoneal in die Mäuse injiziert werden konnten. Die Therapie der infizierten Tiere erfolgte 0,5 und 3 Stunden nach der Infektion. Das Überleben der Mäuse wurde bis zum 6. Tag nach der Infektion

protokolliert.

| | % Überlebende am | | | | | |
|---|---|---|---|---|---|---|
| Beispiel 32 | 1. | 2. | 3. | 4. | 5. | 6. Tag |
| Dosisgruppe | | | | | | |
| 2 × 25 mg/kg | 100 | 50 | 50 | 50 | 50 | 50 |
| 2 × 50 mg/kg | 100 | 67 | 50 | 50 | 50 | 50 |
| 2 × 100 mg/kg | 100 | 100 | 83 | 83 | 83 | 83 |
| Infektionskontrolle | 33 | 17 | 17 | 17 | 17 | 17 |

Beispiel 32 zeigt im Vergleich zur unbehandelten Infektionskontrolle eine dosisabhängige therapeutische Wirkung.

| | % Überlebende am | | % Überlebende Infektionskontrolle | |
|---|---|---|---|---|
| Beispiel-Nr. | 1. Tag | 2. Tag | 1. Tag | 2. Tag |
| 2 | 83 | 50 | 33 | 16 |
| 5 | 67 | 33 | 33 | 16 |
| 16 | 83 | 50 | 33 | 16 |
| 17b | 50 | 50 | 33 | 16 |
| 24 | 83 | 33 | 33 | 16 |
| 37 | 100 | 50 | 67 | 16 |

Der neue Wirkstoff kann in bekannter Weise in die üblichen Formulierungen überführt werden, wie Tabletten, Dragees, Kapseln, Pillen, Granulate, Suppositorien, Aerosole, Sirupe, Emulsionen, Suspensionen und Lösungen, Pasten, Salben, Gele, Cremes, Lotions, Puder oder Sprays, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,1 bis 99,9-Gew.-%, vorzugsweise von etwa 0,5 bis 95-Gew.-% der Gesamtmischung vorhanden sein, d.h. in Mengen, die ausreichend sind, um den angegebenen Dosierungsspielraum zu erreichen.

Der oder die Wirkstoffe können gegebenenfalls in einem oder mehreren der oben angegebenen Trägerstoffe und in mikroverkapselter Form vorliegen.

Die oben aufgeführten pharmazeutischen Zubereitungen können außer den erfindungsgemäßen Verbindungen auch weitere pharmazeutische Wirkstoffe enthalten.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich sowohl in der Human- als auch in der Veterinärmedizin als vorteilhaft erwiesen, den oder die erfindungsgemäßen Wirkstoffe in Gesamtmengen von 0,5 bis 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von 1 bis 80, insbesondere 3 bis 30 mg/kg Körpergewicht.

Im allgemeinen hat es sich als vorteilhaft erwiescn, bei intravenöser Applikation Mengen von 0,001 bis 10 mg/kg, vorzugsweise 0,01 bis 5 mg/kg Körpergewicht zur Erzielung wirksamer Ergebnisse zu verabreichen, und bei oraler Applikation beträgt die Dosierung 0,01 bis 25 mg/kg, vorzugsweise 0,1 bis 10 mg/kg Köpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

**Ausgangsverbindungen**

**Beispiel I**

1,2-cis-4-Methylen-cyclopentan-1,2-dicarbonsäure

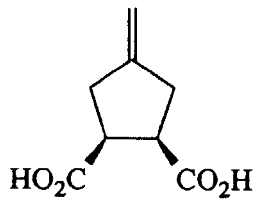

Zur Lösung von 1,2-cis-4-Methylen-cyclopentan-1,2-dicarbonsäurediethylester (19,0 g; 84 mmol) in 100 ml THF tropft man bei 0°C eine Lösung von LiOH x $H_2O$ (7,8 g; 185 mmol) in 150 ml Wasser. Die entstehende Lösung wird 20 h bei Raumtemperatur gerührt, das THF wird im Vakuum abgezogen und der Rückstand einmal mit 40 ml Ether extrahiert. Die wäßrige Phase wird mit 10%iger Salzsäure auf pH 2 gebracht und dreimal mit je 200 ml Essigester extrahiert. Die vereinigten Essigesterphasen werden über $Na_2SO_4$ getrocknet und das Solvens im Vakuum abgezogen.

Ausbeute: 13,4g (93% der Theorie)
Smp.: 116-120°C
$C_8H_{10}O_4$ (170,2)

**Beispiel II**

1,2-cis-4-Methylen-cyclopentan-1,2-dicarbonsäure-anhydrid

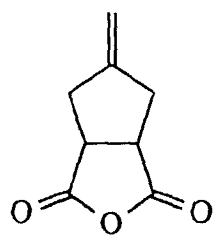

Eine Lösung der Verbindung aus Beispiel I (13,0 g; 76,5 mmol) in 65 ml Propionsäureanhydrid wird 3 h unter Rückfluß erhitzt. Das Lösemittel wird bei 60°C / 0,5 Torr abgezogen und der Rückstand destilliert.

Ausbeute: 10,0 g (86% der Theorie)
Sdp.: 130-140°C / 0,1 Torr
Smp.: 47-49°C
$C_8H_8O_3$ (152,1)

**Beispiel III**

6-Methylen-cyclopentano[3,4-d]oxazin-2,4-(1H)-dion

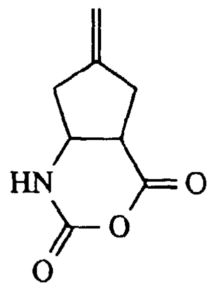

Eine Lösung der Verbindung aus Beispiel II (8,8 g, 58 mmol) und Trimethylsilylazid (7,9 g, 69 mmol) in 60 ml Dioxan wird 2 h auf 80°C erhitzt. Das Solvens wird im Vakuum abgezogen, der Rückstand wird in 80 ml Ether aufgenommen und mit $H_2O$ (0,52 g, 29 mmol) versetzt. Die Mischung wird 5 min kräftig gerührt und 2 Tage bei 6°C aufbewahrt. Ausgefallenes Produkt wird abfiltriert und mit Diethylether gewaschen.

Ausbeute: 4,2 g (43% der Theorie)
Smp.: 145°C (Zersetzung)
$C_8H_9NO_3$ (167,2)

**Beispiel IV**

1,2-cis-2-N-(tert.Butyloxycarbonyl)amino-4-(2-nitrophenyl)selenyl-cyclopentan-1-carbonsäure-methylester

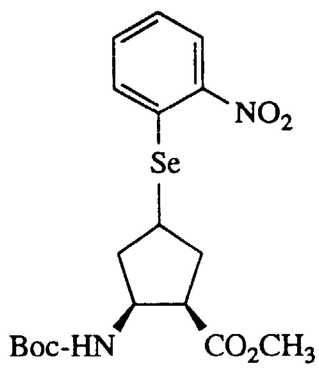

Eine Lösung der Verbindung aus Beispiel 63 (3.30 g, 12,7 mmol) in 50 ml THF wird bei Raumtemperatur unter Argon mit 2-Nitrophenylselenocyanat (3,46 g, 15,2 mmol) und anschließend mit Tri-n-butylphosphin (3,08 g, 15,2 mmol) versetzt. Die Mischung wird 1 h bei Raumtemperatur gerührt, das Lösemittel wird im Vakuum abgezogen und der Rückstand an Kieselgel chromatographiert (Ether : Petrolether = 2:1).

Ausbeute: 4,45 g (79% der Theorie)
Diastereomerenverhältnis $D_1:D_2 = 3:1$
$R_f = 0,28$ und 0,39 (Ether / Petrolether = 2:1)
$C_{18}H_{24}N_2O_6Se$ (443,4)

**Allgemeine Arbeitsvorschrift A für 2-Benzylamino-cyclopent-1-en-carbonsäuremethylester**

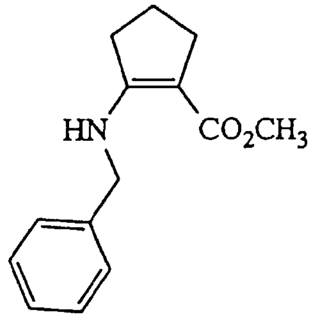

Eine Lösung des substituierten Cyclopentan-2-oncarbonsäuremethylesters (160 mmol) und Benzylamin (34,2 g, 320 mmol) in 540 ml Dichlormethan wird mit p-Toluolsulfonsäure (200 mg) und 108 g Molekularsieb (4 A) versetzt und 24 h am Wasserabscheider unter Rückfluß erhitzt Die Mischung wird filtriert und das Filtrat im Vakuum eingeengt. Der Rückstand wird an Kieselgel chromatographiert.

**Beispiel V**

2-Benzylamino-4,4-dimethyl-cyclopent-1-en-carbonsäuremethylester

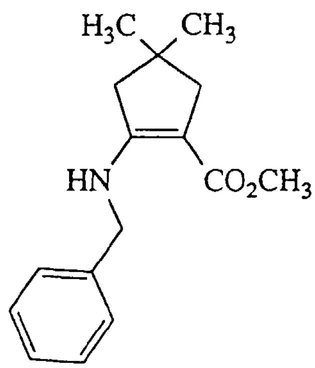

In Analogie zur Arbeitsvorschrift A wird die Titelverbindung hergestellt.

Ausbeute: 30,0 g (72% der Theorie)
$R_f$ = 0,49 (Petrolether / Essigester = 3:1)
$C_{16}H_{21}NO_3$ (259,3)

**Beispiel VI**

2-Benzylamino-5-methyl-cyclopent-1-en-carbonsäuremethylester

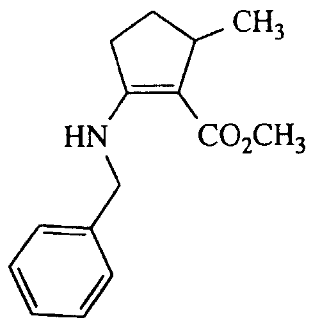

In Analogie zur Arbeitsvorschrift A wird die Titelverbindung hergestellt.

Ausbeute: 27,9 g (71% der Theorie)
$R_f$ = 0,42 (Ether / Petrolether = 5:1)
$C_{15}H_{19}NO_2$ (245,3)

**Beispiel VII**

2-Benzylamino-3-methyl-cyclopent-1-en-carbonsäuremethylester

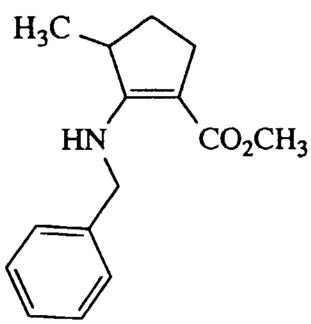

In Analogie zur Arbeitsvorschrift wird die Titelverbindung hergestellt.

Ausbeute: 20,0 g (51% der Theorie)
$R_F$ = 0,45 (Ether / Petrolether 1:5)
$C_{15}H_{19}NO_2$ (245,3)

**Beispiel VIII**

4-Ethyliden-cyclopentan-1,2-dicarbonsäurediethylester

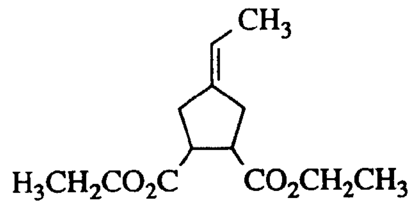

Zur Lösung von Kalium-t-butanolat (24,8 g, 220 mmol) in 1 000 ml Diethylether gibt man bei Raumtemperatur unter Argon Ethyl-triphenylphosphoniumbromid (100 g, 270 mmol) und läßt 20 h bei Raumtemperatur rühren. Bei 0°C tropft man eine Lösung von 4-Cyclopentanon-1,2-dicarbonsäurediethylester (15,8 g, 69 mmol) in 120 ml Diethylether hinzu und läßt 1 h bei 0°C rühren. Man versetzt mit 300 ml Wasser, wäscht die organische Phase mit gesättigter NaCl-Lösung, trocknet über $Na_2SO_4$, filtriert und zieht das Lösungsmittel i. Vak. ab. Der Rückstand wird an Kieselgel (Petrolether/Ether = 2:1) chromatographiert.

Ausbeute: 13,1 g (79 %) eines cis/trans-Diastereomerengemisches
$^1$H-NMR ($CDCl_3$): δ = 1,23 (t, 6H), 1,58 (m, 3H), 2,3-2,6 (m, 4H), 3,0-3,22 (m, 2H), 4,17 (q, 4H), 5,35(cm, 1H)
$C_{13}H_{20}O_4$

**Beispiel IX**

4-Ethyliden-cyclopentan-1,2-dicarbonsäure

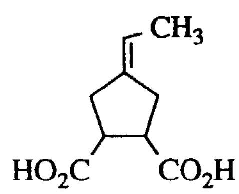

Zur Lösung der Verbindung aus Beispiel VIII (13,1 g, 54,5 mmol) in 70 ml THF tropf man bei O°C eine Lösung von LiOH x $H_2O$ (5,1 g, 120 mmol) in 130 ml Wasser. Die Lösung wird 20 h bei Raumtemperatur gerührt, das THF i. Vak. abgezogen und der Rückstand einmal mit 40 ml Ether extrahiert. Die wäßrige Phase wird mit 10 %iger Salzsäure auf pH 2 gebracht und dreimal mit je 200 ml Essigester extrahiert. Die vereinigten Essigesterphasen werden über $Na_2SO_4$ getrocknet und das Solvens wird i. Vak. abgezogen.

Ausbeute: 9,0 g (90 %) eines Diastereomerengemisches
Smp.: 170°C
$C_9H_{12}O_4$(184,2)

**Beispiel X**

1,2-cis-4-Ethyliden-cyclopentan-1,2-dicarbonsäureanhydrid

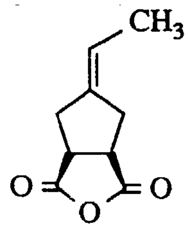

Eine Lösung der Verbindung aus Beispiel IX (8,25 g, 44,7 mmol) in 37 ml Propionsäureanhydrid wird 3 h unter Rückfluß erhitzt. Das Lösemittel wird bei 60°C/0,5 Torr abgezogen und der Rückstand destilliert.

Ausbeute: 2,0 g (27 %)
Smp.: 150°C/0,1 Torr (Kugelrohrdestillation)
$C_9H_{18}O_2$ (166,2)

**Beispiel XI**

6-Ethyliden-cyclopentano[3,4-d]oxazin-2,4-(1H)-dion

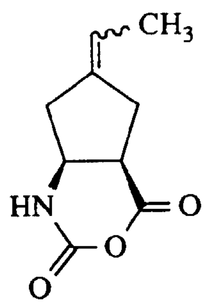

Eine Lösung der Verbindung aus Beispiel X (2,0 g, 12,0 mmol) und Trimethylsilylazid (1,66 g, 14,4 mmol) in 12 ml Dioxan wird 2 h auf 80°C erhitzt. Das Solvens wird i. Vak. abgezogen, der Rückstand wird in 13 ml Ether aufgenommen und mit Wasser (0,22 g, 12 mmol) versetzt. Die Mischung wird 5 min kräftig gerührt und 3 h bei 6°C aufgewahrt. Ausgefallenes Produkt wird abgesaugt und mit Ether gewaschen.

Ausbeute: 0,48 g (22 %) eines Diastereomeremgemisches
Smp.: >250°C (Zers.)
$C_9H_{11}NO_3$ (181,2)

**Beispiel XII**

4-Benzyliden-cyclopentan-1,2-dicarbonsäure-diethylester

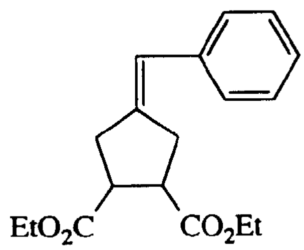

Zur Lösung von Kalium-t-butanolat (22,0 g, 196 mmol) in 1200 ml Diethylether gibt man beim Raumtemperatur unter Argon, Benzyl-triphenylphosphoniumchlorid (95,4 g, 245 mmol) und läßt 4 h bei Raumtemperatur rühren. Anschließend tropft man eine Lösung von 4-Cyclopentanon-1,2-dicarbonsäure-diethylester (14,0 g, 61,3 mmol) bei O°C zu und erhitzt 8 d unter Rückfluß. Die weitere Aufarbeitung erfolgt in Analogie zur Vorschrift des Beispiels VIII.

Ausbeute: 15,9 g (86 %), cis/trans-Isomere
$R_f$= 0,37, 0,43 (Petrolether/Ether = 5:1)
$^1$H-NMR ($CDCl_3$): δ = 1,25 (2t, 6H), 2,70-3,35 (m, 6H), 4,17 (q, 4H), 6,38 (cm, 1H), 7,12-7,40 (m, 5H).
$C_{18}H_{22}O_4$ (302.37)

**Beispiel XIII**

4-Benzyliden-cyclopentan-1,2-dicarbonsäure

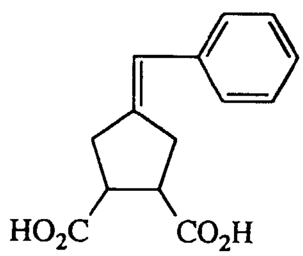

Die Titelverbindung wird in Analogie zur Vorschrift des Beispiels IX hergestellt.

Ausbeute: 12,8 g (100 %)
Smp.: 172°C
$C_{14}H_{14}O_4$ (246.26)

**Beispiel XIV**

1,2-cis-4-Difluormethylen-cyclopentan-1,2-dicarbonsäure-diethylester

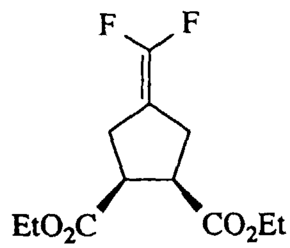

Zur Lösung von 1,2-cis-4-Cyclopentanon-1,2-dicarbonsäure-diethylester(20,0 g, 87,6 mmol) und Dibromdifluormethan (36,8 g, 175 mmol) in THF (400 ml) tropft man bei O°C unter Argon innerhalb von 30 min Tris-(dimethylamino)-phosphin (57,1 g, 350 mmol). Man läßt die Mischung langsam auf Raumtemperatur erwärmen und noch 1 h bei dieser Temperatur rühren. Man versetzt mit Triethylamin (17,6 g, 175 mmol) und läßt 15 h bei Raumtempertur rühren. Nach Zugabe von 500 ml Wasser wird die Reaktionsmischung mit Diethylether extrahiert (3 x 500 ml) und die vereinten org. Phasen werden mit ges. NaCl-Lösung gewaschen (2 x 300 ml), über $Na_2SO_4$ getrocknet und im Vakuum eingeengt. Der Rückstand wird an Kieselgel chromatographiert (Petrolether/Diethylether = 1:1).

Ausbeute: 5,93 g (27 %)
$^1$H-NMR ($CDCl_3$): $\delta$ = 1,28 (t, 6H), 2,55-2,90 (4H), 3,17 (dt, 2H), 4,17 (q, 4H).
$C_{12}H_{16}F_2O_4$ (262.4)

**Beispiel XV**

1,2-cis-4-Difluormethylen-cyclopentan-1,2-dicarbonsäure

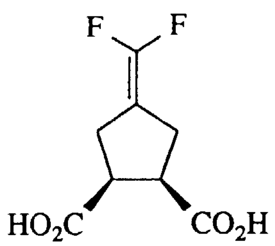

Die Titelverbindung wird in Analogie zur Vorschrift des Beispiels IX hergestellt.

Ausbeute: 3,86 g (85 %)
Smp.: 147-149°C
$C_8H_8F_2O_4$ (206.1)

**Beispiel XVI**

4-Difluormethylen-cyclopentan-1,2-dicarbensäureanhydrid

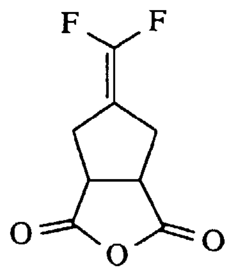

Die Titelverbindung wird in Analogie zur Vorschrift des Beispiels X hergestellt.

Ausbeute: 2,25 g (65 %)
Smp.: 140-145°C/0,05 mbar (Kugelrohrdestillation)
$C_8H_6F_2O_3$(188.1)

**Beispiel XVII**

6-Difluormethylen-cyclopentano[3,4-d]oxazin-2,4-(1H)-dion

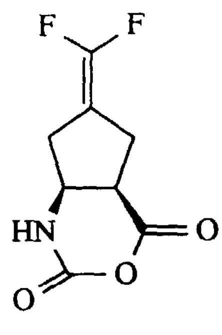

Die Titelverbindung wird in Analogie zur Vorschrift des Beispiels XI hergestellt.

Ausbeute: 1,40 g (59 %)
Smp.: 130°C (Zers.)
$C_8H_7F_2NO_3$ (203.1)

**Beispiel XVIII**

4,4-Difluor-cyclopentan-1,2-dicarbonsäure-diethylester

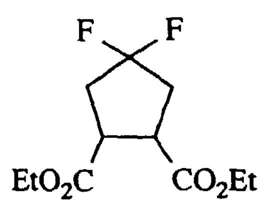

Zur Lösung von 4-Cyclopentanon-1,2-dicarbonsäure-diethylester (6,43 g, 28,2 mmol) in 100 ml Toluol tropft man bei 0°C Diethylaminoschwefeltrifluorid (11,28 g, 70 mmol) und läßt 5 d bei Raumtemperatur rühren. Die Lösung wird auf Eiswasser gegossen, man extrahiert mit Essigester (2 x 200 ml), trocknet die organische Phase ($Na_2SO_4$) und zieht das Solvens im Vakuum ab. Der Rückstand wird an Kieselgel chromatographiert (Petrolether/Diethylether = 1:1).

Ausbeute: 3,79 g (56 %) eines Diastereomerengemisches
$R_f$ = 0,65 (Petrolether/Diethylether = 1:1)
$^1$H-NMR ($CDCl_3$): δ = 1,28 (t, 6H), 2,52 (cm, 4H), 3,29 (cm, 2H), 4,18 (q, 4H)
$C_{11}H_{16}O_4F_2$ (250,2).

**Beispiel XIX**

4,4-Difluor-cyclopentan-1,2-dicarbonsäure

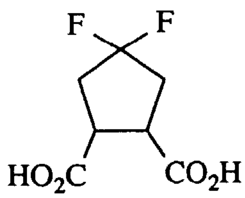

Die Titelverbindung wird in Analogie zur Vorschrift des Beispiels XI hergestellt.

Ausbeute: 4,40g (77 %)
Smp.: 128°C
$C_7H_8O_4F_2$ (194,1).

**Beispiel XX**

4,4-Difluor-cyclopentan-1,2-dicarbonsäureanhydrid

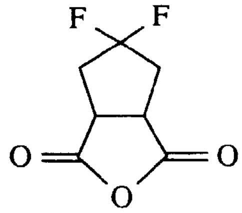

Die Titelverbindung wird in Analogie zur Vorschrift des Beispiels X hergestellt.

Ausbeute: 2,90 g (75 %)
Sdp.: 150°C/0,15 mbar (Kugelrohrdestillation)
$C_7H_6F_2O_3$ (176,12).

**Beispiel XXI**

6,6-Difluor-cyclopentano[3,4-d]oxazin-2,4-(1H)-dion

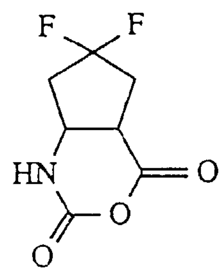

Die Titelverbindung wird in Analogie zur Vorschrift des Beispiels XI hergestellt.

Ausbeute: 2,33 g (74 %)
Smp.: 116°C (Zers.)
$C_7H_7F_2NO_3$ (191,1).

**Beispiel XXII**

3-Benzyloxymethyl-cyclopentan-2-on-1-carbonsäureethylester

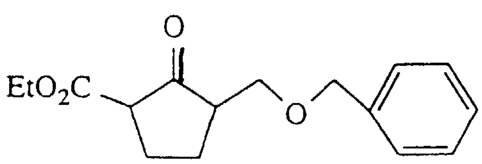

Zur Lösung von Lithiumdiisopropylamid (138 mmol) in 300 ml THF tropft man bei -78°C unter Argon eine Lösung von 2-Benzyloxymethyl-cyclopentan-2-on (23,5 g, 115 mmol, Herstellung nach Murata S., Tetrahedron Letters, 1980, 2527) in 100 ml THF und läßt 30 min bei -78°C und 10 min bei -40°C rühren. Bei -78°C tropft man 1,3-Dimethyltetrahydro-2-(1H)-pyrimidon (14,7 g, 115 mmol) hinzu und tropft schließlich die so hergestellte Lösung bei -78°C zur Lösung von Cyanameisensäureethylester (22,8 g, 230 mmol). Nach 10 min gießt man die Reaktionsmischung auf 200 ml Wasser, zieht das THF im Vakuum ab und nimmt den Rückstand in 1100 ml Diethylether auf. Man wäscht die organische Phase mit Wasser (3 x 100 ml), trocknet ($Na_2SO_4$) und engt im Vakuum ein. Der Rückstand wird an Kieselgel chromatographiert (Petrolether/Diethylether = 2:1).

Ausbeute: 19,3 g (61 %)
$R_f$ = 0,34 (Petrolether/Diethylether = 2:1)
$C_{16}H_{20}O_4$ (276,3).

**Beispiel XXIII**

2-Benzylamino-3-benzyloxymethyl-cyclopent-1-en-1-carbonsäureethylester

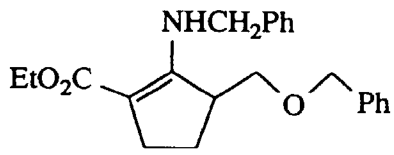

Die Titelverbindung wird in Analogie zur Vorschrift des Beispiels V ausgehend von Beispiel XXII hergestellt.

Ausbeute: 14,2g (54 %)
$R_f$ = 0,62 (Petrolether/Diethylether = 2:1)
$C_{24}H_{27}NO_3$ (377,48).

**Herstellungsbeispiele**

**Beispiel 1**

(+/-)-(1RS,2SR)1,2-cis-2-Amino-4-methylen-cyclopentan-1-carbonsäureethylester Hydrochlorid

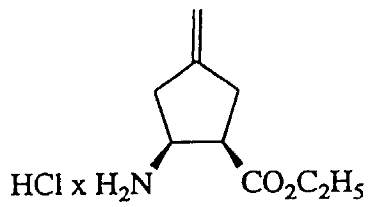

Zur Lösung der Verbindung aus Beispiel III (3,90 g, 23,3 mmol) in 48 ml EtOH wird Acetylchlorid (3,01 g, 38,4 mmol) getropft. Die Lösung wird 20 h bei Raumtemperatur gerührt und das Solvens wird im Vakuum abgezogen.

Ausbeute: 4,79 g (100%)
$R_f$ = 0,48 (Ether: Acetonitril : konz. $NH_3$ / 10:1:0,1)
$C_9H_{15}NO_2$ x HCl (169,2 x 36,5)

**Beispiel 2**

(+/-)-(1RS, 2SR)- 1,2-cis-2-Amino-4-methylen-cyclopentan-1-carbonsäure Hydrochlorid

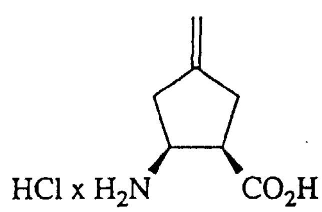

Eine Lösung der Verbindung aus Beispiel III (0,500 g, 3,00 mmol) in 30 ml 0,1 N HCl (3,00 mmol) wird 4h bei Raumtemperatur gerührt. Das Solvens wird im Vakuum bei 30°C abgezogen und der Rückstand 12 h bei 30°C / 0,1 Torr getrocknet.

Ausbeute: 0,513 g (96%)
Smp.: 190°C
$C_7H_{11}NO_3$ x HCl (141,2 x 36,5)

**Beispiel 3**

(+/-)-(1RS, 2SR)-1,2-cis-2-N-(tert.Butyloxycarbonyl)amino-4-methylen-cyclopentan-1-carbonsäureethylester

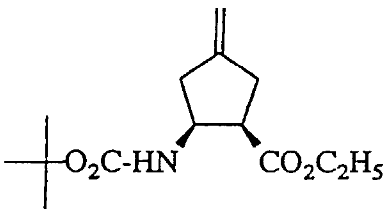

Eine Lösung der Verbindung aus Beispiel 1 (15,4 g, 75,0 mmol) und Triethylamin (22;8 g, 225 mmol) in 225 ml $CH_2Cl_2$ wird bei 0°C mit Di-tert.Butyldicarbonat (24,8 g, 112 mmol) versetzt und 4 h bei Raumtemperatur gerührt. Das Solvens wird im Vakuum abgezogen und der Rückstand wird an Kieselgel chromatographiert (Ether / Petrolether = 1:3).

Ausbeute: 18,0 g (91%)
$R_f$ = 0,29 (Ether / Petrolether = 1:3)
$C_{14}H_{23}NO_4$ (269,3)

**Beispiel 4**

(+/-)-(1RS ,2SR)-1,2-cis-2-N-(tert. Butyloxycarbonyl)amino-4-oxo-cyclopentan-1-carbonsäureethylester

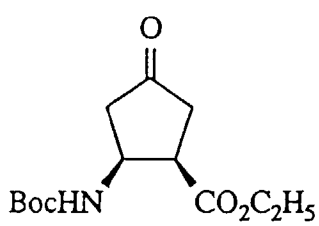

Durch die Lösung der Verbindung aus Beispiel 3 (18,0 g, 67,0 mmol) wird bei -70°C Ozon bis zur Blaufärbung und anschließend Sauerstoff bis zur Entfärbung geleitet. Man versetzt mit Dimethylsulfid (24,8 g, 0,40 mol), läßt auf Raumtemperatur erwärmen und noch 2 h bei dieser Temperatur rühren. Das Solvens wird im Vakuum abgezogen, der Rückstand mit Diisopropylether verrührt, abgesaugt und mit Diethylether gewaschen.

Ausbeute: 15,1 g (83%)
Smp.: 132°C
$C_{13}H_{21}NO_5$ (271,3)

**Beispiel 5**

(+/-)-(1RS, 2SR)-1,2-cis-2-Amino-4-oxo-cyclopentan-1-carbonsäureethylester Hydrochlorid

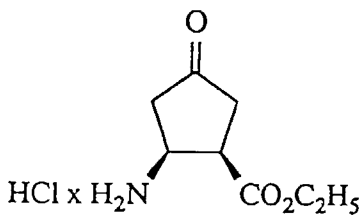

Eine Lösung der Verbindung aus Beispiel 4 (0,980 g, 3,60 mmol) in 5 ml 4 N HCl in Dioxan wird 3 h bei Raumtemperatur gerührt. Das Lösemittel wird im Vakuum abgezogen und der Rückstand 20 h bei 50°C / 0,1 Torr getrocknet.

Ausbeute: 0,734 g (98%)
$^1$H-NMR (DMSO-$d_6$): δ = 1,24 (t, J = 7 Hz, 3H); 2,14 - 2,80 (m, 4H); 3,49 - 3,62 (m, 1H), 4,02 - 4,28 (m, 3H); 8,53 (s, breit, 3H).
$C_8H_{13}NO_3$ x HCl (171,2 x 36,5)

**Beispiel 6**

(+/-)-(1RS, 2SR)1,2-cis-2-Amino-4-oxo-cyclopentan-1-carbonsäure Hydrochlorid

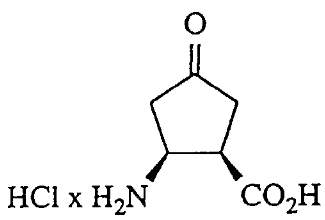

Eine Lösung der Verbindung aus Beispiel 5 (0,500 g, 2,41 mmol) in 40 ml 3 N HCl wird 2 h auf 80°C erhitzt. Das Lösemittel wird im Vakuum abgezogen und der Rückstand 20 h bei 50°C / 0,1 Torr getrocknet.

Ausbeute: 0,432 g (100%)
$^1$H-NMR (DMSO-$d_6$): δ = 2,42 - 2,76 (m, 4H); 3,42 - 3,56 (m, 1H); 4,08 (s, breit, 1H); 8,45 (s, breit, 3H).
$C_6H_9NO_3$ x HCl /(141,3 x 36,5)

**Beispiel 7**

(+/-)-(1RS, 2SR)-1,2-cis-2-N-(tert.Butyloxycarbonyl)amino-4-hydroxy-cyclopentan-carbonsäureethylester

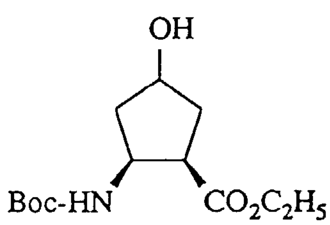

Eine Lösung der Verbindung aus Beispiel 6 (5,00 g, 18,5 mmol) in 150 ml MeOH wird bei 5°C mit $NaBH_4$ (0,345

g, 9,0 mmol) versetzt und 1h bei Raumtemperatur gerührt. Das Solvens wird im Vakuum abgezogen, der Rückstand in Wasser aufgenommen und mit $CH_2Cl_2$ extrahiert. Die organische Phase wird über $Na_2SO_4$ getrocknet und das Lösemittel im Vakuum abgezogen.

Ausbeute: 4,9 g (97%)
Diastereomerenverhältnis $D_1$:$D_2$ = 3:1
$R_f$ = 0,42 und 0,48 (Ether)
$C_{13}H_{23}NO_5$ (273,3)

**Beispiel 8**

(+/-)-(1RS, 2SR)-1,2-cis-2-Amino-4-hydroxy-cyclopentancarbonsäureethylester Hydrochlorid

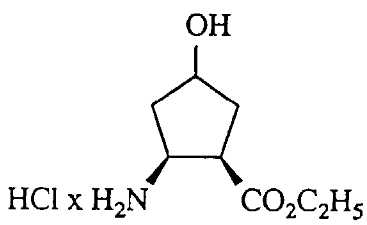

Eine Lösung der Verbindung aus Beispiel 7 (1,10 g, 4,0 mmol) in 6 ml 4N HCl in Dioxan wird 1 h bei Raumtemperatur gerührt. Das Lösemittel wird im Vakuum abgezogen und der Rückstand 20 h bei 50°C / 0,1 Torr getrocknet.

Ausbeute: 0,82 g (97%)
Diastereomerenverhältnis $D_1$:$D_2$ = 3:1
MS (DEI): m/z = 174 (M+H).
$C_8H_{15}NO_3$ x HCl (173,2 x 36,5)

**Beispiel 9**

(+/-)-(1RS, 2SR)-1,2-cis-2-Amino-4-hydroxy-cyclopentan-1-carbonsäure Hydrochlorid

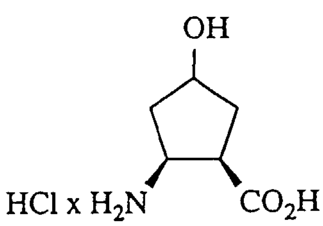

Eine Lösung der Verbindung aus Beispiel 8 (210 mg, 1,0 mmol) in 13 ml 3 N HCl wird 2 h bei 80°C gerührt. Das Lösemittel wird im Vakuum abgezogen und der Rückstand 20 h bei 50°C / 0,1 Torr getrocknet.

Ausbeute: 151 mg (83%)
Diastereomerenverhältnis $D_1$:$D_2$ = 3:1
MS (DEI): m/z = 146 (M+H)
$C_6H_{11}NO_3$ x HCl (145,2 x 36,5)

**Beispiel 10**

(+/-)-(1RS, 2SR)-1,2-cis-2-Benzylamino-4,4-dimethyl-cyclopentan-1-carbonsäuremethylester

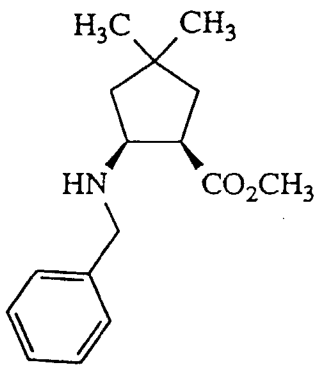

Eine Lösung der Verbindung aus Beispiel VI (8,15 mmol) in 70 ml EtOH wird mit 1 g Platin (5% auf Aktivkohle) versetzt und 20 h bei 35°C und 80 bar $H_2$ hydriert. Der Katalysator wird abfiltriert, das Filtrat im Vakuum eingeengt und der Rückstand an Kieselgel chromatographiert.

Ausbeute: 2,13 g (51%)
$R_f$ = 0,49 (Essigester / Petrolether = 1:2)
$C_{16}H_{23}NO_2$ (261,3)

**Beispiel 11**

(+/-)-(1RS, 2SR)-1,2-cis-2-Benzylamino-5-methyl-cyclopentan-1-carbonsäuremethylester

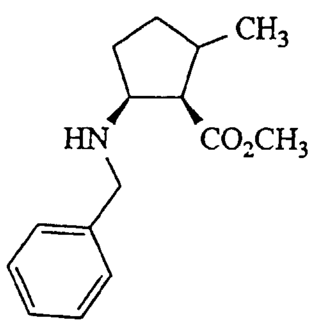

In Analogie zur Vorschrift des Beispiels 10 wird die Titelverbindung hergestellt.

Ausbeute: Diastereomer $D_1$: 0,67 g (33%)
$R_f$ = 0,49 (Essigester / Petrolether = 1:2)
Diastereomer $D_2$ = 0,59 (29%)
$R_f$ = 0,34 (Essigester / Petrolether = 1:2)
$C_{15}H_{21}NO_2$ (247,34)

**Beispiel 12**

(+/-)-(1RS, 2SR)-1,2-cis-2-Benzylamino-3-methyl-cyclopentan-1-carbonsäuremethylester

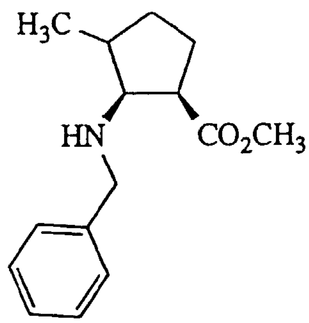

In Analogie zur Vorschrift des Beispiels 10 wird die Titelverbindung hergestellt.

Ausbeute: 1,41 g (71%)
2 Diastereomeren $D_1$:$D_2$ = 4:1
$R_f$ = 0,49 und 0,31 (Essigester / Petrolether = 1:4)
$C_{15}H_{21}NO_2$ (247,34)

**Beispiel 13**

(+/-)-(1RS, 2SR)1,2-cis-2-Amino-4,4-dimethyl-cyclopentan-1-carbonsäuremethylester Hydrochlorid

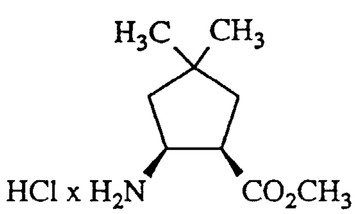

Eine Lösung der Verbindung aus Beispiel 10 (7,70 mmol) in 77 ml 0,1 N HCl (7,70 mmol), 80 ml $H_2O$ und 110 ml EtOH wird mit 710 mg Palladium (10% auf Aktivkohle) versetzt und 20 h bei Raumtemperatur und 3 bar $H_2$ hydiert Der Katalysator wird abfiltriert, das Filtrat im Vakuum eingeengt und der Rückstand 12 h bei 50°C / 0,1 Torr getrocknet.

Ausbeute: 1,52 g (95%)
Smp.: 148°C
$C_9H_{17}NO_2$ x HCl (171,2 x 36,5)

**Beispiel 14**

(+/-)-(1RS, 2SR)-1,2-cis-2-Amino-5-methyl-cyclopentan-1-carbonsäuremethylester Hydrochlorid

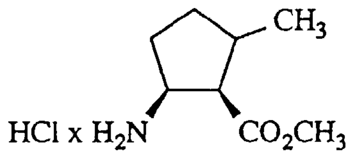

In Analogie zur Vorschrift des Beispiels 10 wird die Titelverbindung erhalten.

Ausbeute: Diastereomer A: 1,43 g (96%)
Smp.: 169°C
Diastereomer B: 1,46 g (98%)
Smp.: 64°C
$C_8H_{15}NO_2$ x HCl (157,2 x 36,5)

**Beispiel 15**

(+/-)-(1RS, 2SR)-1,2-cis-2-Amino-3-methyl-cyclopentan-1-carbonsäuremethylester Hydrochlorid

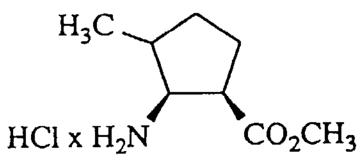

In Analogie zur Vorschrift des Beispiels 10 wird die Titelverbindung erhalten.

Ausbeute: 1,50 g (100%)
2 Diastereomere: $D_1$:$D_2$ = 4:1
$R_F$ = 0,45 (Ether / Acetonitril / konz. $NH_3$ = 10:1:0,1)
$C_8H_{15}NO_2$ x HCl (157,2 x 36,5)

**Beispiel 16**

(+/-)-(1RS, 2SR)1,2-cis-2-Amino-4,4-dimethyl-cyclopentan-1-carbonsäure Hydrochlorid

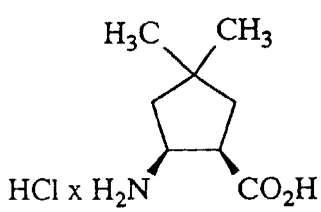

Eine Lösung der Verbindung aus Beispiel 13 (4,20 mmol) in 70 ml 3 N HCl wird 2 h unter Rückfluß erhitzt Das Solvens wird im Vakuum abgezogen, der Rückstand wird mit THF gewaschen und 20 h bei 50°C / 0,1 Torr getrocknet.

Ausbeute: 0,81 g (100%)
Smp.: 190°C (Zers.)
$C_8H_{15}NO_2$ x HCl (157,2 x 36,5)

**Beispiel 17a und 17b**

(+/-)-(1RS, 2SR)-1,2-cis-2-Amino-5-methyl-cyclopentan-1-carbonsäure Hydrochlorid

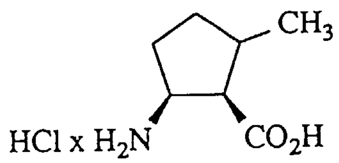

In Analogie zur Vorschrift des Beispiels 16 wird die Titelverbindung hergestellt.

Ausbeute: Diastereomer A: 0,61 (81%) (Beispiel 17a)
Smp.: 134°C
Diastereomer B: 0,73 g (97%) (Beispiel 17b)
Smp.: 200°C (Zers.)
$C_7H_{13}NO_2$ x HCl (143,2 x 36,5)

**Beispiel 18**

(+/-)-(1RS, 2SR)-1,2-cis-2-Amino-3-methyl-cyclopentan-1-carbonsäure Hydrochlorid

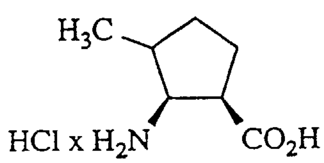

In Analogie zur Vorschrift des Beispiels 16 wird die Titelverbindung hergestellt.

Ausbeute: 0,68 g (90%)
Diastereomerenverhältnis $D_1$:$D_2$ = 4:1
Smp.: 206°C
$C_7H_{13}NO_2$ x HCl (143,2 x 36,5)

**Beispiel 19**

(+/-)-(1RS, 2SR)-1,2-cis-2-Amino-2-methyl-cyclopentan-1-carbonsäure Hydrochlorid

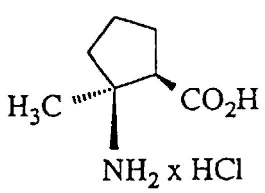

15 g (0,12 mol) 2-Methyl-6-azabicyclo[3.2.0]heptan-7-on [vgl. T. Sasaki et al., Tetrahedron 32, 437 (1976)] werden portionsweise in 100 ml konzentrierter Salzsäure suspendiert, und die Suspension bis zum Klarpunkt bei 40°C gerührt.

Die Lösung wird 1 x mit Diethylether extrahiert und die wäßrige Phase zur Trockene eingeengt. Nach Trocknen im Hochvakuum werden 20,7 g (96%) eines weißen Feststoffs erhalten.

Smp.: 194°C
$C_7H_{13}NO_2$ x HCl (143 x 36,5)

**Beispiel 20**

(+/-)-(1RS, 2SR)-1,2-cis-2-Amino-4-methyl-cyclopentan-1-carbonsäure Hydrochlorid

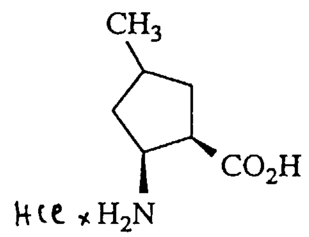

3 g (0,024 mol) 4-Methyl-6-azabicyclo[3.2.0]heptan-7-on [Darstellung: T. Sasaki et al., Tetrahedron 32, (1976)] werden zusammen mit 15 ml konz. Salzsäure 2 h bei Raumtemperatur gerührt. Nach Ausethern der Lösung engt man zur Trockene ein. Der Rückstand wird bei 40°C im Hochvakuum getrocknet.

Ausbeute: 2,9 g (67%)
Smp.: 188,5°C
$C_7H_{13}NO_2$ x HCl (143 x 36,5)

**Beispiel 21**

(+/-)-(1RS, 2SR)-1,2-cis-2-N-(9-Fluorenylmethyloxycarbonyl)amino-4-methylen-cyclopentan-1-carbonsäure

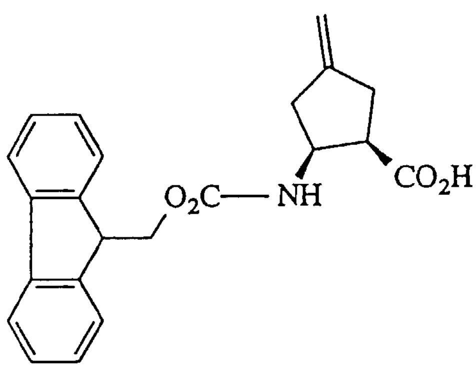

Zur Lösung von Beispiel 2 (0,500 g, 2,81 mmol) in 20 ml 10%iger wäßriger $Na_2CO_3$-Lösung tropft man bei 0°C eine Lösung von N-(9-Fluorenylmethyloxycarbonyloxy)-succinimid (0,995 g, 2,95 mmol) in 10 ml Dioxan. Die Mischung wird 12 h bei Raumtemperatur gerührt und dreimal mit je 10 ml Ether extrahiert Die wäßrige Phase wird bei 0°C mit konz. Salzsäure auf pH 2 eingestellt und zweimal mit jeweils 40 ml Ether extrahiert. Die Etherphasen werden über $Na_2SO_4$ getrocknet und das Lösemittel im Vakuum abgezogen.

Ausbeute: 0,940 g (92%)
Smp.: 137°C
$C_{22}H_{21}NO_4$ (363,4)

**Beispiel 22 und Beispiel 23**

(+/-)-(1RS, 2SR)-1,2-cis-2-N-(9-Fluorenylmethyloxycarbonyl)amino-4-methyl-cyclopent-3-en-1-carbonsäure (Beispiel 22)

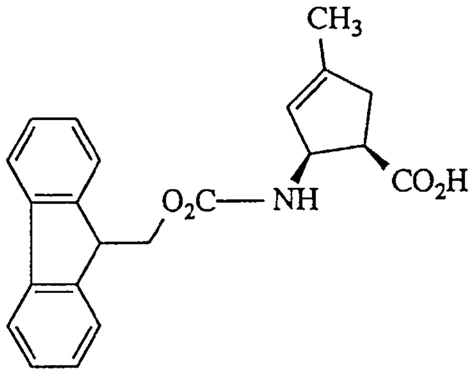

(22)

(+/-)-(1RS, 2SR)-1,2-cis-2-N-(9-Fluorenylmethyloxycarbonyl)amino-4-methyl-cyclopent-4-en-1-carbonsäure (Beispiel 23)

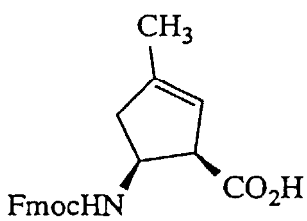

(23)

Eine Lösung der Verbindung aus Beispiel 2 (0,870 g, 4,90 mmol) in 20 ml 10%iger Salzsäure wird 20 h bei Raumtemperatur gerührt und anschließend i. Vak. eingedampft. Der Rückstand wird in 25 ml 10%iger $Na_2CO_3$-Lösung gelöst und bei 0°C mit einer Lösung von N-(9-Fluorenylmethyloxycarbonyloxy)-succinimid (1,65 g, 4,30 mmol) in 15 ml Dioxan versetzt Man läßt 48 h bei Raumtemperatur rühren, gibt 50 ml $H_2O$ hinzu und extrahiert zweimal mit je 20 ml Ether. Die wäßrige Phase wird bei 0°C auf pH 2 eingestellt und zweimal mit je 50 ml Ether extrahiert. Die Etherphasen werden über $Na_2SO_4$ getrocknet, das Lösemittel wird im Vakuum abgezogen und der Rückstand wird an Kieselgel chromatographiert (Methylenchlorid / Methanol = 20:1).

Ausbeute: 0,211 g (12%) (Beispiel 22)
$R_f$ = 0,31 (Methylenchlorid / Methanol 20:1) (Beispiel 22)
Ausbeute: 0,187 g (11%) (Beispiel 23)
$R_f$ = 0,28 (Methylenchlorid / Methanol = 20:1) (Beispiel 23)
$C_{22}H_{21}NO_4$ (363,4)

**Beispiel 24**

(+/-)-(1RS, 2SR)-1,2-cis-2-Amino-4-methyl-cyclopent-4-en-1-carbonsäure Hydrochlorid

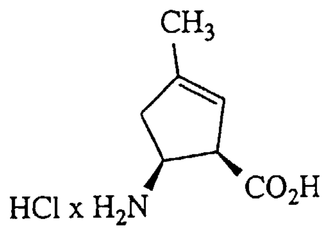

Eine Lösung aus Beispiel 2 (0,870 g, 4,90 mmol) in 20 ml 10 %ige Salzsäure wird 20 h bei Raumtemperatur gerührt und anschließend im Vakuum eingedampft. Der Rückstand wird in 8 ml Ethanol gelöst, mit 15 ml Ether versetzt und 5 d bei 5°C stehen gelassen. Ausgefallenes Produkt wird abgesaugt und mit Ether gewaschen.

Ausbeute: 0,246 g (28%)
Smp.: 196°C
$C_7H_{11}NO_2$ x HCl (141,2 x 36,5)

**Beispiel 25**

(+/-)-(1RS, 2SR)-1,2-cis-2-Amino-4-methyl-cyclopent-4-en-1-carbonsäure

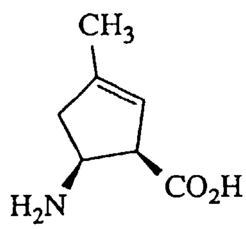

Eine Lösung aus Beispiel 23 (0,380 g, 1,05 mmol) in 30 ml flüssigem Ammoniak wird 10 h gerührt. Man läßt den Ammoniak abdampfen, versetzt mit 50 ml Ether, läßt 1 h bei Raumtemperatur rühren, filtriert und wäscht den Rückstand mit 20 ml Ether. Der Rückstand wird in 5 ml Wasser aufgenommen und 10 min gerührt. Man filtriert, wäscht mit 3 ml Wasser und engt das Filtrat im Vakuum ein. Der Rückstand wird aus 80%igem wäßrigem Ethanol umkristallisiert.

Ausbeute: 0,082 g (55%)
Smp.: 190°C
$C_7H_{11}NO_2$ (141,2)

**Beispiel 26**

(+/-)-(1RS, 2SR)-1,2-cis-2-Amino-4-methyl-cyclopent-3-en-1-carbonsäure

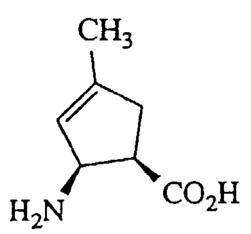

Eine Lösung aus Beispiel 22 (0,410 g, 1,13 mmol) in 30 ml flüssigem Ammoniak wird 10 h gerührt. Man läßt den Ammoniak abdampfen, versetzt mit 50 ml Ether, läßt 1 h bei Raumtemperatur rühren, filtriert und wäscht den Rückstand mit 20 ml Ether. Der Rückstand wird in 5 ml Wasser aufgenommen und 10 min gerührt. Man filtriert, wäscht mit 3 ml Wasser und engt das Filtrat im Vakuum ein. Der Rückstand wird aus 80%igem wäßrigen Ethanol umkristallisiert.

Ausbeute: 0,112 g (70%)
Smp.: 221°C
$C_7H_{11}NO_2$ (141,2)

**Beispiel 27**

(+/-)-(1RS, 2SR)-1,2-cis-2-Amino-4-methyl-cyclopent-3-en-1-carbonsäure Hydrochlorid

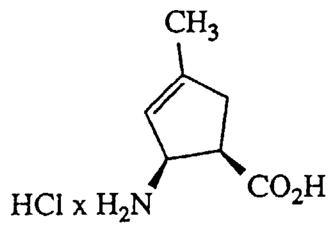

Die Verbindung aus Beispiel 2 (0,110 g, 0,78 mmol) wird in 7,80 ml (0,78 mmol) 0,1 N HCl gelöst. Die Lösung wird anschließend im Vakuum eingedampft.

Ausbeute: 0,138 g (100%)
Smp.: 188°C (Zers.)
$C_7H_{11}NO_2$ x HCl (141,2 x 36,5)

**Beispiel 28**

(+)-(1R*, 2S*)-1,2-cis-2-(9-Fluorenylmethyloxycarbonyl)amino-4-methylen-cyclopentan-1-carbonsäure (R)-Phenethylammoniumsalz

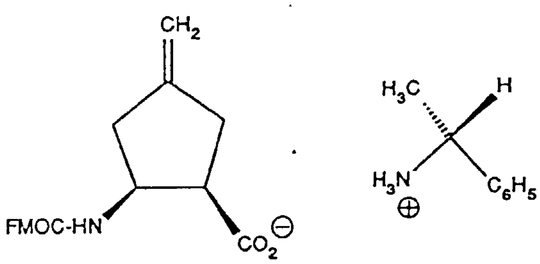

Eine Lösung aus Beispiel 21 (10,0 g, 27,5 mmol) in 4,5 ml tert.-Butylmethylether und 15 ml EtOH wird mit (R)-(+)-Phenethylamin (3,33 g, 27,5 mmol) versetzt. Man erhitzt die Mischung unter Rückfluß und tropft ca. 80 ml EtOH hinzu, bis eine klare Lösung entsteht. Man läßt über Nacht langsam auf Raumtemperatur abkühlen, saugt ausgefallenes Roh-Produkt ab und wäscht mit 20 ml tert.-Butylmethylether/EtOH (3:1). Das Rohprodukt wird anschließend noch einmal aus 30 ml tert.-Butylmethylether und 70 ml Ethanol umkristallisiert.

Ausbeute: 3,49 g (26%)
Smp.: 163°C
$[\alpha]\ D^{20} = +\ 17,1$ (c = 1, MeOH)
$C_{20}H_{21}NO_4$ x $C_8H_{11}N$ (363,4 x 121,2)

**Beispiel 29**

(-)-(1S*, 2R*) -1,2-cis-2-(9-Fluorenylmethyloxycarbonyl)amino-4-methylen-cyclopentan-1-carbonsäure (S)-Phenethylammoniumsalz

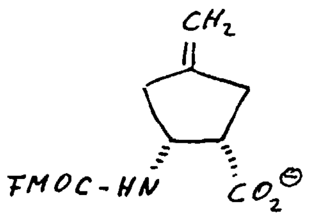

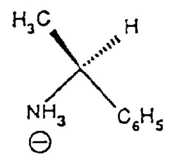

Die Herstellung erfolgt analog wie in Beispiel 28 beschrieben mit (S)-Phenethylamin anstatt (R)-Phenethylamin.

Ausbeute: 3,48 g (26%)
Smp.: 165°C
$[\alpha]^{20}_D$ = 17,8 (c = 0,73, MeOH)
$C_{20}H_{21}NO_4$ x $C_8H_{11}N$ (363,4 x 121,2)

**Beispiel 30**

(-)-(1R*, 2S*)-1,2-cis-2-(9-Fluorenylmethyloxycarbonyl)amino-4-methylen-cyclopentan-1-carbonsäure

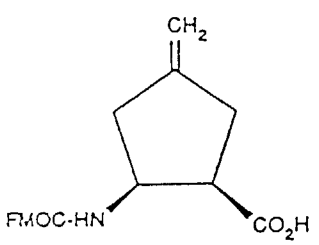

Beispiel 28 (3,49 g, 7,20 mmol) wird in 40 ml Wasser und 40 ml Essigester suspendiert. Man versetzt mit 1 N HCl bis pH2, trennt die Phasen und extrahiert die wäßrige Phase noch 2 x mit je 40 ml Essigester. Die vereinten organischen Phasen werden über $Na_2SO_4$ getrocknet und das Lösungsmittel wird i. Vakuum abgezogen.

Ausbeute: 2,46 g (94%)
$[\alpha]^{20}_D$ = -18,8(c=1,MeOH)
Smp.: 137°C
Enantiomerenüberschuß e.e. = 99,5 % (HPLC, Chiralpak AS)
$C_{22}H_{21}NO_4$ (363,4)

**Beispiel 31**

(+)-(1S*, 2R*)-1,2-cis-2-(9-Fluorenylmethyloxycarbonyl)amino-4-methylen-cyclopentan-1-carbonsäure

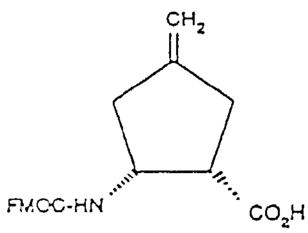

Die Herstellung erfolgt wie für Beispiel 30 beschrieben, ausgehend von Beispiel 29.

Ausbeute: 2,46 g (94%)
$[\alpha]^{20}_{D}$ = + 18,4 (c = 0,48 MeOH)
Smp.: 137°C
Enantiomerenüberschuß e.e. = 99,0 % (HPLC, Chiralpak AS)
$C_{22}H_{21}NO_4$ (363,4)

**Beispiel 32**

(-)-(1R*, 2S*)-1,2-cis-2-Amino-4-methylen-cyclopentan-1-carbonsäure Hydrochlorid

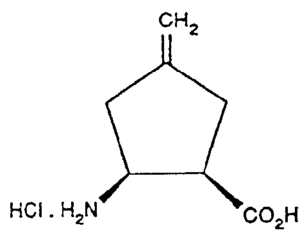

Man versetzt Beispiel 30 (1,35 g, 3,71 mmol) mit 100 ml flüssigem Ammoniak, läßt ca. 10 h rühren und anschließend den Ammoniak verdampfen. Der Rückstand wird mit 120 ml Ether versetzt, und man läßt 1 h rühren bei Raumtemperatur. Man filtriert und nimmt den Rückstand in 5 ml Wasser auf, filtriert erneut, wäscht den Rückstand mit 3 ml Wasser und engt das Filtrat i. Vak. ein. Der Rückstand wird aus 80 %igem wäßrigen Ethanol umkristallisiert. Die erhaltene freie Aminosäure (0,451 g, 3,19 mmol) wird mit IN HCl (31,9 ml, 3,19 mmol) versetzt und die entstandene Lösung wird im Vakuum eingeengt und der Rückstand im Vakuum bei 50°C/0,1 Torr getrocknet.

Ausbeute: 0,567 g (86%)
Smp.: 184°C, $[\alpha]^{20}_{D}$ = - 11,6 (c = 1, $H_2O$)
$C_7H_{11}NO_2$ x HCl (141,2 x 36,5)

**Beispiel 33**

(+)-(1S*, 2R*)-1,2-cis-2-Amino-4-methylen-cyclopentan-1-carbonsäure Hydrochlorid

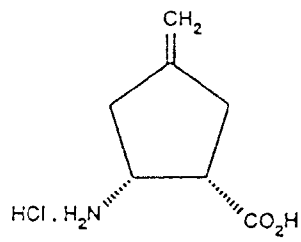

Die Herstellung erfolgt analog wie für Beispiel 32 beschrieben, ausgehend von Beispiel 31.

Ausbeute: 0,566 g (86%)
Smp.: 186°C
$[\alpha]^{20}_D$ = + 11,4 (c = 1,04, $H_2O$)
$C_7H_{11}NO_2$ x HCl (141,2 x 36,5)

**Beispiel 34**

(+/-)-(1RS, 2SR)-1,2-cis-2-Amino-4-ethyliden-cyclopentan-1-carbonsäure Hydrochlorid

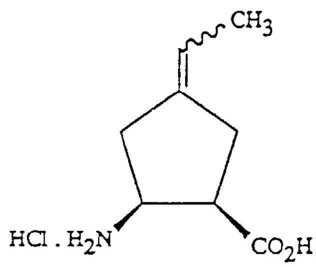

Die Verbindung aus Beispiel XI (0,30 g, 1,66 mmol) wird mit 0,1 N HCl (16,6 mmol, 1,66 mmol) versetzt Die Mischung wird 5 h gerührt bis eine klare Lösung entstanden ist. Das Solvens wird i.Vak. bei 30°C abgezogen und der Rückstand 12 h bei 30°C/0,1 Torr getrocknet.

Ausbeute: 0,32 g (100 *%)* eines Diastereomerengemisches
Smp.: 188°C
$C_8H_{13}NO_2$ x HCl (155,2 x 36,5)

**Beispiel 35**

(-)-(1R*, 2S*)-1,2-cis-2-(t-Butyloxycarbonyl)amino-4-methylen-1-carbonsäure

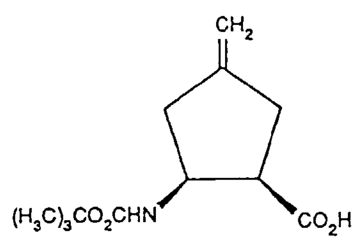

Eine Lösung der Verbindung aus Beispiel 32 (2,0 g, 11,3 mmol) in 20 ml Dioxan wird mit 16.8 ml einer 1 M $Na_2CO_3$-Lösung und bei 0°C mit Di-tert.-butyldicarbonat (2,68 g, 12,3 mmol) versetzt. Man läßt 20 h bei Raumtemperatur rühren, gibt 30 ml Essigester hinzu und bringt die Lösung mit 10 %iger Salzsäure auf pH2. Die wäßrige Phase wird noch zweimal mit je 30 ml Essigester extrahiert. Die vereinigten org. Phasen werden mit ges. NaCl-Lösung gewaschen, über $Na_2SO_4$ getrocknet und i. Vak. eingengt.

Ausbeute: 2,73 g (100%)
$[\alpha]^{20}_D$ = - 41,3 (c = 0,72, $CH_3OH$)
$C_{12}H_{19}NO_4$ (241,3)

**Beispiel 36**

(-)-(1R*, 2S*)-1,2-cis-2-(tert.-Butyloxycarbonyl)amino-4-methylen-cyclopentan-1-carbonsäureethylester

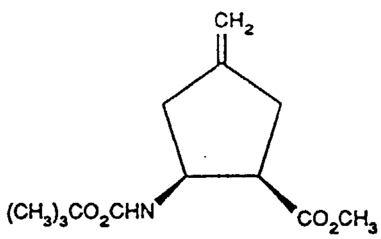

Zur Lösung der Verbindung aus Beispiel 35 (2,73 g, 11,3 mmol), 4-Dimethylaminopyridin (0,14 g, 1,1 mmol) und Methanol (1,09 g, 34 mmol) in 30 ml Dichlormethan tropft man bei O°C eine Lösung von Dicyclohexylcarbodiimid (2,57 g, 12,5 mmol) in 10 ml Dichlormethan. Man läßt 2 h bei Raumtemperatur rühren, saugt ausgefallenen Dicyclohexylharnstoff ab und wäscht mit 50 ml Dichlormethan. Das Filtrat wird mit 30 ml 0,1 NHCl und 30 ml ges. $NaHCO_3$-Lösung gewaschen, über $Na_2SO_4$ getrocknet und im Vakuum eingeengt. Der Rückstand wird an Kieselgel chromatographiert (Petrolether/Essigester = 3:1).

Ausbeute: 2,36 g (82%)
Smp.: 64°C
$[\alpha]^{20}_D$ = - 86,8 (c = 1,02, $CH_3OH$)
$C_{13}H_{21}NO_4$

**Beispiel 37**

(-)-(1R*, 2S*)-1,2-cis-2-Amino-4-methylen-cyclopentan-1-carbonsäuremethylester Hydrochlorid

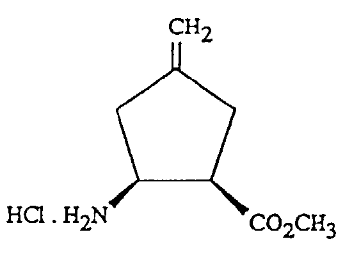

Zur Lösung der Verbindung aus Beispiel 36 (2,10 g, 8,20 mmol) und 2,6-Lutidin (1,76 g, 16,5 mmol) in 50 ml Dichlormethan tropft man bei Raumtemperatur unter Argon Trifluormethansulfonsäure-(tert.-butyldimethyl)silylester (3,27 g, 12,3 mmol). Man läßt 15 min rühren, versetzt mit 100 ml ges. $NH_4Cl$-Lösung und extrahiert zweimal mit je 100 ml Ether. Die org. Phasen werden mit ges. NaCl-Lösung gewaschen, über $MgSO_4$ getrocknet und im Vak. eingeengt. Der Rückstand wird in 50 ml THF gelöst und mit Wasser (0,30 g, 16,5 mmol) und Tetrabutylammoniumfluorid (1,1 M Lösung in THF, 7,5 ml, 8,2 mmol) bei O°C versetzt. Man läßt 1 h bei 0°C rühren, gibt 100 ml Wasser hinzu, bringt die

Lösung auf pH 9 mit konz. $NH_3$, gibt 15 g NaCl hinzu und extrahiert dreimal mit je 80 ml Essigester. Die org. Phasen werden über $MgSO_4$ getrocknet und im Vak. eingeengt. Der Rückstand wird an Kieselgel chromatographiert (Ether/Acetonitril/konz. $NH_3$ = 10:1:0,1).

Ausbeute: 1,15 g (73 %)
Smp.: 146°C/ $[\alpha]^{20}_D$ = - 4,2 (c = 1,23, $H_2O$)
$C_8H_{13}NO_2$ x HCl (155,2 x 36,5)

**Beispiel 38**

(-)-(1R*, 2S*)-1,2-cis-2-(N-(tert-Butyloxycarbonyl)-glycinyl)-amino-4-methylen-cyclopentan1-carbonsäure-methylester

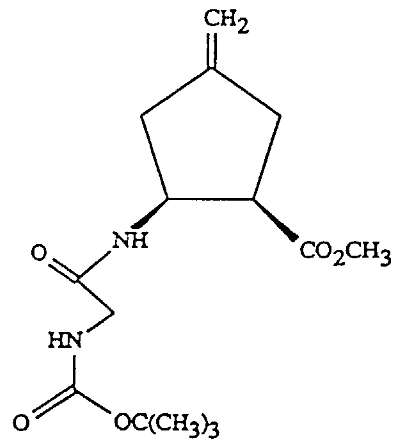

Zur Lösung der Verbindung aus Beipiel 36 (0,40 g, 2,09 mmol), 1-Hydroxy-1Hbenzotriazol x $H_2O$ (0,282 g, 2,09 mmol), N-Ethylmorpholin (0,261 g, 2,09 mmol) und N-(tert.-Butyloxycarbonyl)glycin (0,366 g, 2,09 mmol) in 18 ml THF tropft man unter Argon bei 0°C eine Lösung von Dicyclohexylcarbodiimid (0,430 g, 2,09 mmol) in 2 ml THF. Man läßt 1 h bei 0°C und 20 h bei Raumtemperatur rühren, filtriert, wäscht mit 10 ml THF und engt das Filtrat i.Vak. ein. Der Rückstand wird in 40 ml Essigester gelöst, mit 20 ml ges. $NaHCO_3$-Lösung und 20 ml ges. NaCl-Lösung gewaschen, über $Na_2SO_4$ getrocknet und i. Vak. eingeengt.

Ausbeute: 0,585 g (100%)
$^1$H-NMR ($d_6$-DMSO): $\alpha$ = 1,38 (s, 5H); 2,18-2,73 (m, 4H); 3,08 (dt, 1H); 3,18 und 3,46 (AB von ABX, 2H); 3,57 (s, 3H); 4,40 (ddt, 1H); 4,90 (m, 2H); 6,88 (X von ABX, 1H), 7,71 (d, 1H)
$C_{15}H_{24}N_2O_5$ (312,4)

**Beispiel 39**

(-)-(1R*, 2S*)-1,2-cis-2-(N-(tert.-Butyloxycarbonyl)(S)-alanyl)amino-4-methylen-cyclopentan1 -carbonsäuremethylester

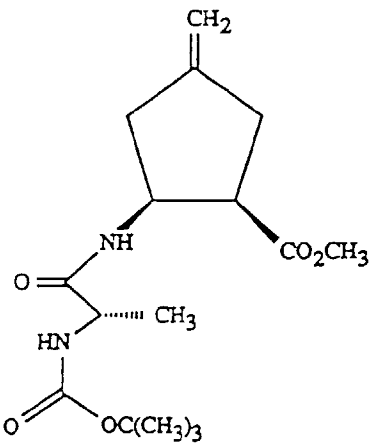

Die Titelverbindung wird in Analogie zur Vorschrift des Beispiels 38 hergestellt.

Ausbeute: 0,630 g (86 %)
$^1$H-NMR ($d_6$-DMSO): $\alpha$ = 1,09 (d, 3H), 1,36 (s, 9H), 2,22-2,72 (m, 4H), 3,10 (dt, 1H), 3,52 (s, 3H), 3,95 (dq, 1H), 4,40 (ddt, 1H), 4,90 (cm, 2H), 6,78 (d, 1H) 7,83 (d, 1H).
$C_{16}H_{26}N_2O_5$ (326,4)

**Beispiel 40**

(-)-(1R*, 2S*)-1,2-cis-2-(N-glycinyl)amino-4-methylen-cyclopentan-1-carbonsäuremethylester Hydrochlorid

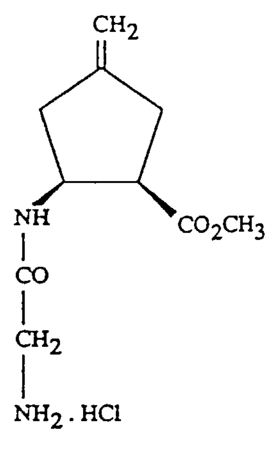

Zur Lösung der Verbindungen aus Beispiel 38 (0,52 g, 1,66 mmol) und 2,6-Lutidin (0,59 g, 5,50 mmol) in 10 ml Dichlormethan tropft man bei 0°C unter Argon Trifluormethansulfonsäure-(tert.-butyldimethyl)silylester (1,10 g, 4,15 mmol) und läßt noch 20 h bei Raumtemperatur rühren. Man versetzt mit 20 ml ges. $NH_4Cl$-Lösung, extrahiert zweimal mit je 50 ml Ether, wäscht die org. Phase mit ges. NaCl-Lösung, trocknet über $MgSO_4$ und zieht das Solvens i.Vak. ab. Der Rückstand wird in 10 ml THF aufgenommen, mit Wasser (0,06 g, 3,3 mmol) versetzt und eine 1,1 M Lösung im Tetrabutylammoniumfluorid in THF (3,0 ml, 3,3 mmol) wird bei 0°C zugetropft. Man läßt 1 h bei 0°C rühren, versetzt mit 20 ml Wasser und bringt die Lösung mit konz. $NH_3$ auf pH 9. Es werden 4 g NaCl zugegeben, dreimal mit je 20 ml Essigester extrahiert, die org. Phasen mit ges. NaCl-Lösung gewaschen, über $MgSO_4$ getrocknet und i. Vak. eingeengt. Der Rückstand wird an Kieselgel chromatographiert (Essigester/MeOH/konz. $NH_3$ = 10:1:0). Die so erhaltene freie

Base wird in 10 ml 0,1 N HCl aufgenommen und das Solvens wird i. Vak. abgezogen. Der Rückstand wird 12 h bei 30°C/0,1 Torr getrocknet.

Ausbeute: 0,202 g (49 %).
$^1$H-NMR (DMSO): $\alpha$ = 2,25-2,72 (m, 4H), 3,12 (dt, 1H), 3,40-3,62 (m, 2H), 3,59 (s, 3H), 4,49 (ddt, 1H), 4,92 (cm, 2H), 8,05 (s, breit, 3H), 8,42(d, 1H).
$C_{10}H_{16}N_2O_3$ x HCl (212,2 x 36,5).

**Beispiel 41**

(-)-(1R*, 2S*)-1,2-cis-2-(N-(S)-Alanyl)amino-4-methylen-cyclopentan-1-carbonsäuremethylester Hydrochlorid

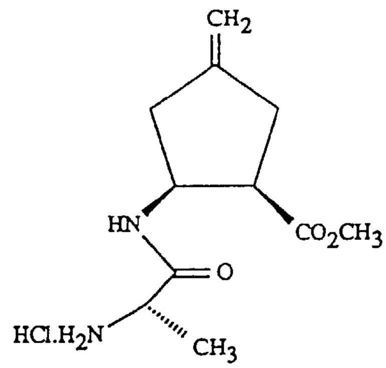

Die Titelverbindung wird in Analogie zur Vorschrift des Beispiels 40 hergestellt.

Ausbeute: 0,249 g (57 %)
$[\alpha]^{20}_D$ = - 66,3 (c = 1,1 $H_2O$)
$C_{11}H_{18}H_2O_3$ x HCl

**Beispiel 42**

(-)-(1R*, 2S*)-1,2-cis-2-[N-(9-Fluorenylmethyloxycarbonyl)-(S)-norvalinyl]amino-4methylen-cyclopentan-1-carbonsäure

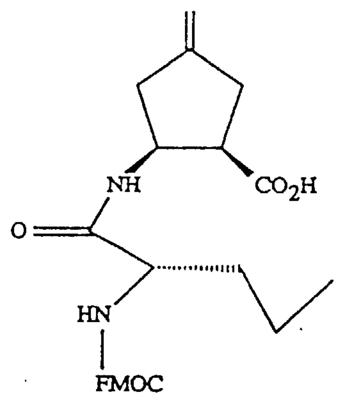

Zur Lösung der Verbindung aus Beispiel 32 (3,00 g, 16,9 mmol) und $NaHCO_3$ (2,84 g, 33,8 mmol) in 60 ml Wasser tropft man eine Lösung von FMOC-Norvalin-O-Succinimid (7,38 g, 16,9 mmol) in Dimethoxyethan (72 ml) und läßt über Nacht bei Raumtemperatur rühren. Man versetzt mit THF (180 ml) und bringt die Lösung mit 10 %iger Salzsäure auf pH 2. Man extrahiert mit Ether (3 x 300 ml), wäscht die vereinigten Etherphasen mit Wasser (100 ml) und ges. NaCl-Lösung (100 ml) und trocknet über $Na_2SO_4$. Das Lösungsmittel wird im Vakuum abgezogen und der Rückstand

wird an Kieselgel chromatographiert (Dichlormethan/Methanol = 10:1).

Ausbeute: 4,58 g (59 %)
Smp.: 124°C
$R_f$ = 0,43 ($CH_2Cl_2$/MeOH = 10:1)
$C_{27}H_{30}N_2O_5$ (462,54).

**Beispiel 43**

(-)-(1R*, 2S*)-1,2-cis-2-[N-(9-Fluorenylmethyloxycarbonyl)-(S)-norleucyl]amino-4-methylen-cyclopentan- -carbonsäure

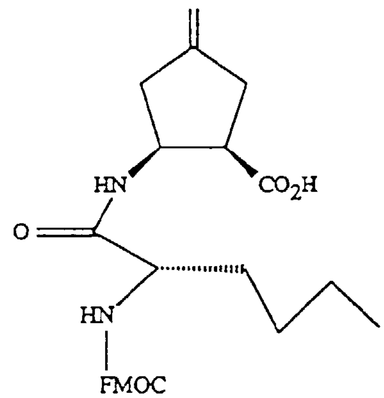

Die Titelverbindung wird in Analogie zur Vorschrift des Beispiels 42 hergestellt.

Ausbeute: 4,97 g (74 %)
Smp.: 151°C
$R_f$ = 0,45 ($CH_2Cl_2$/MeOH = 10:1)
$C_{28}H_{32}N_2O_5$ (476,57).

**Beispiel 44**

(-)-(1R*, 2S*)-1,2-cis-2-[N-(9-Fluorenylmethyloxycarbonyl)-(S)leucyl]amino-4-methylen-cyclopentan-1-carbonsäure

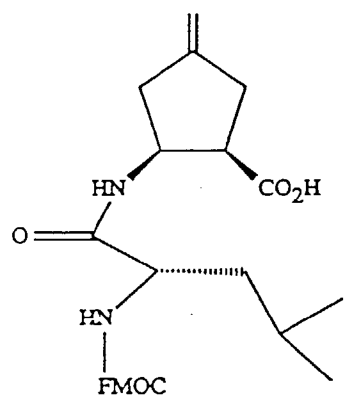

Die Titelverbindung wird in Analogie zur Vorschrift des Beispiels 42 hergestellt.

Ausbeute: 3,17 g (47 %)

$[\alpha]_D^{20}$ = -28,3 (c = 1,24, MeOH)
$R_f$ 0,21 ($CH_2Cl_2$/MeOH = 20:1)
$C_{28}H_{32}N_2O_5$ (476,57).

**Beispiel 45**

(-)-(1R*, 2S*)-1,2-cis-2-(N-(S)-Norvalinyl)amino-4-methylen-cyclopentan-1-carbonsäure Hydrochlorid

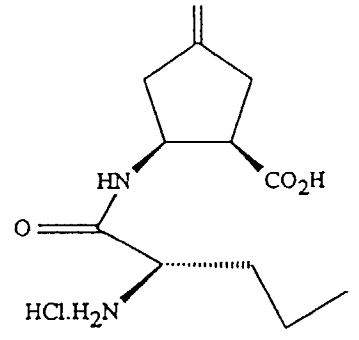

Man versetzt Beispiel 42 (4,53 g, 9,80 mmol) mit 150 ml flüssigem Ammoniak, läßt ca. 10 h rühren und anschließend den Ammoniak verdampfen. Der Rückstand wird mit 200 ml Ether versetzt, und man läßt 1 h bei Raumtemperatur rühren. Man filtriert und nimmt den Rückstand in 60 ml Wasser auf, filtriert erneut, wäscht den Rückstand mit 20 ml Wasser und engt das Filtrat i.Vak. ein. Der Rückstand wird in 89 ml 0,1 N Salzsäure gelöst, das Lösungsmittel wird i. Vak. abgezogen und der Rückstand wird über $P_2O_5$ i.Vak. getrocknet.

Ausbeute: 2,50 g (92 %)
Smp.: 130-135°C
$[\alpha]_D^{20}$ = -27,1 (c = 1,05, MeOH)
$C_{12}H_{20}N_2O_3$ x HCl (240,3 x 36,5).

**Beispiel 46**

(-)-(1R*, 2S*)-1,2-cis-2(N-(S)-Norleucyl)amino-4-methylen-cyclopentan-1-carbonsäure Hydrochlorid

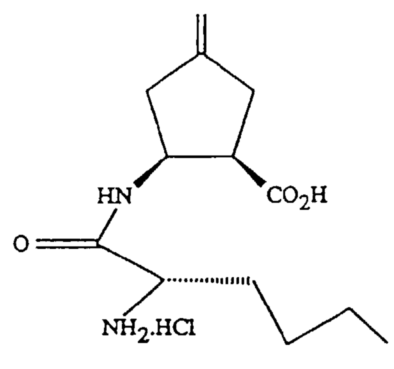

Die Titelverbindung wird in Analogie zur Vorschrift des Beispiels 45 hergestellt.

Ausbeute: 1,63 g (54 %)
Smp.: 108°C
$[\alpha]_D^{20}$ = -34,3 (c = 1,27, MeOH)

$C_{13}H_{22}N_2O_3$ x HCl (254,3 x 36,5).

**Beispiel 47**

(-)-(1R*, 2S*)-1,2-cis-2-(N-(S)-Leucyl)amino-4-methylen-cyclopentan-1-carbonsäure Hydrochlorid

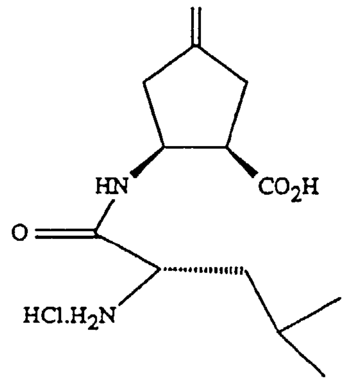

Die Titelverbindung wird in Analogie zur Vorschrift des Beispiels 45 hergestellt.

Ausbeute: 1,82 g (96 %)
Smp.: 70-80°C
$[\alpha]_D^{20}$ = -21,5 (c = 1,4, MeOH)
$C_{13}H_{22}N_2O_3$ x HCl (254,3 x 36,5).

**Beispiel 48**

(-)-(1R*, 2S*)-1,2-cis-2-(N-(tert.-Butyloxycarbonyl)-(S)-leucyl)amino-4-methylen-cyclopentan-1-carbonsäure-methylester

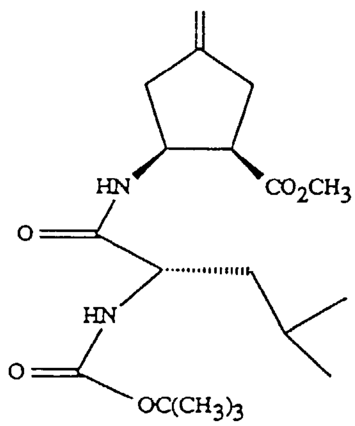

Die Titelverbindung wird in Analogie zur Vorschrift des Beispiels 38 hergestellt.

Ausbeute: 0,584 g (56 %)
Smp.: 124°C
$C_{18}H_{30}N_2O_5$ (354,5).

**Beispiel 49**

(-)-(1R*, 2S*)-1,2-cis-2-(N-(S)-Leucyl)amino-4-methylen-cyclopentan-1-carbonsäuremethylester Hydrochlorid

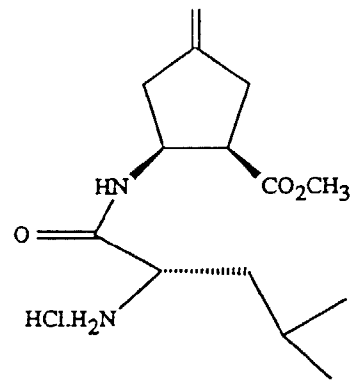

Die Titelverbindung wird in Analogie zur Vorschrift des Beispiels 40 hergestellt.

Ausbeute: 0,259 g (65 %)
Smp.: 70°C
$[\alpha]_D^{20}$ = -52,1 (c = 1,04, $H_2O$).
$C_{14}H_{24}N_2O_3$ x HCl (268,4 x 36,5).

**Beispiel 50**

(+/-)-(1RS, 2SR)-1,2-cis-2-Amino-4-benzyliden-cyclopentan-1-carbonsäure Hydrochlorid

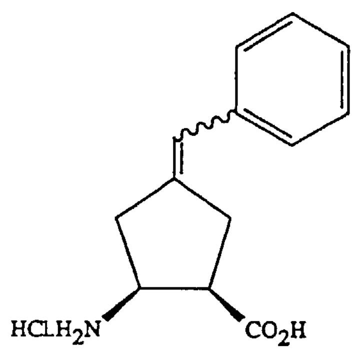

Eine Lösung der Verbindung aus Beispiel XIII (12,53 g, 50,9 mmol) in 43 ml Propionsäureanhydrid wird 3 h unter Rückfluß erhitzt Das Lösemittel wird bei 60°C/0,5 Torr abgezogen, der Rückstand 30 min bei 100°C/0,1 Torr getrocknet und in 60 ml Dioxan gelöst. Nach Zugabe von Trimethylsilylazid (6,84 g, 59,4 mmol) wird das Reaktionsgemisch 2 h auf 80°C erhitzt. Das Solvens wird i.Vak. abgezogen, der Rückstand wird in 200 ml Ether aufgenonunen und mit 0,78 g (43,4 mmol) Wasser versetzt. Man läßt 30 min kräftig rühren und bewahrt die Mischung 2 d bei 5°C auf. Der Niederschlag wird abgesaugt und verworfen. (Der Niederschlag besteht überwiegend aus Beispiel XIII). Das Filtrat wird auf ein Volumen von ca. 50 ml eingeengt und 12 h auf 0°C gekühlt, wobei 6-Benzyliden-cyclopentano[3,4]oxazin-2,4-(1H)-dion (2,52 g) auskristallisiert, man saugt die Kristalle ab, wäscht mit wenig Ether und versetzt den Feststoff mit 103 ml 0,1 N Salzsäure. Man läßt 1 h bei Raumtemperatur rühren, filtriert und engt das Filtrat i.Vak. ein. Der Rückstand wird über $P_4O_{10}$ i.Vak. getrocknet.

Ausbeute: 0,95 g (7 %) eines 5:1 E/Z-Isomerengemisches
Smp.: 234°C
$^1$H-HMR (DMSO-$d_6$): $\delta$ = 2,70-3,55 (m, 5H), 3,70 (cm, 1H), 6,48 (s, 1H), 7,12-7,42 (m, 5H)

$C_{13}H_{15}NO_2$ x HCl (217,3 x 36,5).

**Beispiel 51**

(+/-)-(1RS, 2SR)-1,2-cis-2-Amino-4-difluormethylen-cyclopentan-1-carbonsäure-Hydrochlorid

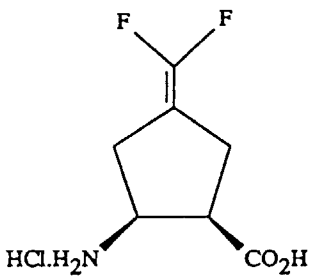

Die Titelverbindung wird in Analogie zur Vorschrift des Beispiels 2 hergestellt.

Ausbeute: 1,26 g (96 %)
Smp.: 215°C (Zers.)
$C_7H_9F_2NO_2$ x HCl (177,2 x 36,5)

**Beispiel 52**

(+/-)-(1RS, 2SR)-1,2-cis-2-Amino-4,4-difluor-cyclopentan-1-carbonsäure-Hydrochlorid

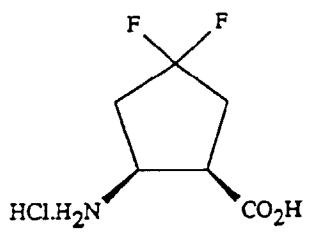

Die Titelverbindung wird in Analogie zur Vorschrift des Beispiels 2 hergestellt.

Ausbeute: 1,85 g (83 %)
Smp.: 222°C (Zers.)
$C_6H_9F_2NO_2$ x HCl (165,1 x 36,5)

**Beispiel 53**

(-)-(1R*, 2S*)-1,2-cis-2-Amino-4-methyl-cyclopentan-1-carbonsäure-Hydrochlorid

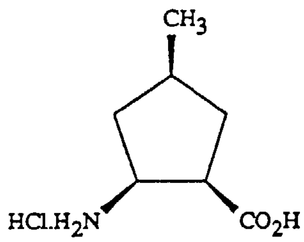

Eine Lösung des Beispiels 32 (400 mg, 2,25 mmol) in 30 ml EtOH und 5 ml $H_2O$ wird in Gegenwart von 50 mg Palladium auf Aktivhöhle (10 %) bei 3 bar und Raumtemperatur 3 h hydriert Man filtriert durch Kieselgur und engt das Filtrat im Vakuum ein. Der Rückstand wird 12 h bei 25°C/0,1 mbar getrocknet.

Ausbeute: 396 mg (98 %) eines 5:1-Diastereomerengemisches an C-4
Smp.: 156°C
$C_7H_{13}NO_2$ x HCl (143,2 x 36,5)

**Beispiel 54**

(-)-(1R*, 2S*)-1,2-cis-2-Amino-4-ethyl-cyclopentan-1-carbonsäure-Hydrochlorid

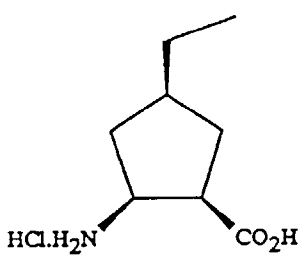

Die Titelverbindung wird in Analogie zur Vorschrift des Beispiels 53 ausgehend von Beispiel 34 hergestellt.

Ausbeute: 140 g (93 %) eines 16:1 Diastereomerengemisches
Smp.: 205°C (Zers.)
$C_8H_{15}NO_2$ x HCl (157,2 x 36,5)

**Beispiel 55**

(+/-)-(1RS, 2SR)-1,2-cis-2-Amino-4-benzyl-cyclopentan-1-carbonsäure-Hydrochlorid

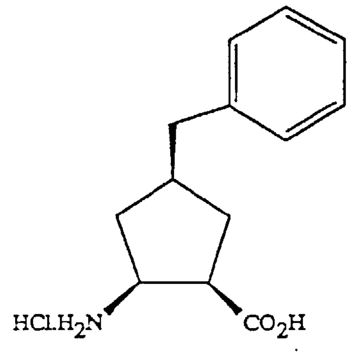

Die Titelverbindung wird in Analogie zur Vorschrift des Beispiels 53 ausgehend von Beispiel 50 hergestellt.

Ausbeute: 198 mg (90 %) eines 3:1 Diastereomerengemisches an C-4
Smp.: 104°C (Zers.)
$C_{13}H_{17}NO_2$ x HCl (219,3 x 36,5)

**Beispiel 56**

(+/-)-(1RS, 2SR)-1,2-cis-2-N-(tert.-Butyloxycarbonyl)amino-4-methylen-cyclopentan-1-carbonsäure

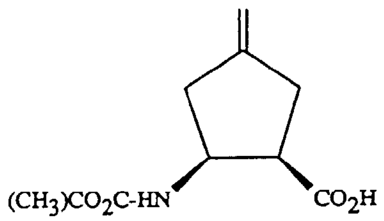

Eine Lösung der Verbindung aus Beispiel 2 (30,0 g, 170 mmol) in 350 ml Dioxan und 252 ml 1N $Na_2CO_3$-Lösung wird bei 0°C mit Di-tert.-butyldicarbonat (40,5 g, 185 mmol) versetzt und 16 h bei Raumtemperatur gerührt. Das Dioxan wird im Vakuum abgezogen und der wäßrige Rückstand wird mit 200 ml Essigester versetzt. Durch Zugabe von 1N wäßrige $KHSO_4$-Lösung wird der pH-Wert der wäßrigen Phase auf pH 2-3 gestellt. Die organische Phase wird abgetrennt und die wäßrige Phase wird mit Essigester extrahiert (2 x 200 ml). Die vereinten organischen Phasen werden mit Wasser gewaschen (2 x 100 ml), über $Na_2SO_4$ getrocknet und im Vakuum eingeengt.

Ausbeute: 38,3 g (93 %)
$^1$H-NMR (DMSO-$d_6$): $\delta$ = 1,39 (s, 9H), 2,30-2,72 (m, 4H), 3,00 (dt, 1H), 4,12 (dt, 1H), 4,85 (s, 2H), 6,78 (d, 1H), 12,08 (s, 1H)
$C_{12}H_{19}NO_4$ (214,3)

**Beispiel 57**

(+/-)-(1RS, 2SR)1,2-cis-2-N-(tert.-Butyloxycarbonyl)amino-4-methylen-cyclopentan-1-carbonsäure methylester

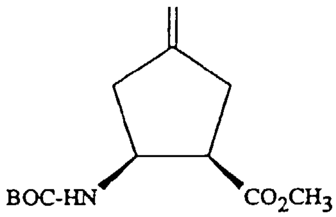

Eine Lösung der Verbindung aus Beispiel 56 (54,5 g, 226 mmol), Methanol (21,8 g, 680 mmol) und 4-(N,N-Dimethylamino)pyridin (2,76 g, 22,6 mmol) in 600 ml $CH_2Cl_2$ wird bei 0°C langsam mit einer Lösung von Dicyclohexylcarbodiimid (51,4 g, 250 mmol) in 200 ml $CH_2Cl_2$ versetzt. Nach 2 h Rühren bei Raumtemperatur wird filtriert, das Filtrat wird mit 0,1N HCl (300 ml), ges. $NaHCO_3$-Lösung (300 ml) und Wasser (300 ml), gewaschen, getrocknet ($Na_2SO_4$) und im Vakuum eingeengt. Der Rückstand wird an Kieselgel chromatographiert (Petrolether/Essigester = 3:1)

Ausbeute: 42,0 g (73 %)
Smp.: 55°C
$R_f$ = 0,30 (Petrolether/Essigester = 3:1)
$C_{13}H_{21}NO_4$ (255,3)

**Beispiel 58**

(+/-)-(1RS, 2SR)-1,2-cis-2-N-(tert.-Butyloxycarbonyl)amino-4-oxo-cyclopentan-1-carbonsäuremethyl ester

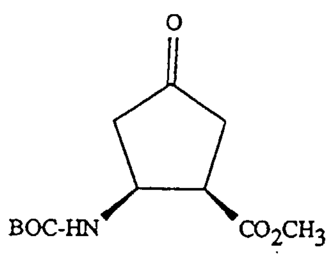

Die Titelverbindung wird in Analogie zur Vorschrift des Beispiels 4 ausgehend von Beispiel 57 hergestellt.

Ausbeute: 34,4 g (92 %)
Smp.: 135°C
$C_{12}H_{19}NO_5$ (257,3)

**Beispiel 59**

(+/-)-(1RS, 2SR)-1,2-cis-2-N-(tert.-Butyloxycarbonyl)amino-4-oximino-cyclopentan-1-carbonsäurem ethylester

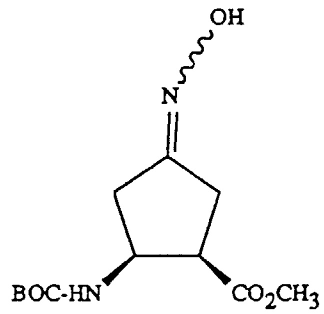

Eine Lösung des Beispiels 58 (500 mg, 1,94 mmol), Pyridin (0,80 ml, 9,80 mmol) und Hydroxylamin-Hydrochlorid (148 mg, 2,25 mmol) in 10 ml EtOH wird 20 h unter Rückfluß erhitzt. Das Lösungsmittel wird im Vakuum abgezogen, der Rückstand in Wasser (20 ml) aufgenommen und mit Ether extrahiert (3 x 20 ml). Die vereinten Etherphasen werden mit Wasser gewaschen (1 x 10 ml), getrocknet ($Na_2SO_4$) und im Vakuum eingeengt. Der Rückstand wird an Kieselgel chromatographiert (Diethylether).

Ausbeute: 269 mg (51 %)
$R_f$ = 0,67/0,71 (Diethylether)
$C_{12}H_{20}N_2O_5$ (272,3)

**Beispiel 60**

(+/-)-(1RS, 2SR)-1,2-cis-2-Amino-4-oximino-cyclopentan-1-carbonsäuremethylester-Hydrochlorid

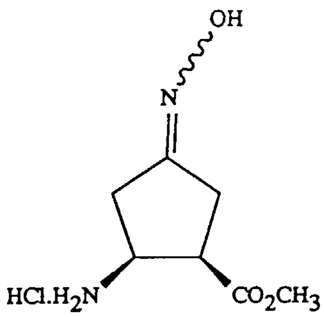

Die Titelverbindung wird in Analogie zur Vorschrift des Beispiels 5 ausgehend von Beispiel 59 hergestellt.

Ausbeute: 103 mg (54 %)
Smp.: 90-95°C (Zers.)
$C_7H_{12}N_2O_3$ x HCl (223,1 x 36,5)

**Beispiel 61**

(+/-)-(1RS, 2SR)-1,2-trans-2-N-(tert.-Butyloxycarbonyl)amino-4-methylen-cyclopentan-1-carbonsäuremethylester

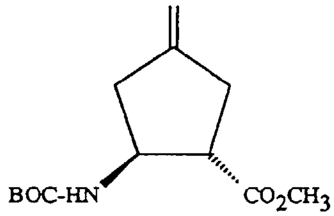

Eine Lösung des Beispiels 57 (1,00 g, 3,9 mmol) und 1,8-Diazabicyclo[5.4.0]-undec-7-en (0,90 g, 5,9 mmol) in 20 ml MeOH wird 12 h unter Rückfluß erhitzt. Das Lösungsmittel wird im Vakuum abgezogen, der Rückstand wird in Essigester (30 ml) aufgenommen, mit 1N HCl (10 ml) und Wasser (10 ml) gewaschen, getrocknet ($Na_2SO_4$) und im Vakuum eingeengt. Der Rückstand wird an Kieselgel chromatographiert (Petrolether/Essigester = 3:1).

Ausbeute: 520 mg (52 %)
Smp.: 140°C
$R_f$ = 0,26 (Petrolether/Essigester = 3:1)
$C_{13}H_{21}NO_4$ (255,3)

**Beispiel 62**

(+/-)-(1RS, 2SR)-1,2-trans-2-Amino-4-methylen-cyclopentan-1-carbonsäuremethylester

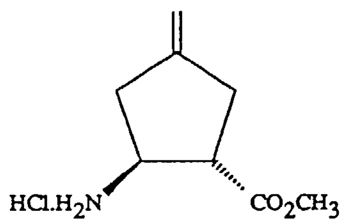

Die Titelverbindung wird in Analogie zur Vorschrift des Beispiels 37 ausgehend von Beispiel 61 hergestellt.

Ausbeute: 141 mg (30 %)
$^1$H-NMR (DMSO): $\delta$ = 2,30-2,50, 2,67-2,90 (2m, 4H), 3,08 (dt, 1H), 3,68 (s, 3H), 3,72 (dt, 1H), 4,95 (s, 2H), 8,40 (s, 3H)
$C_8H_{13}NO_2$ x HCl (155,2 x 36,5)

**Beispiel 63**

(+/-)-(1RS, 2SR)-1,2-cis-2-N-(tert.-Butyloxycarbonyl)amino-4-hydroxy-cyclopentan-1-carbonsäuremethylester

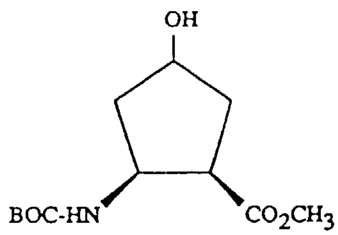

Die Titelverbindung wird in Analogie zur Vorschrift des Beispiels 7 ausgehend von Beispiel 58 hergestellt.

Ausbeute: 3,30 g (97 %) eines 3:1 Diastereomerengemisches
$^1$H-NMR ($CDCl_3$): δ = 1,45 (s, 9H), 3,14 und 3,32 (2dt, 1H), 3,68 und 3,70 (2s, 3H), 4,40 und 4,46 (2dt, 1H), 5,40 (d, 1H)
$C_{12}H_{21}NO_5$ (259,3)

**Beispiel 64**

(+/-)-(1RS, 2SR)-1,2-cis-2-N-(tert.-Butyloxycarbonyl)amino-4-cyclopenten-1-carbonsäuremethylester

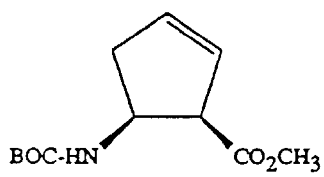

Eine Lösung von Beispiel IV (3,90 g, 8,80 mmol) in 87 ml THF wird bei 0°C langsam mit 30 %igem $H_2O_2$ (5,23 g, 46,3 mmol) versetzt und noch 3 h bei Raumtemperatur gerührt. Nach Zugabe von 100 ml Eiswasser wird mit $CH_2Cl_2$ (3 x 100 ml) extrahiert, die vereinten organischen Phasen werden mit ges. NaCl-Lösung (100 ml) gewaschen, getrocknet ($Na_2SO_4$) und im Vakuum eingeengt. Der Rückstand wird an Kieselgel chromatographiert (Petrolether/Diethylether = 2:1, $R_f$ = 0,33). Man erhält 1,32 g (62 %) eines 35:65-Gemisches der Titelverbindung und des entsprechenden 3-Cyclopenten-Isomers. Nach fraktionierter Kristallisation aus n-Hexan (die Titelverbindung befindet sich jeweils in der Mutterlauge) erhält man die Titelverbindung.

Ausbeute: 142 mg (7 %)
$^1$H-NMR ($CDCl_3$): δ = 1,48 (s, 9H), 2,37, 2,70 (AB-Teil eines ABX-Systems, 2H), 3,70 (s, 3H), 3,71 (m, 1H), 4,62 (dt, 1H), 5,20 (d, 1H), 5,71 (m, 1H), 5,96 (m, 1H)
$C_{12}H_{19}NO_4$ (241,3)

**Beispiel 65**

(+/-)-(1RS, 2SR)-1,2-cis-2-Amino-4-cyclopenten-1-carbonsäuremethylester-Hydrochlorid

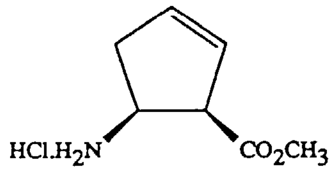

Die Titelverbindung wird in Analogie zur Vorschrift des Beispiels 5 ausgehend von Beispiel 64 hergestellt.

Ausbeute: 93 mg (89 %)
$^1$H-NMR ($d_6$-DMSO): $\delta$ = 2,40-2,85 (m, 2H), 3,86 (m, 1H), 4,01 (dt, 1H), 5,76 (m, 1H), 5,97 (m, 1H), 8,10 (s, 3H)
$C_7H_{11}NO_2$ x HCl (141,2 x 36,5)

**Beispiel 66**

(+/-)-(1RS, 2SR)-1,2-cis-2-Benzylamino-3-benzyloxymethyl-cyclopentan-1-carbonsäureethylester

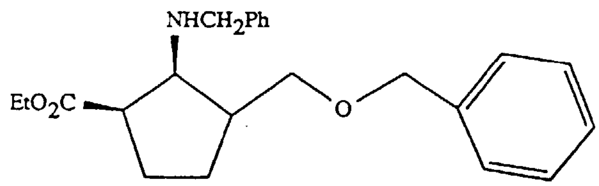

Die Titelverbindung wird in Analogie zur Vorschrift des Beispiels 10 ausgehend von Beispiel XXIII hergestellt.

Ausbeute: 8,38 g (76 %)
Smp.: 215°C (Zers.)
Diastereomerenverhältnis $D_1$:$D_2$ = 6:1
$R_f$ = 0,43 ($D_1$), 0,34 ($D_2$), (Petrolether/Diethylether = 1:1)
$C_{24}H_{29}NO_3$ (379,50)

**Beispiel 67**

(+/-)-(1RS, 2SR)-1,2-cis-2-Amino-3-hydroxymethyl-cyclopentan-1-carbonsäureethylester-Hydrochlorid

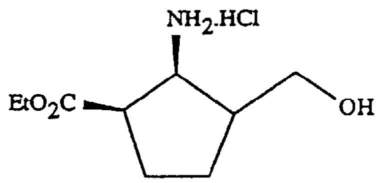

Die Titelverbindung wird in Analogie zur Vorschrift des Beispiels 13 ausgehend von Beispiel 66 hergestellt.

Ausbeute: 3,14 g (97 %)
Diastereomerenverhältnis $D_1$:$D_2$ = 6:1
$^1$H-NMR ($CDCl_3$): $\delta$ = 1,28, 1,29 (2t, 3H), 1,72-2,30 (m, 4H), 2,71 ($D_2$) und 2,88 ($D_2$) (2m, 1H), 3,06 ($D_2$) und 3,27 ($D_2$) (2m, 1H), 3,50-3,90 (m, 3H), 4,10-4,32 (m, 3H), 8,40 (s, 3H)

$C_9H_{17}NO_3$ x HCl (187,2 x 36,5)

**Beispiel 68**

(+/-)-(1RS, 2SR)-1,2-cis-2-N-(tert.-Butyloxycarbonyl)amino-3-hydroxymethyl-cyclopentan-1-carbonsäureethylester

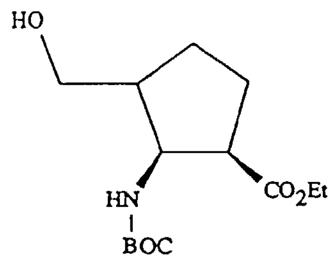

Die Titelverbindung wird in Analogie zur Vorschrift des Beispiels 3 ausgehend von Beispiel 67 hergestellt.

Ausbeute: 2,98 g (98 %)
Diastereomerenverhältnis $D_1$:$D_2$ = 5:1
$^1$H-NMR ($CDCl_3$): 1,28 (2t, 3H), 1,45 (2s, 9H), 1,65-2,38 (m, 5H), 3,02 ($D_2$), 3,06 ($D_1$) (2dt, 1H), 3,22-3,60 (m, 2H), 3,95-4,40 (m, 4H), 4,95 (Di), 5,61 ($D_2$) (2d, 1H)
$C_{14}H_{25}NO_5$ (287,4)

**Beispiel 69**

(+/-)-(1RS, 2SR)-1,2-cis-2-N-(tert.-Butyloxycarbonyl)amino-3-methylen-cyclopentan-1-carbonsäureethylester

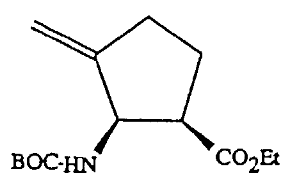

Zur Lösung von Beispiel 68 (2,25 g, 7,83 mmol) in 210 ml THF tropft man unter Argon bei Raumtemperatur 2-Nitrophenylselenocyanat (3,56 g, 15,7 mmol) und eine Lösung von Tri-n-butylphosphin (3,17 g, 15,7 mmol) in 20 ml THF. Nach 30 min Rühren tropft man 30 %iges $H_2O_2$ (1,33 g, 39,2 mmol) hinzu und läßt über Nacht bei Raumtemperatur rühren. Nach Zugabe von Wasser (500 ml) wird mit Essigester (3 x 250 ml) extrahiert, die vereinten organischen Phasen werden mit ges. $NaHCO_3$-Lösung (200 ml) gewaschen, getrocknet ($MgSO_4$) und das Lösungsmittel wird im Vakuum abgezogen. Der Rückstand wird an Kieselgel chromatographiert (Petrolether/Diethylether = 2:1)

Ausbeute: 1,67 g (79 %)
Smp.: 64°C
$C_{14}H_{23}NO_4$ (269,3)

**Beispiel 70**

(+/-)-(1RS, 2SR)1,2-cis-2-N-(tert.-Butyloxycarbonyl)amino-3-methylen-cyclopentan-1-carbonsäure

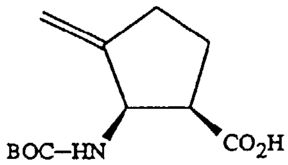

Die Titelverbindung wird in Analogie zur Vorschrift des Beispiels IX ausgehend von Beispiel 69 hergestellt.

Ausbeute: 1,71 g (97 %)
Smp.: 135°C
$C_{12}H_{19}NO_4$ (241,3)

**Beispiel 71**

(+/-)-(1RS, 2SR)-1,2-cis-2-N-(tert.-Butyloxycarbonyl)amino-3-methylen-cyclopentan-1-carbonsäuremethylester

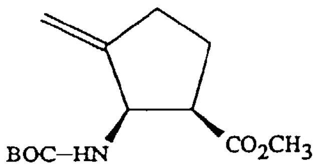

Die Titelverbindung wird in Analogie zur Vorschrift des Beispiels 57 ausgehen von Beispiel 70 hergestellt.

Ausbeute: 1,59 g (91 %)
Smp.: 44°C
$C_{13}H_{21}NO_4$ (255,3)

**Beispiel 72**

(+/-)-(1RS, 2SR)1,2-cis-2-Amino-3-methylen-cyclopentan-1-carbonsäure-Hydrochlorid

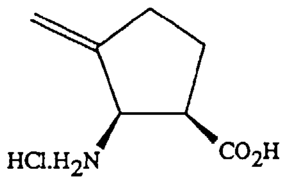

Zur Lösung des Beispiels 70 (500 mg, 2,07 mmol) und 2,6-Lutidin (890 mg, 8,30 mmol) in 5 ml $CH_2Cl_2$ tropft man Trifluormethansulfonsäure-(tert.-butyldimethyl)silylester (1,64 g, 6,21 mmol) bei Raumtemperatur unter Argon. Man läßt 3 h rühren, gibt 10 ml ges. $NH_4Cl$-Lösung hinzu, extrahiert mit Ether (2 x 20 ml), wäscht die vereinten organischen Phasen mit ges. NaCl-Lösung (10 ml), trocknet ($Na_2SO_4$) und zieht das Solvens im Vakuum ab. Der Rückstand wird in 20,7 ml 0,1N Salzsäure und 20 ml THF aufgenommen, man läßt 20 h rühren, zieht das THF im Vakuum ab, wäscht den Rückstand mit Ether (10 ml) und engt die wäßrige Phase im Vakuum ein. Der Rückstand wird in 7 ml Propenoxid gelöst und 30 min unter Rückfluß erhitzt. Man saugt den ausgefallenen Feststoff ab, wäscht mit Ether und nimmt den

Rückstand (136 mg) in 9,6 ml 0,IN Salzsäure auf. Das Lösungsmittel wird im Vakuum abgezogen und der Rückstand im Vakuum über $P_4O_{10}$ getrocknet.

Ausbeute: 190 mg (52 %)
Smp.: 208°C (Zers.)
$C_7H_{11}NO_2$ x HCl (141,2 x 36,5)

**Beispiel 73**

(+/-)-(1RS, 2SR)-1,2-cis-2-Amino-3-methylen-cyclopentan-1-carbonsäuremethylester-Hydrochlorid

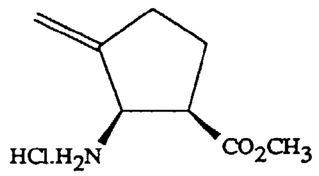

Die Titelverbindung wird in Analogie zur Vorschrift des Beispiels 37 ausgehend von Beispiel 71 hergestellt.

Ausbeute: 145 mg (39 %)
Smp.: 143°C
$C_8H_{13}NO_2$ (155,2 x 36,5)

**Beispiel 74**

(+/-)-(1RS, 2SR)1,2-trans-2-N-(tert.-Butyloxycarbonyl)amino-3-methylen-cyclopentan-1-carbonsäuremethylester

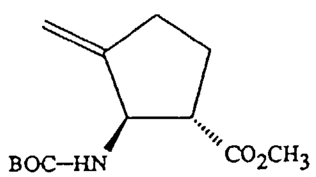

Die Titelverbindung wird in Analogie zur Vorschrift des Beispiels 61 ausgehend von Beispiel 71 hergestellt.

Ausbeute: 410 mg (82 %)
Smp.: 74°C
$R_f$ = 0,43 (Petrolether/Essigester = 3:1)
$C_{13}H_{21}NO_4$ (255,3)

**Beispiel 75**

(+/-)-(1RS, 2SR)-1,2-trans-2-Amino-3-methylen-cyclopentan-1-carbonsäuremethylester-Hydrochlorid

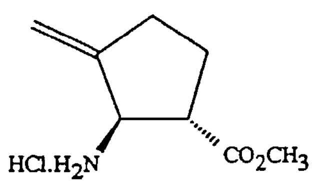

Die Titelverbindung wird in Analogie zur Vorschrift des Beispiels 37 ausgehend von Beispiel 74 hergestellt.

Ausbeute: 228 mg (82 %)
Smp.: 166°C
$C_8H_{13}NO_2$ x HCl (155,2 x 36,5)

**Beispiel 76**

2-N-(tert.-Butyloxycarhonyl)amino-cyclopentan-3-on-carbonsäuremethylester

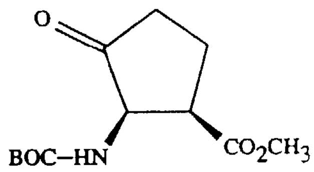

Die Titelverbindung wird in Analogie zur Vorschrift des Beispiels 4 ausgehend von Beispiel 71 hergestellt.

Ausbeute: 898 mg (89 %),
2 Diastereomere cis:trans = 2:1
Smp.: 98°C
$C_{12}H_{19}NO_5$ (257,3)

**Beispiel 77**

2-Amino-cyclopentan-3-on-1-carbonsäuremethylester

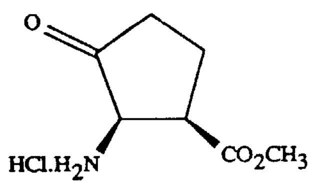

Die Titelverbindung wird in Analogie zur Vorschrift des Beispiels 5 ausgehend von Beispiel 76 hergestellt.

Ausbeute: 139 mg (92 %)
2 Diastereomere cis:trans = 2:1
Smp.: 250°C
$C_7H_{11}NO_3$ x HCl (157,2 x 36,5)

**Beispiel 78**

2-N-(tert.-Butyloxycarbonyl)amino-3,3-difluor-cyclopentan-1-carbonsäuremethylester

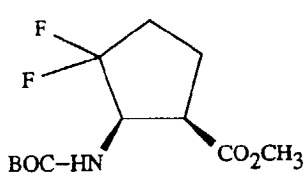

Die Titelverbindung wird in Analogie zur Vorschrift des Beispiels XVIII ausgehend von Beispiel 76 hergestellt.

Ausbeute: 176 mg (32 %), cis:trans = 2:1
$^1$H-NMR ($CDCl_3$): $\delta$ = 1,46 (s, 9H), 2,10-2,42 (m, 2H), 2,49-2,70 (m, 2H), 2,72 (dt, 2H), 3,72 (s, 3H), 4,30-4,52 (m, 1H), 4,85 (cis, s, 1H), 5,20 (trans, s, 1H)
$C_{12}H_{19}F_2NO_4$ (279,3)

**Beispiel 79**

2-Amino-3,3-difluor-cyclopentan-1-carbonsäuremethylester

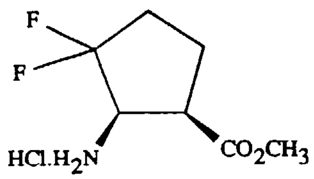

Die Titelverbindung wird in Analogie zur Vorschrift des Beispiels 5 ausgehend von Beispiel 78 hergestellt.

Ausbeute: 61 mg (48 %), cis/trans = 2:1
Smp.: 118°C
$C_7H_{11}F_2NO_2$ x HCl (176,2 x 36,5)

**Patentansprüche**

**1.** Cyclopentan- und -penten-β-Aminosäuren der allgemeinen Formel (I)

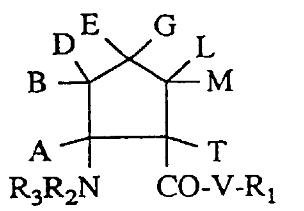

(I),

in welcher

A, B, D, E, G,L, M und T gleich oder verschieden sind und mit der Maßgabe, daß mindestens einer der vorgenannten Substituenten ≠ H ist, für Wasserstoff, Halogen, Benzyl, Hydroxy oder für geradkettiges oder verzweigtes Alkyl mit bis zu 8 Kohlenstoffatomen stehen, das gegebenenfalls 1- bis 2-fach gleich oder verschieden durch Halogen, Hydroxy, Phenyl, Benzyloxy, Carboxy oder durch geradkettiges oder verzweigtes Alkoxy, Acyl oder Alkoxycarbonyl mit jeweils bis zu 6 Kohlenstoffatomen oder durch eine Gruppe der Formel $-NR^4R^5$ substituiert ist,
worin

$R^4$ und $R^5$ gleich oder verschieden sind und Wasserstoff, Phenyl oder geradkettiges oder verzweigtes Alkyl mit bis zu 6 Kohlenstoffatomen bedeuten,

oder jeweils B und D, E und G oder L und M gemeinsam für einen Rest der Formel

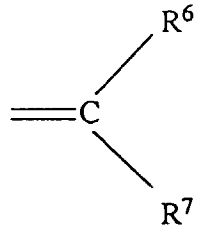

oder =N-OH stehen,
worin

$R^6$ und $R^7$ gleich oder verschieden sind und Wasserstoff, Halogen oder geradkettiges oder verzweigtes Alkyl, Alkoxy oder Oxyacyl mit jeweils bis zu 8 Kohlenstoffatomen, Benzyl oder Phenyl bedeuten,

oder

E und G und/oder B und D zusammen für den Rest der Formel =O oder =S stehen,
oder

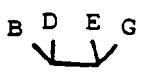
oder
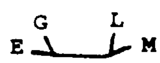

jeweils einen Rest der Formel

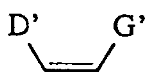
oder
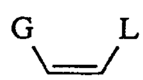

bilden,
worin

D' und G' die oben angegebene Bedeutung von D und G haben, aber nicht gleichzeitig Wasserstoff bedeuten
und

G und L die oben angegebene Bedeutung haben

$R^2$ für Wasserstoff oder

für eine in der Peptidchemie übliche Aminoschutzgruppe steht, oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl mit bis zu 8 Kohlenstoffatomen, oder
für Benzoyl steht, das gegebenenfalls wie oben beschrieben substituiert ist, oder
für eine Gruppe der Formel $-SO_2R^8$ steht,

worin

$R^8$ geradkettiges oder verzweigtes Alkyl mit bis zu 8 Kohlenstoffatomen, Benzyl oder Phenyl bedeutet, wobei die beiden letztgenannten Reste gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe $-NR^4R^5$ substitituiert sind,
worin
$R^4$ und $R^5$ die oben angegebene Bedeutung haben,

oder für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel $-NR^4R^5$ oder $-SO_2R^8$ substituiert ist,
worin
$R^4$, $R^5$ und $R^8$ die oben angegebene Bedeutung haben,
oder
für einen Aminosäurerest der Formel

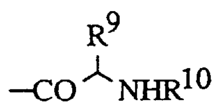

steht,
worin

$R^9$ Cycloalkyl mit 3 bis 8 Kohlenstoffatomen oder Aryl mit 6 bis 10 Kohlenstoffatomen oder Wasserstoff bedeutet, oder geradkettiges oder verzweigtes Alkyl mit bis zu 8 Kohlenstoffatomen bedeutet, wobei das Alkyl gegebenenfalls durch Cyano, Methylthio, Hydroxy, Mercapto, Guanidyl oder durch eine Gruppe der Formel $-NR^{11}R^{12}$ oder $R^{13}$-OC- substituiert ist,

worin

$R^{11}$ und $R^{12}$ unabhängig voneinander für Wasserstoff, geradkettiges oder verzweigtes Alkyl mit bis zu 8 Kohlenstoffatomen oder Phenyl stehen,
und

$R^{13}$ Hydroxy, Benzyloxy, Alkoxy mit bis zu 6 Kohlenstoffatomen oder die oben aufgeführte Gruppe $-NR^{10}R^{11}$ bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{11}R^{12}$ substituiert ist,
worin

$R^{11}$ und $R^{12}$ die oben angegebene Bedeutung haben,
und

$R^{10}$ Wasserstoff oder eine in der Peptidchemie übliche Aminoschutzgruppe bedeutet

$R^3$ für Wasserstoff oder für geradkettiges oder verzweigtes Alkyl mit bis zu 8 Kohlenstoffatomen steht, das gegebenenfalls durch Phenyl substituiert ist,
oder

$R^2$ und $R^3$ gemeinsam für den Rest der Formel $=CHR^{14}$ stehen,
worin

$R^{14}$ Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,

V für ein Sauerstoff- oder Schwefelatom oder für die -NH-Gruppe steht,

$R^1$ für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel $-NR^4R^5$ oder $-SO_2R^8$ substituiert sind,
worin
$R^4$, $R^5$ und $R^8$ die oben angegebene Bedeutung haben,
oder für den Fall, daß V für die -NH-Gruppe steht,

$R^1$ für die Gruppe der Formel $-SO_2R^8$ steht,
worin
$R^8$ die oben angegebene Bedeutung hat, mit der Maßgabe, daß

falls V für ein Sauerstoffatom steht,
T ungleich Methyl, Ethyl, Benzyl oder Hydroxyl ist,
$R^1$ ungleich Methyl oder Ethyl ist, und
M und L ungleich $-CH_2-COOH$ sind.

**2.** Cyclopentan- und -penten-β-Aminosäuren der allgemeinen Formel (I) gemäß Anspruch 1, in welcher

A, B, D, E, G, L, M und T gleich oder verschieden sind und mit der Maßgabe, daß mindestens einer der vorgenannten Substituenten ≠ H ist, für Wasserstoff, Halogen, Benzyl, Hydroxy oder für geradkettiges oder verzweigtes Alkyl mit bis zu 6 Kohlenstoffatomen stehen, das gegebenenfalls ein- bis zweifach gleich oder verschieden durch Halogen, Hydroxy, Benzyloxy, durch geradkettiges oder verzweigtes Alkoxy, Acyl oder Alkoxycarbonyl mit jeweils bis zu 4 Kohlenstoffatomen oder durch eine Gruppe der Formel $-NR^4R^5$ substituiert ist,
worin

$R^4$ und $R^5$ gleich oder verschieden sind und Wasserstoff oder geradkettiges oder verzweigtes Alkyl mit bis zu 4 Kohlenstoffatomen bedeuten,

oder jeweils B und D, E und G oder L und M gemeinsam für einen Rest der Formel

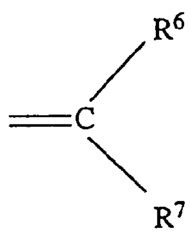

oder =N-OH stehen,
worin

$R^6$ und $R^7$ gleich oder verschieden sind und Wasserstoff, Fluor, Chlor, Brom oder geradkettiges oder verzweigtes Alkyl mit bis zu 6 Kohlenstoffatomen, Benzyl oder Phenyl bedeuten,

oder

E und G und/oder B und D zusammen für den Rest der Formel =O oder =S stehen,

oder

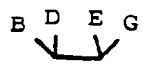 oder 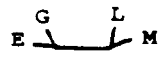

jeweils einen Rest der Formel

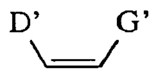 oder 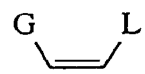

bilden,
worin

D' und G' die oben angegebene Bedeutung von D und G haben, aber nicht gleichzeitig für Wasserstoff stehen
und

G und L die oben angegebene Bedeutung haben,

$R^2$ für Wasserstoff oder

für Boc, Benzyl, Benzyloxycarbonyl, Allyloxycarbonyl oder 9-Fluorenylmethyloxycarbonyl (Fmoc) steht, oder
für geradkettiges oder verzweigtes Alkyl mit bis zu 6 Kohlenstoffatomen steht, das gegebenenfalls durch Hydroxy, Formyl oder durch geradkettiges oder verzweigtes Acyl mit bis zu 4 Kohlenstoffatomen oder durch Phenyl oder Benzoyl substiutiert ist, die gegebenenfalls durch Halogen, Nitro, Cyano, oder durch geradkettiges oder verzweigtes Alkyl mit bis zu 4 Kohlenstoffatomen substituiert sind,
oder
für geradkettiges oder verzweigtes Acyl mit bis zu 6 Kohlenstoffatomen oder für Benzoyl steht, das gegebenenfalls wie oben beschrieben substituiert ist, oder
für eine Gruppe der Formel $-SO_2R^8$ steht,
worin

$R^8$ geradkettiges oder verzweigtes Alkyl mit bis zu 6 Kohlenstoffatomen, Phenyl oder Benzyl bedeutet, wobei die beiden letztgenannten Reste gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel $-NR^4R^5$ substituiert ist,

worin
$R^4$ und $R^5$ die oben angegebene Bedeutung haben,
oder für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel $-NR^6R^7$ oder $-SO_2R^8$ substituiert ist,
worin
$R^6$, $R^7$ und $R^8$ die oben angegebene Bedeutung haben,
oder
für einen Aminosäurerest der Formel

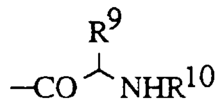

steht,
worin

$R^9$ Wasserstoff, geradkettiges oder verzweigtes Alkyl mit bis zu 6 Kohlenstoffatomen oder Benzyl bedeutet
und

$R^{10}$ Wasserstoff, Benzyloxycarbonyl, tert. Butoxycarbonyl oder Fmoc bedeutet,

$R^3$ für Wasserstoff oder für geradkettiges oder verzweigtes Alkyl mit bis zu 6 Kohlenstoffatomen oder Benzyl steht,
oder

$R^2$ und $R^3$ gemeinsam für den Rest der Formel $=CHR^{14}$ stehen,
worin

$R^{14}$ Wasserstoff oder geradkettiges oder verzweigtes Alkyl mit bis zu 6 Kohlenstoffatomen bedeutet, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist,

V für ein Sauerstoff- oder Schwefelatom oder für die -NH-Gruppe steht,

$R^1$ für Wasserstoff oder für geradkettiges oder verzweigtes Alkyl mit bis zu 6 Kohlenstoffatomen oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe der Formel $-NR^4R^5$ oder $-SO_2R^8$ substituiert sind,
worin
$R^4$, $R^5$ und $R^8$ die oben angegebene Bedeutung haben,
oder für den Fall, daß V für die -NH-Gruppe steht,

$R^1$ für die Gruppe der Formel $-SO_2R^8$ steht,
worin

$R^8$ die oben angegebene Bedeutung hat.

**3.** Cyclopentan- und -penten-β-Aminosäuren der allgemeinen Formel (I) gemäß Anspruch 1, in welcher

A, B, D, E, G, L, M und T gleich oder verschieden sind und mit der Maßgabe, daß mindestens einer der vorgenannten Substituenten $\neq$ H ist, für Wasserstoff, Fluor, Chlor, Brom, Benzyl, Hydroxy oder für geradkettiges oder verzweigtes Alkyl mit bis zu 4 Kohlenstoffatomen stehen, das gegebenenfalls ein- oder zweifach gleich oder verschieden durch Hydroxy oder Benzyloxy substituiert ist,

oder jeweils B und D, E und G oder L und M gemeinsam für einen Rest der Formel

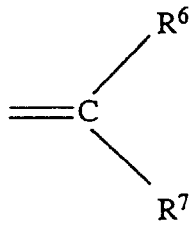

oder =N-OH stehen,
worin

$R^6$ und $R^7$ gleich oder verschieden sind und Wasserstoff, Fluor, Chlor, Brom oder geradkettiges oder verzweigtes Alkyl mit bis zu 4 Kohlenstoffatomen oder Phenyl bedeuten, oder

E und G und/oder B und D zusammen für den Rest der Formel =O oder =S stehen,
oder

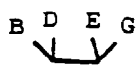 oder 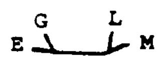

jeweils gemeinsam einen Rest der Formel

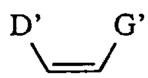 oder 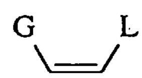

bilden,
worin

D' und G' die oben angegebene Bedeutung von D und G haben, aber nicht gleichzeitig für Wasserstoff stehen

G und L gleich oder verschieden sind und Wasserstoff oder Methyl bedeuten,

$R^2$ für Wasserstoff, Allyloxycarbonyl, Benzyl, Boc oder Fmoc steht,
oder

für geradkettiges oder verzweigtes Alkyl mit bis zu 4 Kohlenstoffatomen steht, oder
für geradkettiges oder verzweigtes Acyl mit bis zu 4 Kohlenstoffatomen oder
für eine Gruppe der Formel $-SO_2R^8$ steht,
worin

$R^8$ geradkettiges oder verzweigtes Alkyl mit bis zu 4 Kohlenstoffatomen, Phenyl oder Benzyl bedeutet, wobei die beiden letztgenannten Reste gegebenenfalls durch Hydroxy, Fluor, Chlor, Brom, Nitro, Cyano, Methyl, Ethyl oder Methoxy substituiert sind, oder

für einen Aminosäurerest der Formel

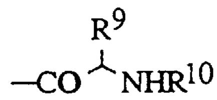

steht,
worin

$R^9$ Wasserstoff, geradkettiges oder verzweigtes Alkyl mit bis zu 4 Kohlenstoffatomen oder Benzyl bedeutet
und

$R^{10}$ Wasserstoff, tert. Butoxycarbonyl oder Fmoc bedeutet,

$R^3$ für Wasserstoff oder für geradkettiges oder verzweigtes Alkyl mit bis zu 4 Kohlenstoffatomen steht,
oder

$R^2$ und $R^3$ gemeinsam für den Rest der Formel $=CHR^{14}$ stehen,
worin

$R^{14}$ Wasserstoff oder geradkettiges oder verzweigtes Alkyl mit bis zu 4 Kohlenstoffatomen bedeutet,

V für ein Sauerstoff- oder ein Schwefelatom oder für die -NH-Gruppe steht,

$R^1$ für Wasserstoff oder für geradkettiges oder verzweigtes Alkyl mit bis zu 4 Kohlenstoffatomen oder Phenyl steht, wobei letztere gegebenenfalls durch Fluor, Chlor, Brom, Nitro, Cyano, Methoxy, Ethoxy oder durch eine Gruppe der Formel $-NR^4R^5$ oder $-SO_2R^8$ substituiert sind,
worin
$R^4$ und $R^5$ gleich oder verschieden sind und Wasserstoff, Methyl oder Ethyl bedeuten
und
$R^8$ die oben angegebene Bedeutung hat, oder im Fall, daß V für die -NH-Gruppe steht,

$R^1$ für die Gruppe der Formel $-SO_2R^8$ steht,
worin

$R^8$ die oben angegebene Bedeutung hat.

**4.** Cyclopentan- und -penten-β-Aminosäuren der allgemeinen Formel (I) gemäß den Ansprüchen 1-3, dadurch gekennzeichnet, daß sie in einer isomeren Form, als Säureadditions-Salze oder Metallsalzkomplexe vorliegen.

**5.** Cyclopentan-β-aminosäure der allgemeinen Formel (I) gemäß Anspruch 1, in welcher

A, B, D, L, M und T für Wasserstoff stehen,
E und G gemeinsam für einen Rest der Formel

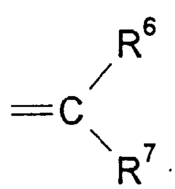

stehen,

worin

$R^6$ und $R^7$ Wasserstoff bedeuten,
$R^2$ für Wasserstoff steht,
$R^3$ für Wasserstoff steht und
$R^1$ für Wasserstoff steht und
in Form des (-)-1R*,2S*-cis Enantiomeren vorliegt.

**6.** Cyclopentan-β-aminosäure der allgemeinen Formel (I) gemäß Anspruch 1, in welcher

A, B, D, L, M und T für Wasserstoff stehen,
E und G gemeinsam für einen Rest der Formel

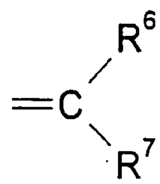

stehen,
worin

$R^6$ und $R^7$ Wasserstoff bedeuten,
$R^2$ für Wasserstoff steht,
$R^3$ für Wasserstoff steht und
$R^1$ für Wasserstoff steht und
in Form des Hydrochlorid-Salzes des (-)-1R*,2S*-cis Enantiomeren vorliegt.

**7.** Verfahren zur Herstellung der Verbindungen der allgemeinen Formel (I) gemäß den Ansprüchen 1-6, dadurch gekennzeichnet, daß man

[A] Verbindungen der allgemeinen Formel (II)

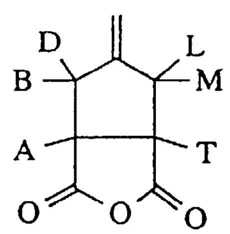

(II),

in welcher
A, B, D, L, M und T die oben angegebene Bedeutung haben,
in organischen Lösemitteln, vorzugsweise Dioxan, zunächst mit $(C_1\text{-}C_3)$-Trialkylsilylaziden, anschließend mit Ethern, in Anwesenheit von Wasser, in die Verbindungen der allgemeinen Formel (III)

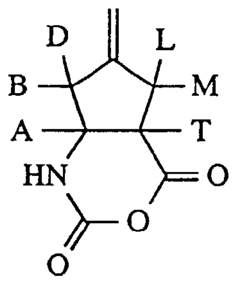

(III),

in welcher
A, B, D, L, M und T die oben angegebene Bedeutung haben,
überführt,
und in einem nächsten Schritt mit Säuren, vorzugsweise Salzsäure, unter Ringöffnung zu den Verbindungen der allgemeinen Formel (Ia)

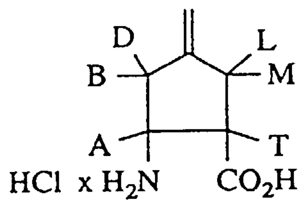

(Ia),

in welcher
A, B, D, L, M und T die oben angegebene Bedeutung haben,
umsetzt,
und gegebenenfalls eine Eliminierung mit Säuren, vorzugsweise Salzsäure, anschließt, oder
[B] Verbindungen der allgemeinen Formel (IV)

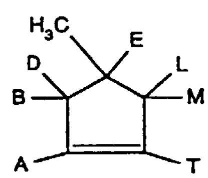

(IV),

in welcher
A, B, D, E, L, M und T die oben angegebene Bedeutung haben,
durch Umsetzung mit Chlorsulfonylisocyanat zunächst in die Verbindungen der allgemeinen Formel (V)

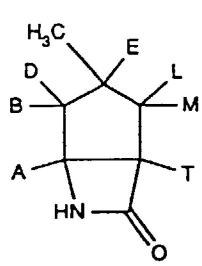

(V),

in welcher

A, B, D, E, L, M und T die oben angegebene Bedeutung haben,
überführt und anschließend mit Säuren, vorzugsweise Salzsäure, unter Ringöffnung die Amin- und Carboxyfunktion freisetzt, oder
[C] Verbindungen der allgemeinen Formel (VI)

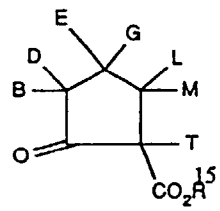

(VI),

in welcher
B, D, E, G, L, M und T die oben angegebene Bedeutung haben,
und

$R^{15}$ für $C_1$-$C_4$-Alkyl steht,

durch Umsetzung mit Aminen der allgemeinen Formel (VII)

$$H_2N\text{-}R^{16} \qquad \text{(VII)},$$

in welcher

$R^{16}$ für Benzyl steht, das gegebenenfalls durch Halogen, $NO_2$, Cyano oder $C_1$-$C_4$-Alkyl oder für den Rest der Formel $-CH(C_6H_4\text{-}pOCH_3)_2$ steht,

in organischen Lösemitteln, gegebenenfalls in Anwesenheit einer Base, in die Verbindungen der allgemeinen Formel (VIII)

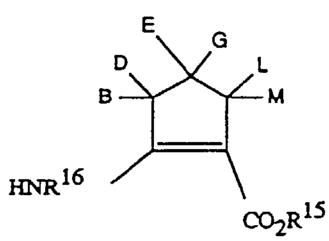

(VIII),

in welcher
B, D, E, G, L, M, $R^{15}$ und $R^{16}$ die oben angegebene Bedeutung haben,
überführt,
und anschließend durch zweifache Hydrierung zunächst die Doppelbindung reduziert, anschließend die Aminfunktion freisetzt und in einem letzten Schritt den Carbonsäureester mit Säuren verseift,
und grundsätzlich die Substituenten A - T, gegebenenfalls unter vorgeschalteter Blockierung der Aminfunktion durch Umsetzung der Schutzgruppen nach üblichen Methoden, beispielsweise durch Oxidation, Reduktion oder Alkylierung derivatisiert,
und im Fall der Säuren, die Ester nach üblichen Methoden verseift
und im Fall der für V und $R^1$ oben aufgeführten anderen Definitionen, ebenfalls nach üblichen Verfahren, wie beispielsweise Amidierung, Sulfonierung oder Sulfoamidierung, gegebenenfalls in Anwesenheit von Hilfsstoffen wie Katalysatoren und Dehydratisierungsmitteln, ausgehend von den entsprechenden Carbonsäuren, gegebenenfalls unter vorgeschalteter Aktivierung, derivatisiert, und im Fall der reinen Enantiomere eine Racematspaltung durchführt.

**8.** Verbindungen der allgemeinen Formel (I) nach den Ansprüchen 1-6 zur Verwendung bei der Bekämpfung von Krankheiten.

**9.** Verwendung von Verbindungen der Formel (I) nach den Ansprüchen 1-6 zur Herstellung von Arzneimitteln.

**10.** Arzneimittel enthaltend Cyclopentan- und -penten-β-Aminosäuren der allgemeinen Formel (I) gemäß den Ansprüchen 1-6.

**Claims**

**1.** Cyclopentane- and -pentene-β-amino acids of the general formula (I)

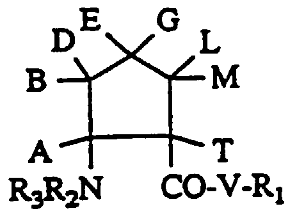

(I)

in which

A, B, D, E, G, L, M and T are identical or different and with the proviso that at least one of the abovementioned substituents ≠ H, represent hydrogen, halogen, benzyl, hydroxyl or straight-chain or branched alkyl having up to 8 carbon atoms, which is optionally monosubstituted or disubstituted by identical or different halogen, hydroxyl, phenyl, benzyloxy and carboxyl substituents or by straight-chain or branched alkoxy, acyl or alkoxycarbonyl each having up to 6 carbon atoms or by a group of the formula $-NR^4R^5$,
in which

$R^4$ and $R^5$ are identical or different and denote hydrogen, phenyl or straight-chain or branched alkyl having up to 6 carbon atoms,

or B and D, E and G or L and M in each case together represent a radical of the formula

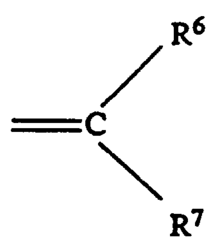

or = N-OH
in which

$R^6$ and $R^7$ are identical or different and denote hydrogen, halogen or straight-chain or branched alkyl, alkoxy or oxyacyl each having up to 8 carbon atoms, benzyl or phenyl,

or

E and G and/or B and D together represent the radical of the formula =O or =S, or

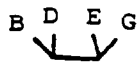 or 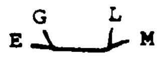

in each case form a radical of the formula

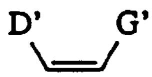 or 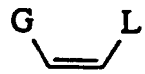 ,

in which

D' and G' have the abovementioned meaning of D and G, but do not simultaneously denote hydrogen and

G and L have the abovementioned meaning

$R^2$ represents hydrogen or

represents an amino protective group customary in peptide chemistry, or
represents straight-chain or branched alkyl having up to 8 carbon atoms, which is optionally monosubstituted or disubstituted by identical or different hydroxyl or formyl substituents or by straight-chain or branched acyl having up to 6 carbon atoms or by phenyl or benzoyl, each of which is optionally substituted up to 2 times by identical or different halogen, nitro or cyano substituents, or by straight-chain or branched alkyl having up to 6 carbon atoms,
or
represents straight-chain or branched acyl having up to 8 carbon atoms, or
represents benzoyl which is optionally substituted as described above, or represents a group of the formula $-SO_2R^8$,

in which

$R^8$ denotes straight-chain or branched alkyl having up to 8 carbon atoms, benzyl or phenyl, where the two last-mentioned radicals are optionally substituted up to 3 times by identical or different halogen, hydroxyl, nitro, cyano, trifluoromethyl or trifluoromethoxy substituents or by straight-chain or branched alkyl, alkoxy or alkoxycarbonyl each having up to 6 carbon atoms, carboxyl or by the abovementioned group $-NR^4R^5$,
in which
$R^4$ and $R^5$ have the abovementioned meaning,

or represents phenyl which is optionally substituted up to 3 times by identical or different halogen, hydroxyl, nitro, trifluoromethyl, trifluoromethoxy, straight-chain or branched alkyl, acyl, alkoxy or alkoxycarbonyl substituents each having up to 6 carbon atoms or by a group of the formula $-NR^4R^5$ or $-SO_2R^8$,
in which
$R^4$, $R^5$ and $R^8$ have the abovementioned meaning,
or
represents an amino acid radical of the formula

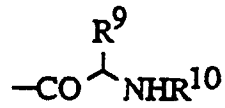

in which

$R^9$ denotes cycloalkyl having 3 to 8 carbon atoms or aryl having 6 to 10 carbon atoms or hydrogen, or
denotes straight-chain or branched alkyl having up to 8 carbon atoms,

where the alkyl is optionally substituted by cyano, methylthio, hydroxyl, mercapto or guanidyl or by a group of the formula $-NR^{11}R^{12}$ or $R^{13}$-OC-,
in which

$R^{11}$ and $R^{12}$ independently of one another represent hydrogen, straight-chain or branched alkyl having up to 8 carbon atoms or phenyl,
and

$R^{13}$ denotes hydroxyl, benzyloxy, alkoxy having up to 6 carbon atoms or the abovementioned group $-NR^{10}R^{11}$,
or the alkyl is optionally substituted by cycloalkyl having 3 to 8 carbon atoms or by aryl having 6 to 10 carbon atoms, which is in turn substituted by hydroxyl, halogen, nitro or alkoxy having up to 8 carbon atoms or by the group $-NR^{11}R^{12}$,
in which

$R^{11}$ and $R^{12}$ have the abovementioned meaning,

and

$R^{10}$ denotes hydrogen or an amino protective group customary in peptide chemistry

$R^3$ represents hydrogen or straight-chain or branched alkyl having up to 8 carbon atoms, which is optionally substituted by phenyl,
or

$R^2$ and $R^3$ together represent the radical of the formula $=CHR^{14}$,
in which

$R^{14}$ denotes hydrogen or straight-chain or branched alkyl having up to 8 carbon atoms, which is optionally substituted by halogen, hydroxyl, phenyl or carboxyl or by straight-chain or branched alkoxy or alkoxycarbonyl each having up to 6 carbon atoms,

V represents an oxygen or sulphur atom or the -NH group,

$R^1$ represents hydrogen or straight-chain or branched alkyl having up to 8 carbon atoms or phenyl, where the latter are optionally substituted up to 3 times by identical or different hydroxyl, halogen, nitro, cyano, carboxyl, trifluoromethyl or trifluoromethoxy substitutents, by straight-chain or branched alkoxy, in the case of phenyl also by alkyl, acyl or alkoxycarbonyl each having up to 6 carbon atoms or by a group of the formula $-NR^4R^5$ or $-SO_2R^8$,
in which
$R^4$, $R^5$ and $R^8$ have the abovementioned meaning,
or in the case in which V represents the -NH group,

$R^1$ represents the group of the formula $-SO_2R^8$, in which

$R^8$ has the abovementioned meaning,

with the proviso that

if V represents an oxygen atom,
T is not methyl, ethyl, benzyl or hydroxyl,
$R^1$ is not methyl or ethyl, and
M and L are not $-CH_2-COOH$.

**2.** Cyclopentane- and -pentene-β-amino acids of the general formula (I) according to Claim 1, in which

A, B, D, E, G, L, M and Tare identical or different and with the proviso that at least one of the abovementioned substituents ≠ H, represent hydrogen, halogen, benzyl, hydroxyl or straight-chain or branched alkyl having up to 6 carbon atoms, which is optionally monosubstituted or disubstituted by identical or different halogen, hydroxyl or benzyloxy, by straight-chain or branched alkoxy, acyl or alkoxycarbonyl each having up to 4 carbon atoms or by a group of the formula $-NR^4R^5$, in which

$R^4$ and $R^5$ are identical or different and denote hydrogen or straight-chain or branched alkyl having up to 4 carbon atoms,

or B and D, E and G or L and M in each case together represent a radical of the formula

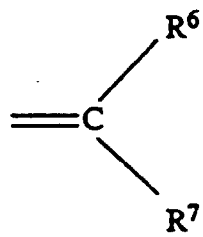

or -N-OH, in which

$R^6$ and $R^7$ are identical or different and denote hydrogen, fluorine, chlorine or bromine, or straight-chain or branched alkyl having up to 6 carbon atoms, benzyl or phenyl,

or

E and G and/or B and D together represent the radical of the formula =O or =S,
or

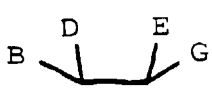 or 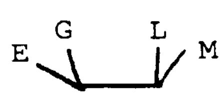

in each case form a radical of the formula

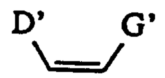 or 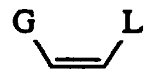 ,

in which

D' and G' have the abovementioned meaning of D and G, but do not simultaneously represent hydrogen and

G and L have the abovementioned meaning,

$R^2$ represents hydrogen or

represents Boc, benzyl, benzyloxycarbonyl, allyloxycarbonyl or 9-fluorenylmethoxycarbonyl (Fmoc), or
represents straight-chain or branched alkyl having up to 6 carbon atoms, which is optionally substituted by hydroxyl or formyl or by straight-chain or branched acyl having up to 4 carbon atoms or by phenyl or benzoyl, each of which is optionally substituted by halogen, nitro or cyano, or by straight-chain or branched alkyl having up to 4 carbon atoms,
or
represents straight-chain or branched acyl having up to 6 carbon atoms or benzoyl which is optionally substituted as described above,
or
represents a group of the formula $-SO_2R^8$, in which

$R^8$ denotes straight-chain or branched alkyl having up to 6 carbon atoms, phenyl or benzyl, where the two last-mentioned radicals are optionally substituted up to 2 times by identical or different halogen, hydroxyl, nitro, cyano, trifluoromethyl or trifluoromethoxy substituents or by straight-chain or branched alkyl or alkoxy each having up to 4 carbon atoms or by the abovementioned group of the formula $-NR^4R^5$,
in which
$R^4$ and $R^5$ have the abovementioned meaning
or represents phenyl which is optionally substituted up to 2 times by identical or different halogen, hydroxyl, nitro, trifluoromethyl, trifluoromethoxy, straight-chain or branched alkyl, acyl, alkoxy or alkoxycarbonyl substituents each having up to 4 carbon atoms or by a group of the formula $-NR^6R^7$ or $-SO_2R^8$,
in which
$R^6$, $R^7$ and $R^8$ have the abovementioned meaning,
or
represents an amino acid radical of the formula

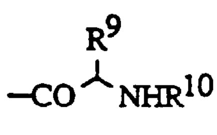

in which

$R^9$ denotes hydrogen, straight-chain or branched alkyl having up to 6 carbon atoms or benzyl

and

$R^{10}$ denotes hydrogen, benzyloxycarbonyl, tert-butoxycarbonyl or Fmoc,

$R^3$ represents hydrogen or straight-chain or branched alkyl having up to 6 carbon atoms or benzyl,

or

$R^2$ and $R^3$ together represent the radical of the formula $=CHR^{14}$,

in which

$R^{14}$ denotes hydrogen or straight-chain or branched alkyl having up to 6 carbon atoms, which is optionally substituted by halogen or hydroxyl, or by straight-chain or branched alkoxy or alkoxycarbonyl each having up to 4 carbon atoms,

V represents an oxygen or sulphur atom or the -NH group,

$R^1$ represents hydrogen or straight-chain or branched alkyl having up to 6 carbon atoms or phenyl, where the latter are optionally substituted up to 2 times by identical or different hydroxyl, halogen, nitro, cyano, trifluoromethyl or trifluoromethoxy substituents, by straight-chain or branched alkoxy, acyl or alkoxycarbonyl each having up to 4 carbon atoms or by a group of the formula $-NR^4R^5$ or $-SO_2R^8$,

in which

$R^4$, $R^5$ and $R^8$ have the abovementioned meaning,

or in the case in which V represents the -NH group,

$R^1$ represents the group of the formula $-SO_2R^8$,

in which

$R^8$ has the abovementioned meaning.

**3.** Cyclopentane- and -pentene-β-amino acids of the general formula (I) according to Claim 1, in which

A, B, D, E, G, L, M and T are identical or different and with the proviso that at least one of the abovementioned sustituents ≠ H, represent hydrogen, fluorine, chlorine, bromine, benzyl, hydroxyl or represent straight-chain or branched alkyl having up to 4 carbon atoms, which is optionally monosubstituted or disubstituted by identical or different hydroxyl or benzyloxy,

or B and D, E and G or L and M in each case together represent a radical of the formula

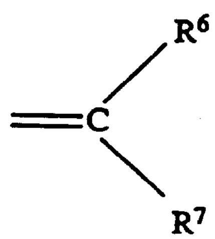

or =N-OH, in which

$R^6$ and $R^7$ are identical or different and denote hydrogen, fluorine, chlorine, bromine or straight-chain or branched alkyl having up to 4 carbon atoms or phenyl, or

E and G and/or B and D together represent the radical of the formula =O or =S,

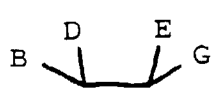

or

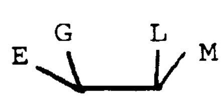

in each case form a radical of the formula

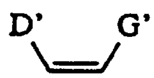 or 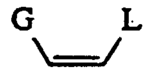 ,

in which

D' and G'have the abovementioned meaning of D and G, but do not simultaneously represent hydrogen,

G and L are identical or different and denote hydrogen or methyl,

$R^2$ represents hydrogen, allyloxycarbonyl, benzyl, Boc or Fmoc, or

represents straight-chain or branched alkyl having up to 4 carbon atoms, or represents straight-chain or branched acyl having up to 4 carbon atoms or represents a group of the formula $-SO_2R^8$,

in which

$R^8$ denotes straight-chain or branched alkyl having up to 4 carbon atoms, phenyl or benzyl, where the two last-mentioned radicals are optionally substituted by hydroxyl, fluorine, chlorine, bromine, nitro, cyano, methyl, ethyl or methoxy, or

represents an amino acid radical of the formula

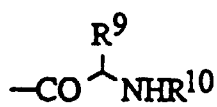

in which

$R^9$ denotes hydrogen, straight-chain or branched alkyl having up to 4 carbon atoms or benzyl

and

$R^{10}$ denotes hydrogen, tert-butoxycarbonyl or Fmoc,

$R^3$ represents hydrogen or represents straight-chain or branched alkyl having up to 4 carbon atoms,
or

$R^2$ and $R^3$ together represent the radical of the formula $=CHR^{14}$
in which

$R^{14}$ denotes hydrogen or straight-chain or branched alkyl having up to 4 carbon atoms,

V represents an oxygen or a sulphur atom or the -NH group,

$R^1$ represents hydrogen or straight-chain or branched alkyl having up to 4 carbon atoms or phenyl, where the latter are optionally substituted by fluorine, chlorine, bromine, nitro, cyano, methoxy or ethoxy or by a group of the formula $-NR^4R^5$ or $-SO_2R^8$,
in which

$R^4$ and $R^5$ are identical or different and denote hydrogen, methyl or ethyl

and

$R^8$ has the abovementioned meaning,

or in the case in which V represents the -NH group,
$R^1$ represents the group of the formula $-SO_2R^8$,
in which

$R^8$ has the abovementioned meaning,

**4.** Cyclopentane- and -pentene-β-amino acids of the general formula (I) according to Claims 1-3, characterised in that they are present in an isomeric form, as acid addition salts or as metal salt complexes.

**5.** Cyclopentane-β-amino acid of the general formula (I) according to Claim 1, in which

A, B, D, L, M and T represent hydrogen,
E and G together represent a radical of the formula

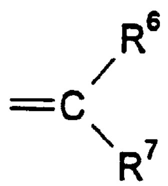

in which

$R^6$ and $R^7$ denote hydrogen,
$R^2$ represents hydrogen,
$R^3$ represents hydrogen and
$R^1$ represents hydrogen and
is present in the form of the (-)-1R*, 2S*-cis enantiomer.

**6.** Cyclopentane-β-amino acid of the general formula (I) according to Claim 1, in which
A, B, D, L, M and T represent hydrogen,
E and G together represent a radical of the formula

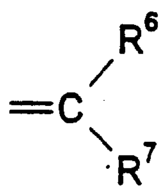

in which

$R^6$ and $R^7$ denote hydrogen,
$R^2$ represents hydrogen,
$R^3$ represents hydrogen and
$R^1$ represents hydrogen and
is present in the form of the hydrochloride salt of the (-)-1R*, 2S*-cis enantiomer.

**7.** Process for the preparation of the compounds of the general formula (I) according to Claims 1-6, characterised in that

[A] compounds of the general formula (II)

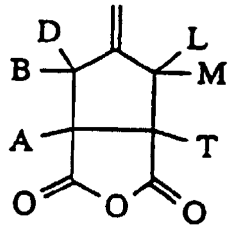

(II)

in which
A, B, D, L, M and T have the abovementioned meaning, are converted in organic solvents, preferably dioxane, first with ($C_1$-$C_3$)-trialkylsilyl azides, then with ethers, in the presence of water, to the compounds of the general formula (III)

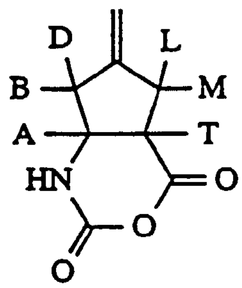

(III)

in which
A, B, D, L, M and T have the abovementioned meaning, and in a next step the products are reacted with acids, preferably hydrochloric acid, with ring opening to give the compounds of the general formula (Ia)

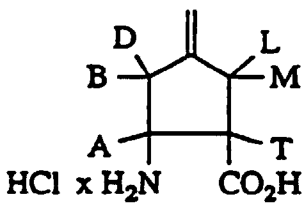

(Ia)

in which
A, B, D, L, M and T have the abovementioned meaning, and if appropriate an elimination with acids, preferably hydrochloric acid, is added,
or
[B] compounds of the general formula (IV)

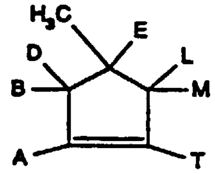

(IV)

in which
A, B, D, E, L, M and T have the abovementioned meaning, are converted by reaction with chlorosulphonyl isocyanate first to the compounds of the general formula (V)

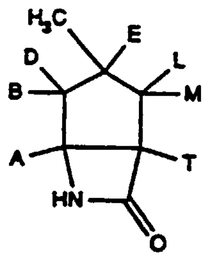

(V)

in which
A, B, D, E, L, M and T have the abovementioned meaning,
and then the amine and carboxyl function are set free with acids, preferably hydrochloric acid, with ring opening,
or
[C] compounds of the general formula (VI)

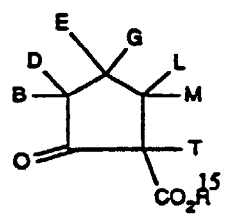

(VI)

in which
B, D, E, G, L, M and T have the abovementioned meaning,
and

$R^{15}$ represents $C_1$-$C_4$-alkyl,

are converted by reaction with amines of the general formula (VII)

$$H_2N\text{-}R^{16} \quad \text{(VII)}$$

in which

$R^{16}$ represents benzyl which is optionally substituted by halogen, $NO_2$, cyano or $C_1$-$C_4$-alkyl or represents the radical of the formula $-CH(C_6H_4\text{-}pOCH_3)_2$,

in organic solvents, if appropriate in the presence of a base, to the compounds of the general formula (VIII)

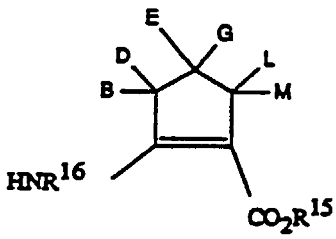

(VIII)

in which
B, D, E, G, L, M, $R^{15}$ and $R^{16}$ have the abovementioned meaning,
and then by double hydrogenation first the double bond is reduced, then the amine function is set free and in a last step the carboxylic acid esters are hydrolysed using acids,
and fundamentally the substituents A - T are derivatised, if appropriate with prior blockage of the amine function, by reaction of the protective groups according to customary methods, for example by oxidation, reduction or alkylation,
and in the case of the acids the esters are hydrolysed according to customary methods,
and in the case of the other definitions mentioned above for V and $R^1$, they are likewise derivatised according to customary methods, such as, for example, amidation, sulphonation or sulphoamidation, if appropriate in the presence of auxiliaries such as catalysts and dehydrating agents, starting from the corresponding carboxylic acids, if appropriate with prior activation,
and in the case of the pure enantiomers a resolution is carried out.

**8.** Compounds of the general formula (I) according to Claims 1-6 for use in combating diseases.

**9.** Use of compounds of the formula (I) according to Claims 1-6 for the production of medicaments.

**10.** Medicaments containing cyclopentane- and -pentene-βamino acids of the general formula (I) according to Claims 1-6.

**Revendications**

**1.** Cyclopentane- et cyclopentène-β-aminoacides de formule générale (I)

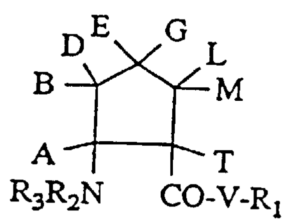

(I),

dans laquelle

A, B, D, E, G, L, M et T sont identiques ou différents et, sous réserve que l'un au moins des substituants mentionnés ci-dessus soit différent de l'hydrogène, représentent de l'hydrogène, un halogène, un groupe benzyle, un groupe hydroxy ou un groupe alkyle linéaire ou ramifié ayant jusqu'à 8 atomes de carbone, qui est substitué le cas échéant une ou deux fois identiques ou différentes par un halogène, un groupe hydroxy, phényle, benzyloxy, carboxy ou par un groupe alkoxy, acyle ou alkoxycarbonyle linéaire ou ramifié ayant chacun jusqu'à 6 atomes de carbone ou par un groupe de formule $-NR^4R^5$,
dans laquelle
$R^4$ et $R^5$ sont identiques ou différents et représentent de l'hydrogène, un groupe phényle ou un groupe alkyle linéaire ou ramifié ayant jusqu'à 6 atomes de carbone,

ou bien B et D, E et G ou L et M forment ensemble dans chaque cas un reste de formule

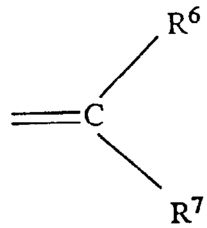

ou =N-OH dans laquelle

$R^6$ et $R^7$ sont identiques ou différents et représentent de l'hydrogène, un halogène ou un groupe alkyle, alkoxy ou oxyacyle linéaire ou ramifié ayant chacun jusqu'à 8 atomes de carbone, un groupe benzyle ou un groupe phényle,

ou bien

E et G et/ou B et D représentent ensemble le reste de formule =O ou =S,

ou bien

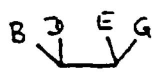 ou 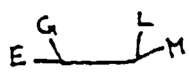

forment dans chaque cas un reste de formule

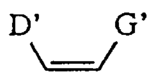 ou 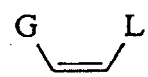

où

D' et G' ont la définition indiquée ci-dessus pour D et G, mais ne représentent pas en même temps de l'hydrogène,

et

G et L ont la définition indiquée ci-dessus,

$R^2$ représente de l'hydrogène ou

un groupe protégeant la fonction amino classique dans la chimie des peptides, ou

un groupe alkyle linéaire ou ramifié ayant jusqu'à 8 atomes de carbone, qui est substitué le cas échéant une ou deux fois identiques ou différentes par un groupe hydroxy, formyle ou par un groupe acyle linéaire ou ramifié ayant jusqu'à 6 atomes de carbone ou par un groupe phényle ou un groupe benzoyle qui sont substitués le cas échéant jusqu'à deux fois identiques ou différentes par un halogène, un groupe nitro, cyano ou par un groupe alkyle linéaire ou ramifié ayant jusqu'à 6 atomes de carbone,

ou bien

un groupe acyle linéaire ou ramifié ayant jusqu'à 8 atomes de carbone, ou bien

un groupe benzoyle qui est éventuellement substitué comme décrit ci-dessus, ou bien un groupe de formule $-SO_2R^8$,

dans laquelle

$R^8$ représente un groupe alkyle linéaire ou ramifié ayant jusqu'à 8 atomes de carbone, un reste benzyle ou phényle, ces deux derniers restes étant substitués le cas échéant jusqu'à

3 fois identiques ou différentes par un halogène, un groupe hydroxy, nitro, cyano, trifluorométhyle, trifluorométhoxy ou par un groupe alkyle, alkoxy ou alkoxycarbonyle linéaire ou ramifié ayant jusqu'à 6 atomes de carbone, un groupe carboxy ou le groupe $-NR^4R^5$ indiqué ci-dessus,

dans lequel

$R^4$ et $R^5$ ont la définition indiquée ci-dessus,

ou bien représente un groupe phényle qui est substitué le cas échéant jusqu'à trois fois identiques ou différentes par un halogène, un groupe hydroxy, nitro, trifluorométhyle, trifluorométhoxy, un groupe alkyle, acyle, alkoxy ou alkoxycarbonyle linéaire ou ramifié ayant jusqu'à 6 atomes de carbone ou par un groupe de formule $-NR^4R^5$ ou $-SO_2R^8$
dans laquelle

$R^4$, $R^5$ et $R^8$ ont la définition indiquée ci-dessus

ou bien
un reste aminoacide de formule

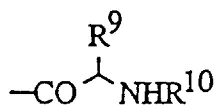

dans laquelle

$R^9$ représente un groupe cycloalkyle ayant 3 à 8 atomes de carbone ou un groupe aryle ayant 6 à 10 atomes de carbone ou de l'hydrogène, ou représente un groupe alkyle linéaire ou ramifié ayant jusqu'à 8 atomes de carbone,
le groupe alkyle étant substitué le cas échéant par un groupe cyano, méthylthio, hydroxy, mercapto, guanidyle ou par un groupe de formule $-NR^{11}R^{12}$ ou $R^{13}$-OC-,

dans laquelle

$R^{11}$ et $R^{12}$ représentent, indépendamment l'un de l'autre, de l'hydrogène, un groupe alkyle linéaire ou ramifié ayant jusqu'à 8 atomes de carbone ou un groupe phényle,

et

$R^{13}$ représente un groupe hydroxy, benzyloxy, alkoxy ayant jusqu'à 6 atomes de carbone ou le groupe $-NR^{10}R^{11}$ indiqué ci-dessus, ou bien le groupe alkyle étant substitué le cas échéant par un groupe cycloalkyle ayant 3 à 8 atomes de carbone ou par un groupe aryle ayant 6 à 10 atomes de carbone, qui est substitué de son côté par un groupe hydroxy, un halogène, un groupe nitro, alkoxy ayant jusqu'à 8 atomes de carbone ou par le groupe $-NR^{11}R^{12}$,

dans lequel

$R^{11}$ et $R^{12}$ ont la définition indiquée ci-dessus

et

$R^{10}$ représente de l'hydrogène ou un groupe protégeant la fonction amino classique dans la chimie des peptides

$R^3$ est de l'hydrogène ou un groupe alkyle linéaire ou ramifié ayant jusqu'à 8 atomes de carbone, qui est substitué par un groupe phényle,

ou bien

$R^2$ et $R^3$ forment ensemble le reste de formule $=CHR^{14}$, dans laquelle

$R^{14}$ est de l'hydrogène ou un groupe alkyle linéaire ou ramifié ayant jusqu'à 8 atomes de carbone, qui est substitué le cas échéant par un halogène, un groupe hydroxy, phényle, carboxy ou par un groupe alkoxy ou alkoxycarbonyle linéaire ou ramifié ayant jusqu'à 6 atomes de carbone,

V représente un atome d'oxygène ou de soufre ou le groupe -NH-,
$R^1$ est de l'hydrogène ou un groupe alkyle linéaire ou ramifié ayant jusqu'à 8 atomes de carbone ou un groupe phényle, ces derniers groupes étant substitués le cas échéant jusqu'à 3 fois identiques ou différents par un groupe hydroxy, un halogène, un groupe nitro, cyano, carboxy, trifluorométhyle, trifluorométhoxy, un groupe alkoxy linéaire ou ramifié ainsi que, dans le cas du groupe phényle, par un groupe alkyle, acyle ou alkoxycarbonyle ayant dans chaque cas jusqu'à 6 atomes de carbone ou par un groupe de formule $-NR^4R^5$ ou $-SO_2R^8$,
dans laquelle
$R^4$, $R^5$ et $R^8$ ont la définition indiquée ci-dessus,

ou bien au cas où V est le groupe -NH-,

$R^1$ représente le groupe de formule $-SO_2R^8$, dans laquelle

$R^8$ a la définition indiquée ci-dessus, sous réserve que

lorsque V est un atome d'oxygène,

T ne soit pas un groupe méthyle, éthyle, benzyle ou hydroxyle,
$R^1$ ne soit pas un groupe méthyle ou éthyle, et
M et L ne soient pas un groupe $-CH_2-COOH$.

**2.** Cyclopentane- et cyclopentène-β-aminoacides de formule générale (I) suivant la revendication 1, dans laquelle

A, B, D, E, G, L, M et T sont identiques ou différents et, sous réserve que l'un au moins des substituants mentionnés ci-dessus soient différents de l'hydrogène, ils représentent de l'hydrogène, un halogène, un groupe benzyle, hydroxy, ou un groupe alkyle linéaire ou ramifié ayant jusqu'à 6 atomes de carbone, qui est substitué le cas échéant une ou deux fois identiques ou différentes par un halogène, un groupe hydroxy, benzyloxy, ou par un groupe alkoxy, acyle ou alkoxycarbonyle linéaire ou ramifié ayant chacun jusqu'à 4 atomes de carbone ou par un groupe de formule $-NR^4R^5$,

dans laquelle

$R^4$ et $R^5$ sont identiques ou différents et représentent de l'hydrogène ou un groupe alkyle linéaire ou ramifié ayant jusqu'à 4 atomes de carbone,

ou bien B et D, E et G ou L et M représent ensemble dans chaque cas un reste de formule

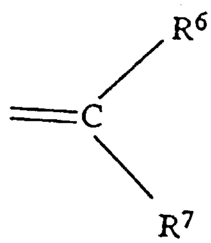

ou =N-OH

dans laquelle

$R^6$ et $R^7$ sont identiques ou différents et représentent de l'hydrogène, du fluor, du chlore, du brome ou un groupe alkyle linéaire ou ramifié ayant jusqu'à 6 atomes de carbone, un groupe benzyle ou un groupe phényle, ou bien

E et G et/ou B et D forment ensemble le reste de formule =O ou =S,

ou bien

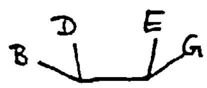 ou 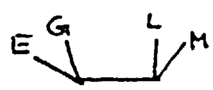

forment ensemble dans chaque cas un reste de formule

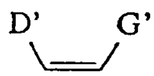 ou 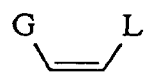

dans laquelle

D' et G' ont les définitions indiquées ci-dessus pour D et G, mais ne représentent pas en même temps de l'hydrogène,

et

G et L ont la définition indiquée ci-dessus,

$R^2$ représente de l'hydrogène ou

un groupe Boc, benzyle, benzyloxycarbonyle, allyloxycarbonyle ou 9-fluorénylméthyloxycarbonyle (Fmoc), ou

un groupe alkyle linéaire ou ramifié ayant jusqu'à 6 atomes de carbone, qui est substitué le cas échéant par un groupe hydroxy, formyle ou par un groupe acyle linéaire ou ramifié ayant jusqu'à 4 atomes de carbone ou par un groupe phényle ou un groupe benzoyle, qui sont substitués le cas échéant par un halogène, un groupe nitro, cyano ou par un groupe alkyle linéaire ou ramifié ayant jusqu'à 4 atomes de carbone, ou bien

un groupe acyle linéaire ou ramifié ayant jusqu'à 6 atomes de carbone ou un groupe benzoyle qui est substitué le cas échéant comme décrit ci-dessus, ou bien un groupe de formule $-SO_2R^8$,

dans laquelle

$R^8$ est un groupe alkyle linéaire ou ramifié ayant jusqu'à 6 atomes de carbone, un groupe phényle ou un groupe benzyle, les deux derniers groupes mentionnés étant substitués le cas échéant jusqu'à deux fois identiques ou différentes par un halogène, un groupe hydroxy, nitro, cyano, trifluorométhyle, trifluorométhoxy ou par un groupe alkyle ou alkoxy linéaire ou ramifié ayant chacun jusqu'à 4 atomes de carbone ou par le groupe de formule $-NR^4R^5$ indiqué ci-dessus,

dans lequel

$R^4$ et $R^5$ ont la définition indiquée ci-dessus, ou représente un groupe phényle qui est substitué le cas échéant jusqu'à deux fois identiques ou différentes par un halogène, un groupe hydroxy, nitro, trifluorométhyle, trifluorométhoxy, un groupe alkyle, acyle, alkoxy ou alkoxycarbonyle linéaire ou ramifié ayant dans chaque cas jusqu'à 4 atomes de carbone ou par un groupe de formule $-NR^6R^7$ ou $-SO_2R^8$,
dans laquelle
$R^6$, $R^7$ et $R^8$ ont la définition indiquée ci-dessus,
ou représente
un reste aminoacide de formule

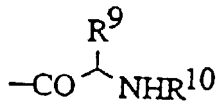

dans laquelle

$R^9$ est de l'hydrogène, un groupe alkyle linéaire ou ramifié ayant jusqu'à 6 atomes de carbone ou un groupe benzyle,

et

$R^{10}$ représente de l'hydrogène, un groupe benzyloxycarbonyle, tertio-butoxycarbonyle ou Fmoc,

$R^3$ est de l'hydrogène ou un groupe alkyle linéaire ou ramifié ayant jusqu'à 6 atomes de carbone ou un groupe benzyle,

ou bien

$R^2$ et $R^3$ forment ensemble le reste de formule $=CHR^{14}$ dans laquelle

$R^{14}$ est de l'hydrogène ou un groupe alkyle linéaire ou ramifié ayant jusqu'à 6 atomes de carbone, qui est substitué le cas échéant par un halogène, un groupe hydroxy ou par un groupe alkoxy ou alkoxycarbonyle linéaire ou ramifié ayant dans chaque cas jusqu'à 4 atomes de carbone,

V est un atome d'oxygène ou de soufre ou un groupe -NH-,
$R^1$ est de l'hydrogène ou un groupe alkyle linéaire ou ramifié ayant jusqu'à 6 atomes de carbone ou un groupe phényle, ces derniers étant substitués le cas échéant jusqu'à deux fois identiques ou différentes par un groupe hydroxy, un halogène, un groupe nitro, cyano, trifluorométhyle, trifluorométhoxy, par un groupe alkoxy, acyle ou alkoxycarbonyle linéaire ou ramifié ayant dans chaque cas jusqu'à 4 atomes de carbone ou par un groupe de formule $-NR^4R^5$ ou $-SO_2R^8$,
dans laquelle
$R^4$, $R^5$ et $R^8$ ont la définition indiquée ci-dessus,

ou bien, au cas où V représente un groupe -NH-,

$R^1$ est le groupe de formule $-SO_2R^8$,
dans laquelle

$R^8$ a la définition indiquée ci-dessus.

**3.** Cyclopentane- et cyclopentène-β-aminoacides de formule générale (I) suivant la revendication 1, dans laquelle

A, B, D, E, G, L, M et T sont identiques ou différents et, sous réserve que l'un au moins des substituants mentionnés ci-dessus soit différent de H, ils représentent de l'hydrogène, du fluor, du chlore, du brome, un groupe benzyle, hydroxy ou un groupe alkyle linéaire ou ramifié ayant jusqu'à 4 atomes de carbone, qui est

substitué le cas échéant une fois ou deux fois identiques ou différentes par un groupe hydroxy ou benzyloxy, ou bien B et D, E et G ou L et M forment ensemble dans chaque cas un reste de formule

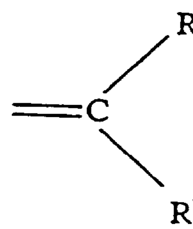

ou

ou =N-OH

dans laquelle

$R^6$ et $R^7$ sont identiques ou différents et représentent de l'hydrogène, du fluor, du chlore, du brome ou un groupe alkyle linéaire ou ramifié ayant jusqu'à 4 atomes de carbone ou un groupe phényle, ou bien E et G et/ou B et D forment ensemble le reste de formule =O ou =S,

ou bien

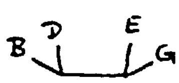

ou

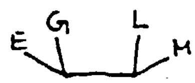

forment ensemble dans chaque cas un reste de formule

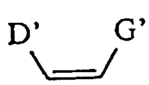

ou

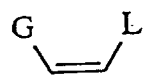

dans laquelle

D' et G' ont la définition indiquée ci-dessus pour D et G, mais ne représentent pas en même temps de l'hydrogène,

G et L sont identiques ou différents et représentent de l'hydrogène ou un groupe méthyle,

$R^2$ représente de l'hydrogène, un groupe allyloxycarbonyle, benzyle, Boc ou Fmoc, un groupe alkyle linéaire ou ramifié ayant jusqu'à 4 atomes de carbone, ou bien un groupe acyle linéaire ou ramifié ayant jusqu'à 4 atomes de carbone, ou bien un groupe de formule $-SO_2R^8$, dans laquelle

$R^8$ est un groupe alkyle linéaire ou ramifié ayant jusqu'à 4 atomes de carbone, un reste phényle ou un reste benzyle, ces deux derniers restes étant éventuellement substitués par un groupe hydroxy, du fluor, du chlore, du brome, un groupe nitro, cyano, méthyle, éthyle ou méthoxy,

ou représente un reste aminoacide de formule

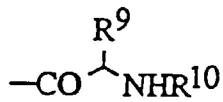

dans laquelle

$R^9$ est de l'hydrogène, un groupe alkyle linéaire ou ramifié ayant jusqu'à 4 atomes de carbone ou un groupe benzyle,

et

$R^{10}$ est de l'hydrogène, un groupe tertiobutoxycarbonyle ou Fmoc,

$R^3$ est de l'hydrogène, ou un groupe alkyle linéaire ou ramifié ayant jusqu'à 4 atomes de carbone,

ou bien

$R^2$ et $R^3$ forment ensemble le reste de formule $=CHR^{14}$, dans laquelle

$R^{14}$ est de l'hydrogène ou un groupe alkyle linéaire ou ramifié ayant jusqu'à 4 atomes de carbone,

V représente un atome d'oxygène ou un atome de soufre ou le groupe -NH-,
$R^1$ est de l'hydrogène ou un groupe alkyle linéaire ou ramifié ayant jusqu'à 4 atomes de carbone ou un groupe phényle, ces derniers étant substitués le cas échéant par du fluor, du chlore, du brome, un groupe nitro, cyano, méthoxy, éthoxy ou par un groupe de formule $-NR^4R^5$ ou $-SO_2R^8$
dans laquelle

$R^4$ et $R^5$ sont identiques ou différents et représentent de l'hydrogène, un groupe méthyle ou éthyle,

et

$R^8$ a la définition indiquée ci-dessus,

ou bien, au cas où V représente le groupe -NH-,

$R^1$ est le groupe de formule $-SO_2R^8$,
dans laquelle

$R^8$ a la définition indiquée ci-dessus.

**4.** Cyclopropane- et cyclopentène-β-aminoacides de formule générale (I) suivant les revendications 1 à 3, caractérisés en ce qu'ils se présentent sous une forme isomère, comme sels d'addition d'acides ou comme complexes de sels métalliques.

**5.** Cyclopentane-β-aminoacide de formule générale (I) suivant la revendication 1, dans lequel
A, B, D, L, M et T représentent de l'hydrogène,
E et G forment ensemble un reste de formule

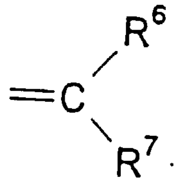

dans laquelle

$R^6$ et $R^7$ représentent de l'hydrogène,
$R^2$ est de l'hydrogène,
$R^3$ est de l'hydrogène, et
$R^1$ est de l'hydrogène, et

qui se présente sous la forme de l'énantiomère (-)-1R*,2S*cis.

**6.** Cyclopentane-β-aminoacide de formule générale (I) suivant la revendication 1, dans lequel A, B, D, L, M et T représentent de l'hydrogène,
E et G forment ensemble un reste de formule

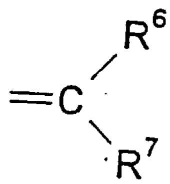

dans laquelle

$R^6$ et $R^7$ représentent de l'hydrogène,
$R^2$ est de l'hydrogène,
$R^3$ est de l'hydrogène, et
$R^1$ est de l'hydrogène, et

qui se présente sous la forme du chlorhydrate de l'énantiomère (-)-1R*,2S*-cis.

**7.** Procédé de production des composés de formule générale (I) suivant les revendications 1 à 6, caractérisé en ce que

[A] on transforme tout d'abord des composés de formule générale (II)

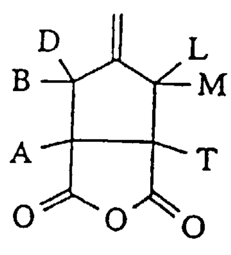

(II),

dans laquelle
A, B, D, L, M et T ont la définition indiquée ci-dessus, dans des solvants organiques, de préférence le dioxanne, avec des tri-(alkyle en $C_1$ à $C_3$)silylazides, puis avec des éthers, en présence d'eau, en les composés de formule générale (III)

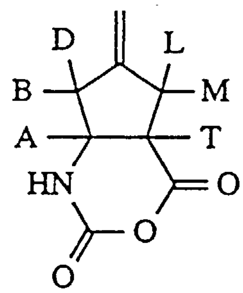

(III),

dans laquelle
A, B, D, L, M et T ont la définition indiquée ci-dessus, et dans une étape subséquente, on convertit ces composés avec des acides, de préférence l'acide chlorhydrique, par décyclisation en les composés de formule générale (Ia)

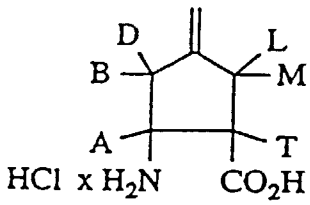

(Ia),

dans laquelle
A, B, D, L, M et T ont la définition indiquée ci-dessus, et le cas échéant, on effectue ensuite une élimination avec des acides, de préférence l'acide chlorhydrique, ou bien
[B] on transforme tout d'abord des composés de formule générale (IV)

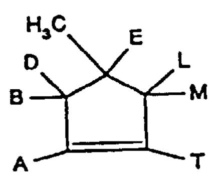

(IV),

dans laquelle
A, B, D, E, L, M et T ont la définition indiquée ci-dessus, par réaction avec l'isocyanate de chlorosulfonyle en les composés de formule générale (V)

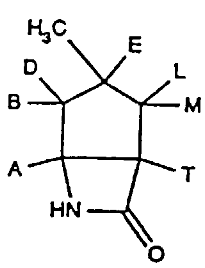

(V),

dans laquelle
A, B, D, L, M et T ont la définition indiquée ci-dessus puis on libère la fonction amine et la fonction carboxy par décyclisation avec des acides, de préférence l'acide chlorhydrique,
ou bien
[C] on transforme tout d'abord des composés de formule générale (VI)

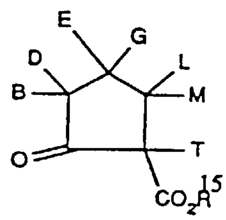

(VI),

dans laquelle
B, D, E, G, L, M et T ont la définition indiquée ci-dessus, et
$R^{15}$ est un groupe alkyle en $C_1$ à $C_4$,
par réaction avec des amines de formule générale (VII)

$$H_2N\text{-}R^{16} \qquad (VII),$$

dans laquelle

$R^{16}$ est un groupe benzyle qui est substitué le cas échéant par un halogène, un groupe $NO_2$, cyano ou alkyle en $C_1$ à $C_4$ ou représente le reste de formule $-CH(C_6H_4\text{-}pOCH_3)_2$,

dans des solvants organiques, éventuellement en présence d'une base, en les composés de formule générale (VIII)

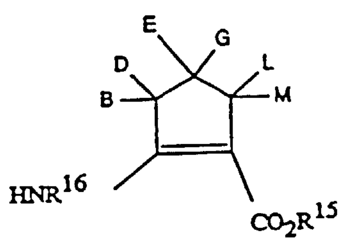

(VIII),

dans laquelle
B, D, E, G, L, M, $R^{15}$ et $R^{16}$ ont la définition indiquée ci-dessus
puis on réduit tout d'abord la double liaison par une double hydrogénation, on libère ensuite la fonction amine et on saponifie dans une dernière étape l'ester d'acide carboxylique avec des acides,

et on dérivatise essentiellement les substituants A à T, le cas échéant avec blocage préalable de la fonction amine par réaction des groupes protecteurs selon des procédés classiques, par exemple par oxydation, réduction ou alkylation,

et dans le cas des acides, on saponifie les esters par des procédés classiques

et dans le cas des autres définitions indiquées ci-dessus pour V et $R^1$, on effectue également une dérivatisation par des procédés classiques, par exemple amidation, sulfonation ou sulfamidation, le cas échéant en présence de substances auxiliaires telles que des catalyseurs et des agents déshydratants, en partant des acides carboxyliques correspondants, le cas échéant après activation préalable, et dans le cas des énantiomères purs, on effectue un dédoublement du racémate.

**8.** Composés de formule générale (I) suivant les revendications 1 à 6, destinés à être utilisés pour combattre des maladies.

**9.** Utilisation de composés de formule (I) suivant les revendications 1 à 6 pour la préparation de médicaments.

**10.** Médicaments contenant des cyclopentane- et cyclopentène-β-aminoacides de formule générale (I) suivant les

revendications 1 à 6.